(19) Europäisches Patentamt
European Patent Office
Office européen des brevets

(11) EP 0 741 128 B1

(12) **EUROPÄISCHE PATENTSCHRIFT**

(45) Veröffentlichungstag und Bekanntmachung des Hinweises auf die Patenterteilung:
**20.06.2001 Patentblatt 2001/25**

(51) Int Cl.[7]: **C07C 305/06**, C07H 15/18, A61K 31/255, A61K 31/70

(21) Anmeldenummer: **96106471.4**

(22) Anmeldetag: **24.04.1996**

(54) **Schwefelsäureester von Zuckeralkoholen zum Hemmen von der Migration und Proliferation von glatten Muskelzellen der Gefässwand**

Sulphuric acid esters of sugar alcohols for the inhibition of the migration and proliferation of smooth muscle cells of the vascular walls

Esters d'acide sulfurique et des alcools de sucre pour l'inhibition de la migration et de la prolifération de cellules des muscles lisses des parois vasculaires

(84) Benannte Vertragsstaaten:
**AT BE CH DE DK ES FI FR GB GR IE IT LI LU NL PT SE**

(30) Priorität: **05.05.1995 CH 131095**

(43) Veröffentlichungstag der Anmeldung:
**06.11.1996 Patentblatt 1996/45**

(73) Patentinhaber: **F. HOFFMANN-LA ROCHE AG**
**4070 Basel (CH)**

(72) Erfinder:
- **Chucholowski, Alexander**
**79639 Grenzach-Wyhlen (DE)**
- **Pech, Michael**
**79258 Hartheim (DE)**
- **Fingerle, Jürgen**
**79618 Rheinfelden (DE)**
- **Rouge, Marianne**
**4058 Basel (CH)**
- **Iberg, Niggi**
**4059 Basel (CH)**
- **Schmid, Gérard**
**4468 Kienberg (CH)**
- **Märki, Hans Peter**
**4059 Basel (CH)**
- **Tschopp, Thomas**
**4107 Ettingen (CH)**
- **Müller, Rita**
**4051 Basel (CH)**
- **Wessel, Hans Peter**
**79423 Heitersheim (DE)**

(74) Vertreter: **Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 312 086**
**EP-A- 0 479 093**
**EP-A- 0 504 645**
**EP-A- 0 663 391**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 741 128 B1

## Beschreibung

Die vorliegende Erfindung betrifft neue Schwefelsäureester von Zuckeralkoholen und Zuckeralkohol-ähnlichen Verbindungen enthaltend mindestens drei Reste von Zuckeralkoholen oder Zuckeralkohol-ähnlichen Verbindungen der allgemeinen Formel Ia und Ib

$$\left.\begin{array}{l}(A^1\text{-}X^1)_{m1}\text{-}(Y^1\text{-}X^2\text{-})_{n1}\text{-}(Q^1\text{-}X^3\text{-})_{m2}\text{-}(Y^2\text{-}X^4)_{n2}\text{-}(\text{-}Z^1\text{-}X^5\text{-})_{m3}\text{-}(Y^3\text{-}X^6)_{n3}\\(A^2\text{-}X^7)_{m4}\text{-}(Y^4\text{-}X^8\text{-})_{n4}\text{-}(Q^2\text{-}X^9\text{-})_{m5}\text{-}(Y^5\text{-}X^{10})_{n5}\text{-}(\text{-}Z^2\text{-}X^{11}\text{-})_{m6}\text{-}(Y^6\text{-}X^{12})_{n6}\end{array}\right\rangle D \qquad \text{Ia}$$

$$\left.\begin{array}{l}(A^1\text{-}X^1)_{m1}\text{-}(Y^1\text{-}X^2\text{-})_{n1}\text{-}(Q^1\text{-}X^3\text{-})_{m2}\text{-}(Y^2\text{-}X^4)_{n2}\text{-}(\text{-}Z^1\text{-}X^5\text{-})_{m3}\text{-}(Y^3\text{-}X^6)_{n3}\\(A^2\text{-}X^7)_{m4}\text{-}(Y^4\text{-}X^8\text{-})_{n4}\text{-}(Q^2\text{-}X^9\text{-})_{m5}\text{-}(Y^5\text{-}X^{10})_{n5}\text{-}(\text{-}Z^2\text{-}X^{11}\text{-})_{m6}\text{-}(Y^6\text{-}X^{12})_{n6}\text{-}\\(A^3\text{-}X^{13})_{m7}\text{-}(Y^7\text{-}X^{14}\text{-})_{n7}\text{-}(Q^3\text{-}X^{15}\text{-})_{m8}\text{-}(Y^8\text{-}X^{16})_{n8}\text{-}(\text{-}Z^3\text{-}X^{17}\text{-})_{m9}\text{-}(Y^9\text{-}X^{18})_{n9}\end{array}\right\rangle W \qquad \text{Ib}$$

worin

$n^1$ - $n^9$ unabhängig voneinander je 0 oder 1 sind; und
$m^1$ - $m^9$ unabhängig voneinander je 0 oder 1 sind, jedoch mit der Massgabe, dass mindestens eines von $m^1$, $m^2$ und $m^3$, mindestens eines von $m^4$, $m^5$ und $m^6$ und, falls vorhanden,mindestens eines von $m^7$, $m^8$ und $m^9$, 1 sind; und worin
$X^1$ - $X^{18}$ unabhängig voneinander je -O-, -CONR$^1$-,-NR$^1$CO-, -NR$^1$-;
$R^1$ Wasserstoff oder $C_1$-$C_7$ Alkyl;
W einen Benzolrest oder einen s-Triazinrest;
$Y^1$ - $Y^9$ unabhängig voneinander je aromatische Ringsysteme;
$A^1$ - $A^3$ unabhängig voneinander je einen um die 1-Hydroxygruppe verminderten Rest eines Zuckeralkohols oder eines Derivats davon, einen um die 1-Carboxygruppe verminderten Rest einer Zuckersäure oder eines Derivats davon oder den Tris-(hydroxymethyl)-methylrest;
D den um 2 Hydroxygruppen verminderten Di-Rest eines Zuckeralkohols oder eines Derivats davon oder den um 2 Carboxygruppen verminderten Di-Rest einer Zuckerdicarbonsäure oder eines Derivats davon;
$Q^1$ - $Q^3$ und $Z^1$ - $Z^3$ unabhängig voneinander je den um 2 Hydroxygruppen verminderten Di-Rest eines Zuckeralkohols oder eines Derivats davon oder den um 2 Carboxygruppen verminderten Di-Rest einer Zuckerdicarbonsäure oder eines Derivats davon oder einen Didesoxyglycopyranosid-Rest oder ein Derivat davon bedeuten, wobei mindestens eine Hydroxygruppe der Reste $A^1$ - $A^3$, D, $Q^1$- $Q^3$ und $Z^1$ - $Z^3$ mit Schwefelsäure verestert ist;
Derivate der Zuckeralkohole sind einfach oder mehrfach desoxygenierte Zuckeralkohole, ein entsprechender Ether an $C_1$ oder eine Verbindung der Formel

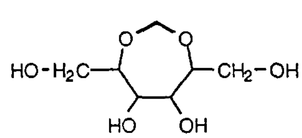
;

Derivate von Zuckermono- und Zuckerdicarbonsäuren sind Zuckersäuren, deren Hydroxyfunktionen frei oder durch Acetonide, Methylenacetale, Arylmethyläther, Alkyl- oder Arylester geschützt, mit Desoxystellen zu Oxiran-Ringen oder mit Carbonsäurefunktionen zu 5 oder 6 gliedrigen Laktonen geschlossen sind;
Derivate des Didesoxyglycopyranosid-Rests sind die entsprechende Ether an $C_1$;

und pharmazeutisch anwendbare Salze davon.

EP 0504645 beschreibt die Verwendung von Verbindungen der Formel (I)

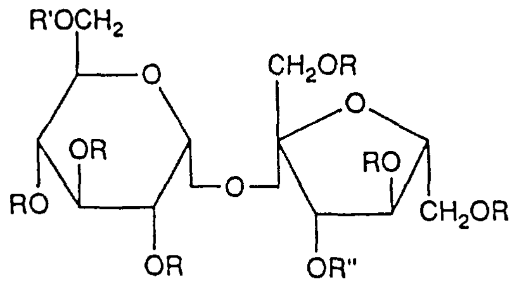

(I)

worin R Wasserstoff oder ein Rest $-SO_3M$; M ein Kation; R' und R" Wasserstoff oder ein $\alpha$-glykosidisch verknüpfter sulfatierter Mono- oder Disaccharidrest ist; und wobei im Mittel mindestens eine $-SO_3M$-Gruppe pro Monosaccharideinheit anwesend ist,

zur Herstellung von pharmazeutischen Präparaten zur Prävention und/oder Behandlung arteriosklerotischer Erkrankungen und zur Verhinderung der Restenosierung nach gefässchirurgischen Eingriffen und nach Organtransplantationen.

EP 0479093 betrifft sulfatierte Oligosaccharidderivate der Formel (I)

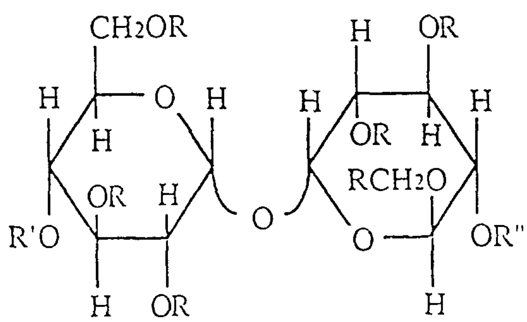

(I)

worin R, R' und R" die in der Beschreibung angegebene Bedeutung haben, können als Heilmittel, besonders zur Behandlung arteriosklerotischer Erkrankungen verwendet werden.

EP 0312086 beschreibt Polyschwefelsäureester von Bis-aldonsäureamiden, bei denen die zugrundeliegenden Aldonsäuren in 3-, 4- oder 6-Stellung glykosidisch mit einem Galaktopyranosyl, Mannopyranosyl-, Glucopyranosyl- , oder Oligopyranosylrest verknüpft sein können.

EP 0663391 betrifft Schwefelsäureester von Zuckeralkoholen und Zuckeralkohol- ähnlichen Verbindungen der allgemeinen Formel (I)

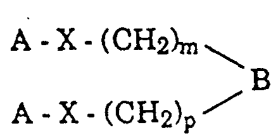

(I)

worin

A ein um die 1-Hydroxygruppe verminderter Rest eines Zuckeralkohols oder eines Derivats davon, oder der tris (Hydroxymethyl)methylrest ist, wobei mindestens eine Hydroxygruppe des Restes A mit Schwefelsäure verestert ist;

X $-NR^1CO-$; -NHCONH-; -NHCSNH-; $-NHSO_2-$, $-NR^1-$ oder -O-;

m und p unabhängig voneinander 0 oder 1;

$R^1$ Wasserstoff, nieder-Alkyl oder Hydroxy-nieder-alkyl; und

B ein System von konjugierten Mehrfachbindungen ist;
und Salze davon.

Ein nicht in einem Ringsystem liegendes Kohlenstoffatom, das eine freie oder mit Schwefelsäure veresterte Hydroxygruppe trägt, darf nicht gleichzeitig direkt an ein weiteres Heteroatom gebunden sein.

Die Erfindung betrifft weiterhin pharmazeutische Präparate, enthaltend eine Verbindung der allgemeinen Formel Ia oder Ib oder Salze davon; die Verwendung der Verbindungen der allgemeinen Formel Ia und Ib und deren Salze als Heilmittel, insbesondere zur Behandlung und/oder Prophylaxe von Krankheiten, die durch exzessive oder destruktive Proliferation von glatten Muskelzellen gekennzeichnet sind sowie von arteriosklerotischen Gefässwandveränderungen, z.B. zur Verhinderung von Restenose nach koronarer oder peripherer Angioplastie oder nach Bypass-Operationen bzw. zur Herstellung von Heilmitteln für die genannten Indikationen; sowie ein Verfahren zur Herstellung der Verbindungen der allgemeinen Formel Ia und Ib und deren Salze.

In der allgemeinen Formeln Ia und Ib lassen sich folgende Strukturelemente unterscheiden:

a) Mono-Reste von Zuckeralkoholen oder Zuckersäuren, die über eine geeignete funktionelle Gruppe verknüpft sind, z.B. $A^1$-$X^1$, $A^2$-$X^7$ etc.
b) Di-Reste von Zuckeralkoholen oder Zuckersäuren oder von Didesoxyglycopyranosiden, die über zwei geeignete funktionelle Gruppen verknüpft sind, z.B. $X^6$-D-$X^{12}$, $X^2$-$Q^1$-$X^3$, $X^4$-$Z^1$-$X^5$ etc.
c) aromatische Einheiten, die über zwei geeignete funktionelle Gruppen verknüpft sind, z.B. $X^1$-$Y^1$-$X^2$, $X^3$-$Y^2$-$X^4$ etc.
d) aromatische Einheiten, die über drei geeignete funktionelle Gruppen verknüpft sind, z.B.

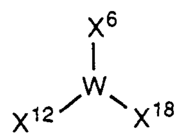

Durch Verknüpfung dieser Strukturelemente lassen sich Verbindungen in unterschiedlicher Molekülgrösse aufbauen.

Gemeinsames Merkmal der erfindungsgemässen Verbindungen ist, dass sie mindestens drei Reste enthalten, die sich von einem Zuckeralkohol, einer Zuckersäure oder von einem Glycopyranosid ableiten.

Bevorzugt sind Verbindungen der allgemeinen Formel Ia und Ib in welchen die zwei bzw. drei Seitenketten identisch sind.

In den aromatischen Einheiten, die über drei geeignete funktionelle Gruppen verknüpft sind, sind Verbindungen der Formel Ib bevorzugt, worin $m^2$, $m^3$, $m^5$, $m^6$, $m^8$, $m^9$ und $n^2$, $n^3$, $n^5$, $n^6$, $n^8$, $n^9$ 0 sind.

Beispiele von Zuckeralkoholen, von denen sich die Reste $A^1$-$A^3$, D, $Q^1$-$Q^3$, $Z^1$-$Z^3$ ableiten, sind Hexitole, wie Glucitol, Galaktitol, Mannitol und Gulitol ; Pentitole, wie Arabinitol, Ribitol und Xylitol; Tetritole wie Threitol und Erythritol oder der Glycerolrest. Derivate solcher Zuckeralkohole können einfach oder mehrfach desoxygenierte Zuckeralkohole, wie L-Rhamnitol sein.

Beispiele von Zuckercarbonsäuren, von denen sich die Reste $A^1$-$A^3$ ableiten, sind Ribonsäure, Glukonsäure, Gulonsäure.

Beispiele von Zuckerdicarbonsäuren, von denen sich die Reste D, $Q^1$-$Q^3$, $Z^1$-$Z^3$ ableiten, sind Weinsäure, Galaktarsäure, Glukarsäure.

Diese Zuckeralkohole und Zuckercarbonsäuren können in der D- oder L-Form oder als Razemate vorliegen, wobei die natürlich vorkommende Form bzw. die Form, die dem zugrundeliegenden, natürlich vorkommenden Zucker entspricht, bevorzugt ist.

Vorzugsweise leitet sich der der Rest $A^1$-$A^3$ von Glucitol, Arabinitol, Glycerol oder von Tris-(hydroxymethyl)-methan ab.

Ein Derivat eines Zuckeralkohols ist beispielsweise ein entsprechender Ether an $C_1$ oder eine cyclische Verbindung der Formel

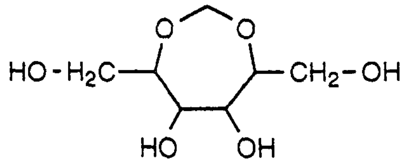

Ein Derivat eines Didesoxyglycopyranosid-Rests ist beispielsweise ein entsprechender Ether an $C_1$.

Beispiele für aromatische Ringsysteme $Y^1$ - $Y^9$ in Formel Ia und Ib sind Reste der Formel a -h,

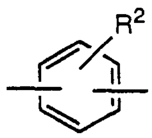
a)

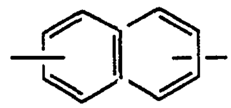
e)

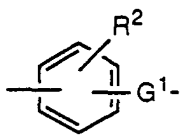
b)

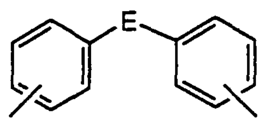
f)

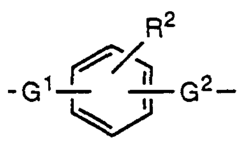
c)

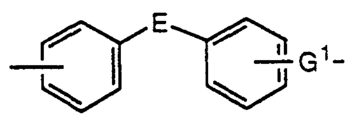
g)

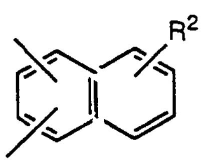
d)

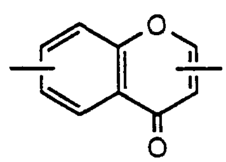
h)

worin

$R^2$ Wasserstoff, Halogen, Nitro, niederes Alkyl, niederes Alkoxy, niederes Aralkoxy, Carbamoyl oder den Glycerolrest, wobei gegebenenfalls vorhandene Hydroxygruppen auch sulfatiert sein können,
$G^1$ und $G^2$ niederes Alkylen, niederes Alkenylen, niederes Alkinylen oder niederes Alkylenoxy;
E eine Kohlenstoff-Kohlenstoff-Bindung; -O-, -CO, $-CH_2-$, $-CH_2-CH_2-$, -CH=CH-, -C≡C-, $-NR^3-CO-$, $-CO-NR^3-$; und
$R^3$ Wasserstoff oder niederes-Alkyl bedeuten.

Der Ausdruck "niederes Alkyl" und " niederes Alkylen" umfasst geradkettige oder verzweigte gesättigte Kohlenwasserstoffreste mit bis zu 7, vorzugsweise bis zu 4 Kohlenstoffatomen, wie Methyl, Ethyl, n-Propyl, Isopropyl, n-Butyl, Isobutyl, tert.Butyl u.dgl.

In analoger Weise umfassen die Ausdrücke "niederes Alkenyl", "niederes Alkenylen" und "niederes Alkinyl", "niederes Alkinylen" ungesättigte, eine Doppel- bzw. Dreifachbindung enthaltende Kohlenwasserstoffreste mit bis zu 7, vorzugsweise bis zu 4 Kohlenstoffatomen, wie Vinyl, Ethinyl, Allyl, Propargyl u.dgl.

Der Ausdruck "niederes Alkoxy", "niederes Alkylenoxy" umfasst Alkyloxygruppen bzw. Alkylenoxygruppen im

Sinne der obigen Umschreibung der Begriffe "niederes Alkyl" bzw. "niederes Alkylen".

Der Ausdruck "Aralkoxy umfasst durch Phenyl und Biphenylreste substituierte Alkoxygruppen.

Der Ausdruck "Halogen" umfasst Fluor, Chlor, Brom und Jod, wovon Brom bevorzugt ist.

Phenylenreste der Formeln a-c sind vorzugsweise 1,4-Phenylenreste.

In der Formeln f und g stehen die von den Ringen ausgehenden Bindungen vorzugsweise in p-Stellung zueinander.

Beispiele von Salzen von Verbindungen der allgemeinen Formel Ia und Ib sind Alkalimetallsalze, wie Na- oder K-Salze, Ammoniumsalze und Salze von tertiären Aminen, wie Triethylamin, oder Pyridinium- oder Imidazoliumsalze, oder quaternäre Ammoniumsalze, wie Dodecyl-trimethylammonium-, Ethylpyridinium- und Benzethoniumsalze; sowie Erdalkalimetallsalze, wie Ca- oder Mg-Salze.

Bevorzugte Verbindungen der Formel I sind:

1,6-Bis-O-[6-methoxy-3-(2,3,4,5,6-penta-O-sulfo-D-glucit-1-ylcarbamoyl)-naphthalin-2-yl]-2,3,4,5-tetra-O-sulfo-galactitol Tetradekanatriumsalz (Beispiel 20);

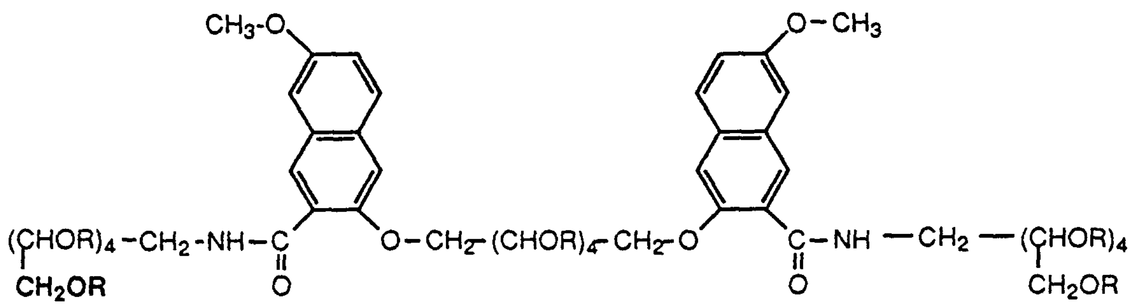

$R=SO_3Na$

1,6-Bis-O-[8-methoxy-3-(2,3,4,5,6-penta-O-sulfo-D-glucit-1-ylcarbamoyl)-naphthalin-2-yl]-2,3,4,5-tetra-O-sulfo-galactitol Tetradekanatriumsalz (Beispiel 21);

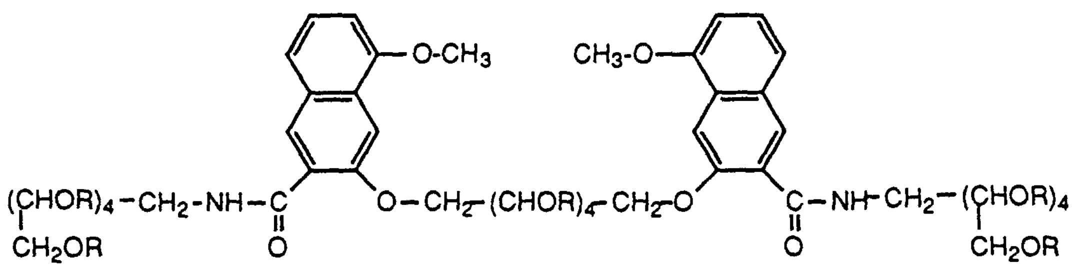

$R=SO_3Na$

1,6-Bis-O-[4-[4-O-[3-(2,3,4,5,6-penta-O-sulfo-D-glucit-1-ylcarbamoyl)-naphthalin-2-yl]-2,3-di-O-sulfo-L-threit-1-yloxy]-phenyl]-2,3,4,5-tetra-O-sulfo-galactitol Octadekanatriumsalz (Beispiel 50);

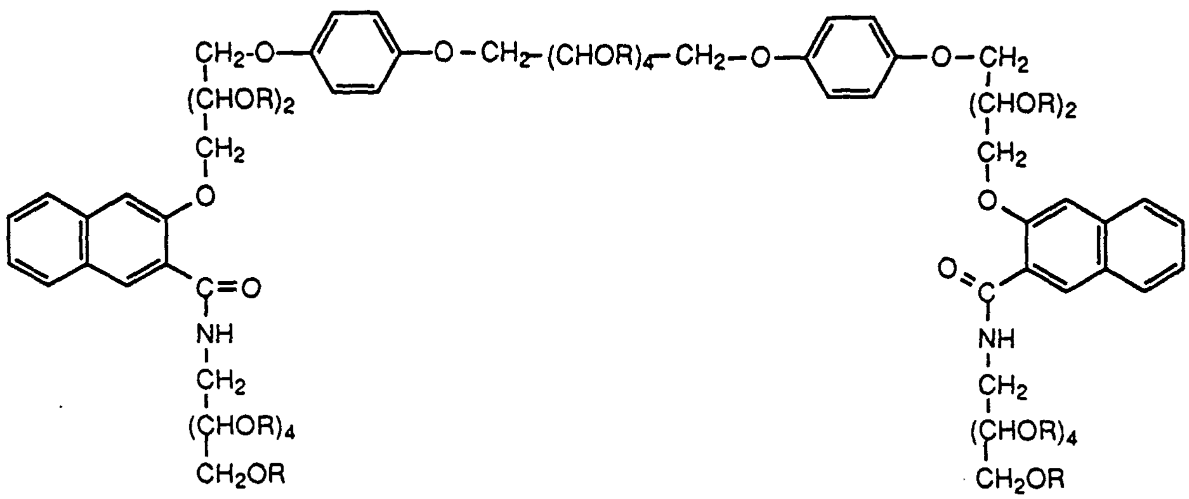

$R=SO_3Na$

1,6-Bis-O-[3-[4-O-[3-(2,3,4,5,6-penta-O-sulfo-D-glucit-1-ylcarbamoyl)-naphthalin-2-yl]-2,3-di-O-sulfo-L-threit-1-yloxy]-naphthalin-2-yl]-2,3,4,5-tetra-O-sulfo-galactitol Octadekanatriumsalz (Beispiel 54);

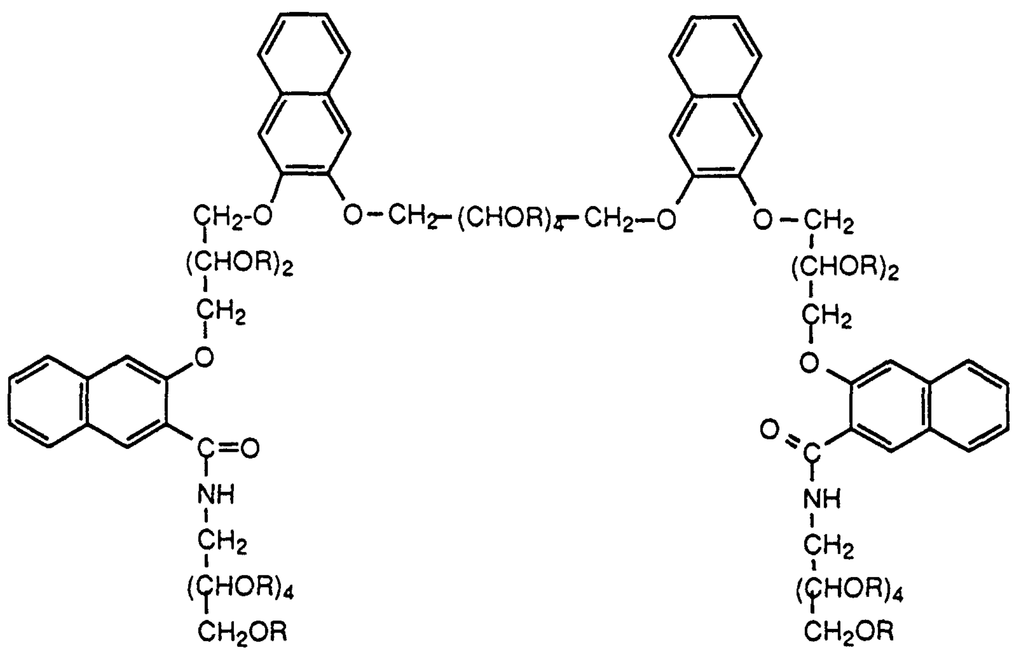

$R=SO_3Na$

1,6-Bis-O-[4-[(E)-2-[benzyl-2,6-didesoxy-6-(2,3,4,5,6-penta-O-sulfo-D-gluconoylamino)-3,4-di-O-sulfo-α-D-glucopyranosid-2-ylcarbamoyl]-vinyl]-phenyl]-2,3,4,5-tetra-O-sulfo-galactitol Octadekanatriumsalz (Beispiel 57);

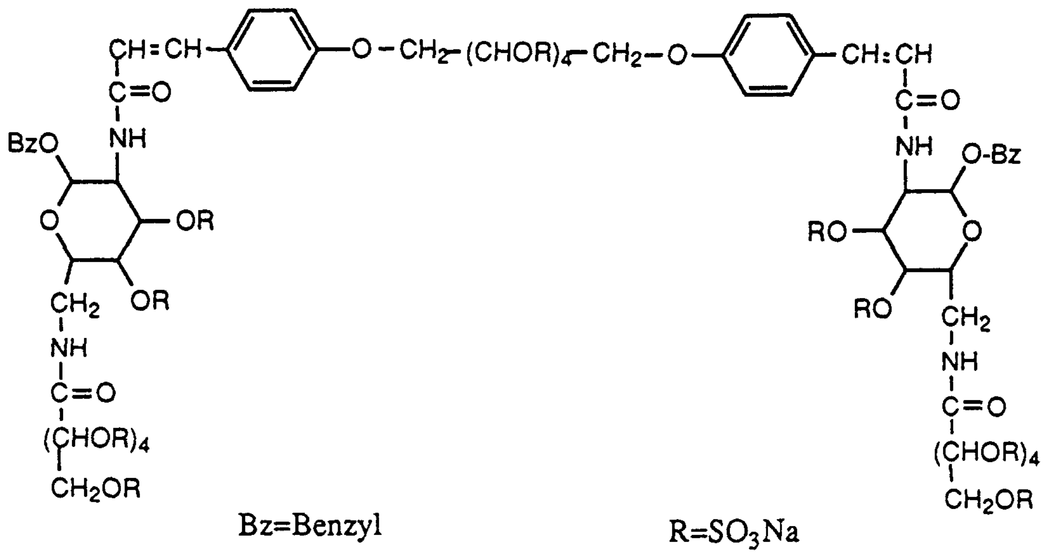

Bz=Benzyl

$R=SO_3Na$

Besonders bevorzugte Verbindungen der allgemeinen Formel I sind :

1,6-Bis-O-[4-[2-(2,3,4,5,6-penta-O-sulfo-D-glucit-1-ylcarbamoyl)-ethyl]-phenyl]-2,3,4,5-tetra-O-sulfo-galactitol Tetradekanatriumsalz (Beispiel 6),

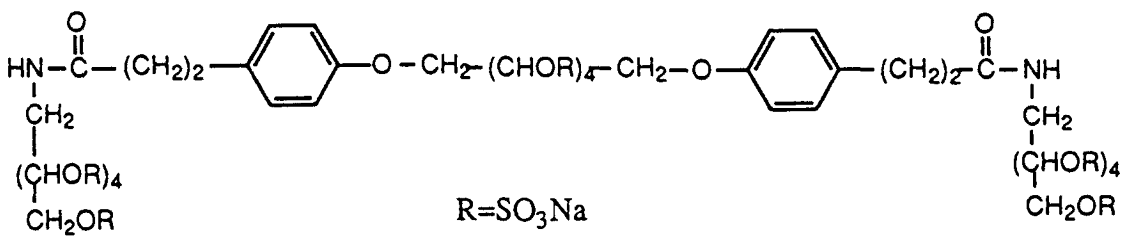

$R=SO_3Na$

1,6-Bis-O-[4-[2-[4-(2,3,4,5,6-penta-O-sulfo-D-glucit-1-ylcarbamoyl)-phenyl]-ethyl]-phenyl]-2,3,4,5-tetra-O-sulfo-galactitol Tetradekanatriumsalz (Beispiel 11),

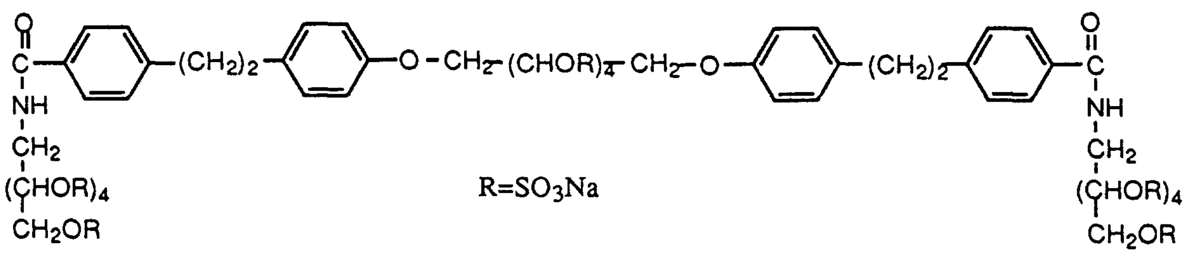

$R=SO_3Na$

1,6-Bis-O-[3-(2,3,4,5,6-penta-O-sulfo-D-glucit-1-ylcarbamoyl)-naphthalin-2-yl]-2,3,4,5-tetra-O-sulfo-galactitol Tetradekanatriumsalz (Beispiel 14),

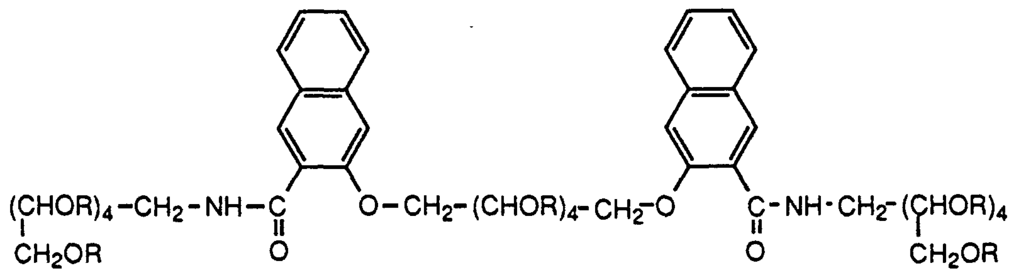

$R=SO_3Na$

1,6-Bis-O-[6-(2,3,4,5,6-penta-O-sulfo-D-glucit-1-ylcarbamoyl)-naphthalin-2-yl]-2,3,4,5-tetra-O-sulfo-galactitol Tetradekanatriumsalz (Beispiel 16),

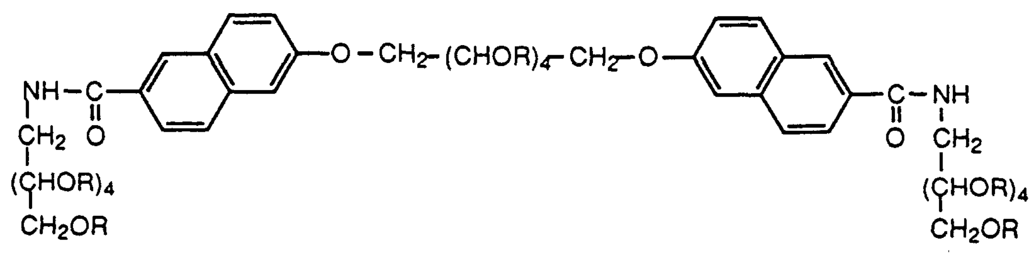

$R=SO_3Na$

1,6-Bis-O-[3-biphenyl-4-ylmethoxy-5-(2,3,4,5,6-penta-O-sulfo-D-glucit-1-ylcarbamoyl)-phenyl]-2,3,4,5-tetra-O-sulfo-galactitol Tetradekanatriumsalz (Beispiel 17),

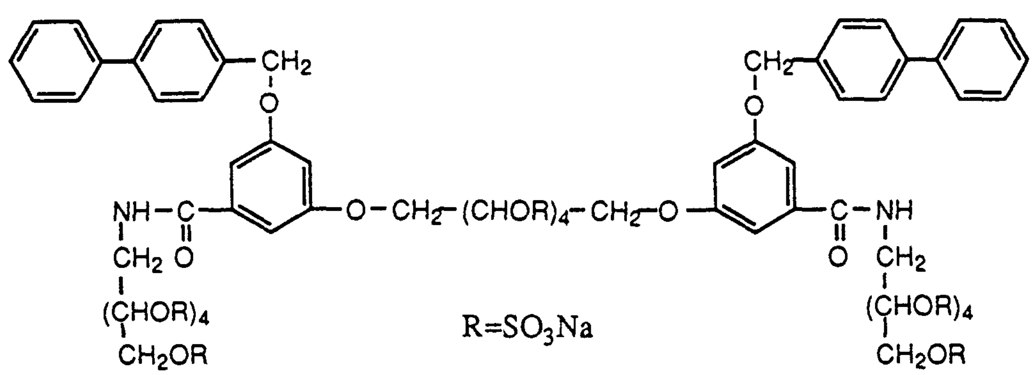

$R=SO_3Na$

1,6-Bis-O-[6-[(S)-2,3-bis-sulfooxy-propoxy]-3-(2,3,4,5,6-penta-O-sulfo-D-glucit-1-ylcarbamoyl)-naphthalin-2-yl]-2,3,4,5-tetra-O-sulfo-galactitol Octadekanatriumsalz (Beispiel 22),

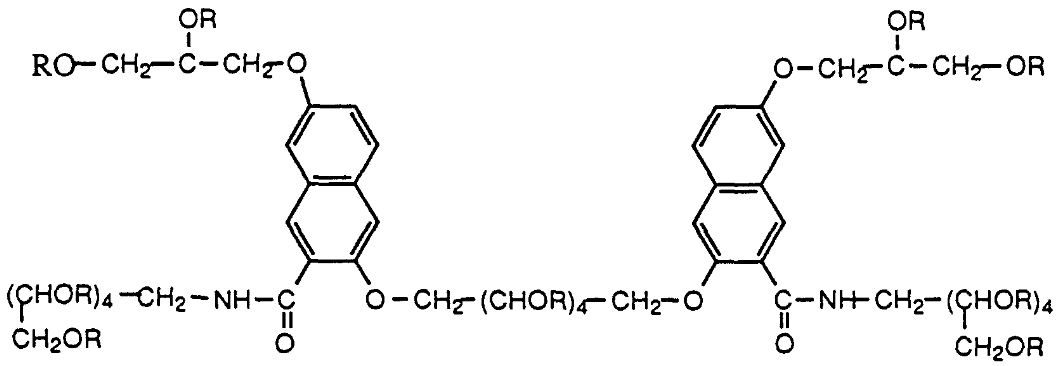

$R=SO_3Na$

1,6-Bis-O-[3-(2,3,4,5-tetra-O-sulfo-D-arabinit-1-yloxy)-naphthalin-2-yl]-2,3,4,5-tetra-O-sulfo-galactitol Dodekanatriumsalz (Beispiel 40),

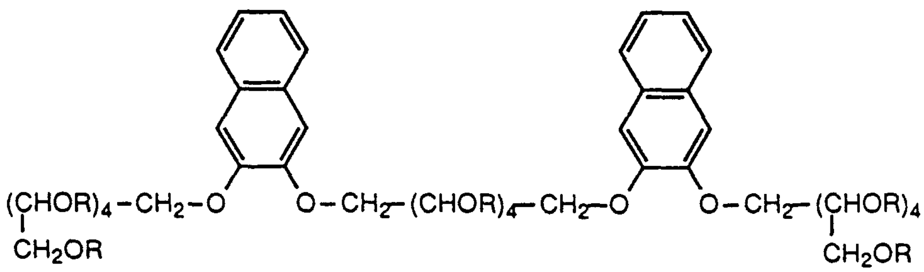

$R=SO_3Na$

2,3,4,5-Tetra-O-sulfo-1,6-bis-O-[4-[4-(2,3,4,5-tetra-O-sulfo-D-arabinit-1-yloxy)-benzyl]-phenyl]-D-mannitol Dodekanatriumsalz (Beispiel 43),

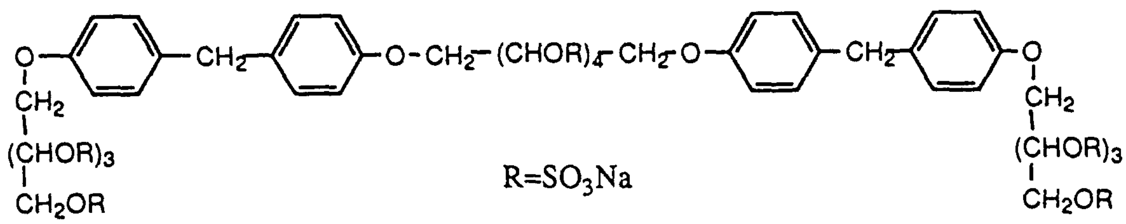

$R=SO_3Na$

1,6-Didesoxy-2,3,4,5-tetra-O-sulfo-1,6-bis-[3-(2,3,4,5-tetra-O-sulfo-D-arabinit-1-yloxy)-naphthalin-2-ylcarbonylamino]-galactitol Dodekanatriumsalz (Beispiel 45),

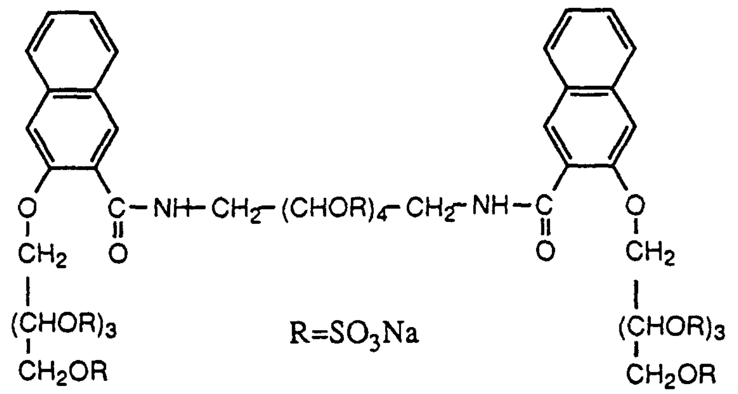

$R=SO_3Na$

1,6-Bis-O-[4-[4-O-[4-[(E)-2-(2,3,4,5,6-penta-O-sulfo-D-glucit-1-ylcarbamoyl)-vinyl]-phenyl]-2,3-di-O-sulfo-D-threit-1-yloxy]-phenyl]-2,3,4,5-tetra-O-sulfo-galactitol Octadekanatriumsalz (Beispiel 48),

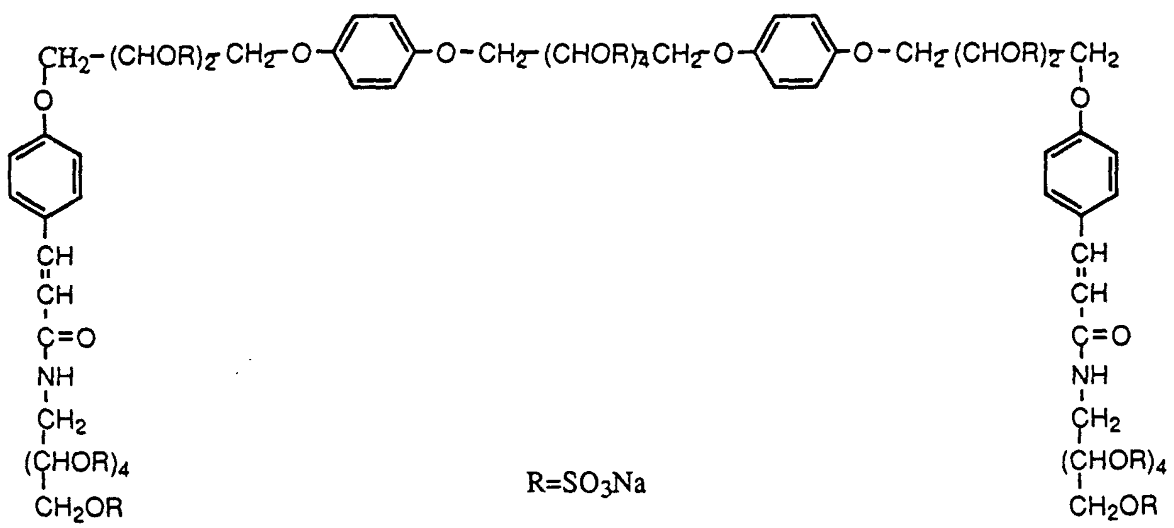

$R=SO_3Na$

1,6-Bis-O-[4-[4-O-[4-[(E)-2-[methyl-(2,3,4,5,6-penta-O-sulfo-D-glucit-1-yl)-carbamoyl]-vinyl]-phenyl]-2,3-di-O-sulfo-D-threit-1-yloxy]-phenyl]-2,3,4,5-tetra-O-sulfo-galactitol Octadekanatriumsalz (Beispiel 52),

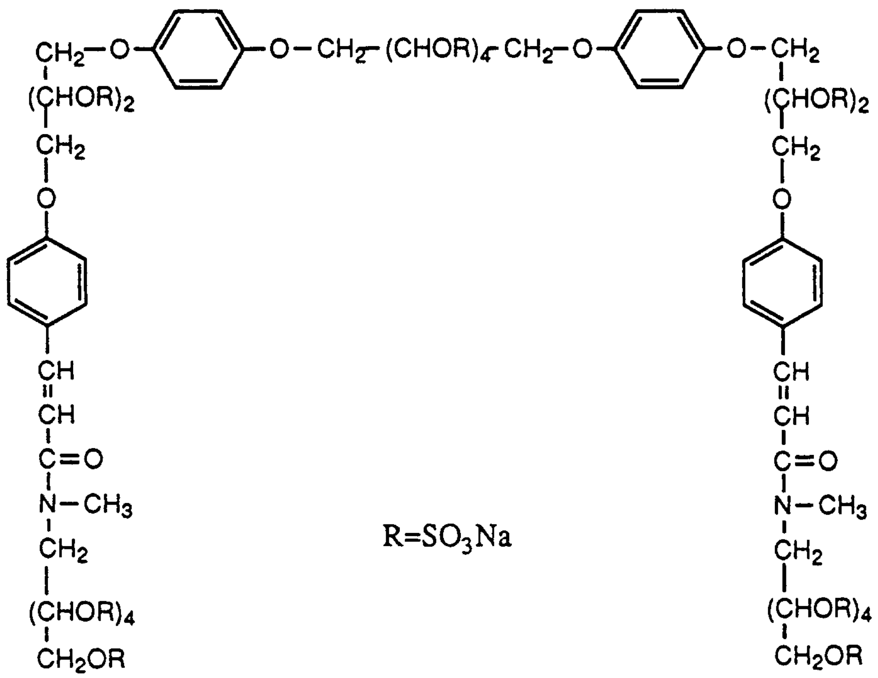

R=$SO_3Na$

1,6-Bis-O-[3-[benzyl-2,6-didesoxy-6-(2,3,4,5,6-penta-O-sulfo-D-gluconoylamino)-3,4-di-O-sulfo-α-D-glucopyranosid-2-ylcarbamoyl]-naphthalin-1-yl]-2,3,4,5-tetra-O-sulfo-galactitol Oktadekanatriumsalz (Beispiel 56),

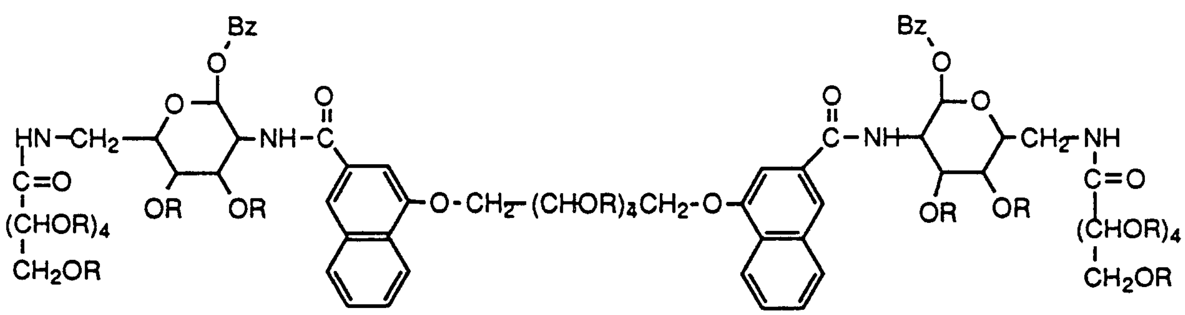

R=$SO_3Na$ Bz=Benzyl

1,6-Bis-O-[4-[2-[benzyl-2,6-didesoxy-6-(2,3,4,5,6-penta-O-sulfo-D-gluconoylamino)-3,4-di-O-sulfo-α-D-glucopyranosid-2-ylcarbamoyl]-ethyl]-phenyl]-2,3,4,5-tetra-O-sulfo-galactitol Oktadekanatriumsalz (Beispiel 58),

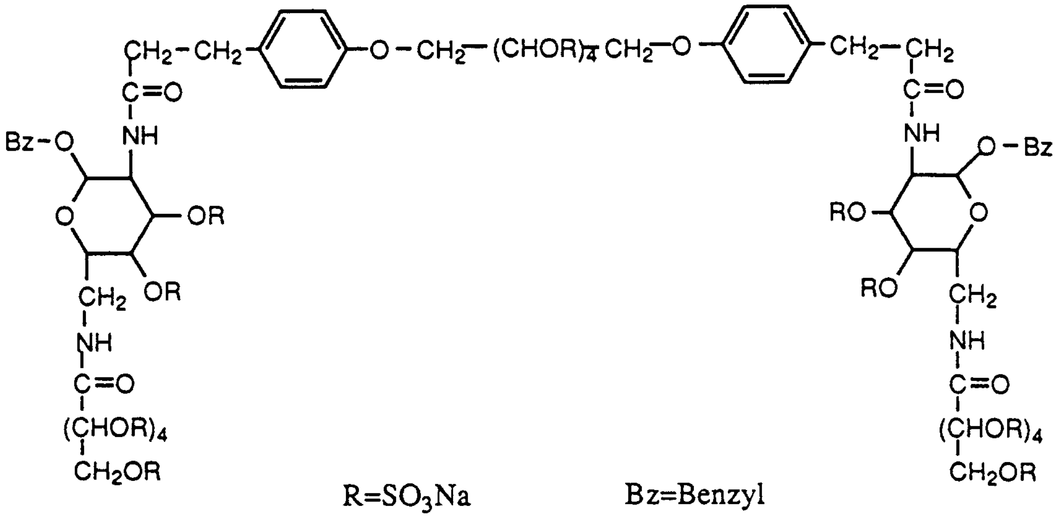

R=SO$_3$Na Bz=Benzyl

1,6-Bis-O-[6-[benzyl-2,6-didesoxy-2-[3-nitro-4-(2,3,4,5,6-penta-O-sulfo-D-glucit-1-ylamino)-benzoylamino]-3,4-di-O-sulfo-α-D-glucopyranosid-6-ylcarbamoyl]-naphthalin-2-yl]-2,3,4,5-tetra-O-sulfo-galactitol Oktadekanatriumsalz (Beispiel 59),

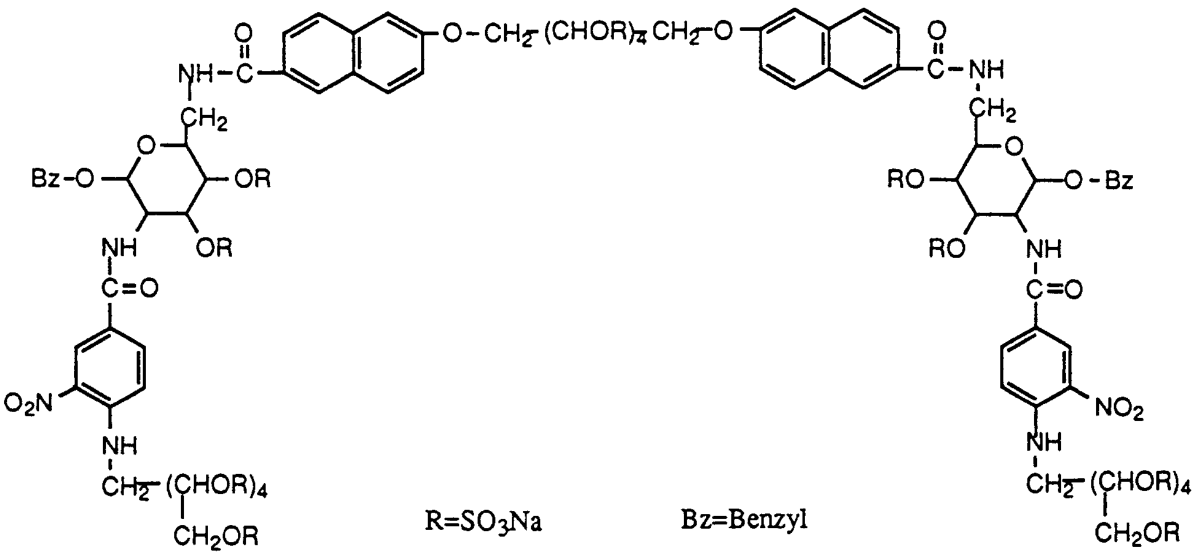

R=SO$_3$Na Bz=Benzyl

1,6-Bis-O-[4-[(E)-2-[benzyl-2,6-didesoxy-2-[4-(2,3,4,5,6-penta-O-sulfo-D-glucit-1-ylamino)-3-nitro-benzoylamino]-3,4-di-O-sulfo-α-D-glucopyranosid-6-ylcarbamoyl]-vinyl]-phenyl]-2,3,4,5-tetra-O-sulfo-galactitol Oktadekanatriumsalz (Beispiel 61).

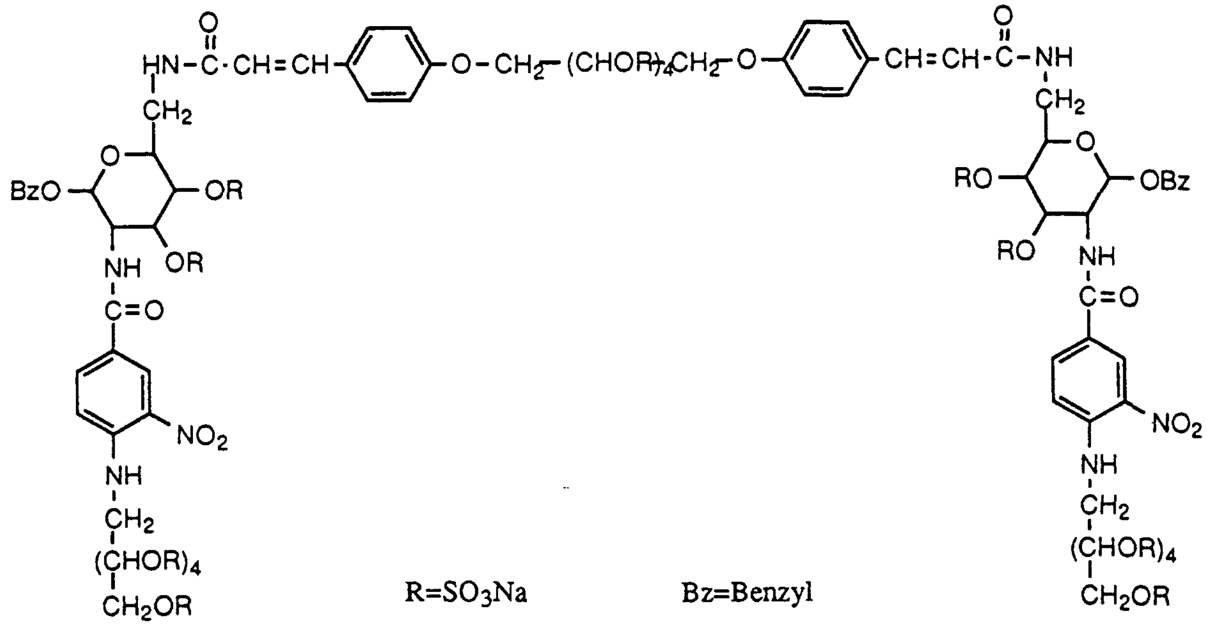

R=SO3Na Bz=Benzyl

Die eingangs definierten Verbindungen können dadurch hergestellt werden, dass man entsprechende, nicht sulfatierte Verbindung mit einem Sulfatierungsmittel umsetzt.

Die erfindungsgemässe Sulfatierung kann mittels Methoden, die für die Sulfatierung von Hydroxygruppen an sich bekannt sind, vorgenommen werden. Beispiele von Sulfatierungsmitteln sind $SO_3$ - Komplexe wie $SO_3$·Pyridin, $SO_3$·Trimethylamin, $SO_3$·Dioxan und $SO_3$·Dimethylformamid. Weitere Beispiele von Sulfatierungsmitteln sind Chlorsulfonsäure, Gemische von Chlorsulfonsäure und Schwefelsäure; und Piperidin-N-Sulfat.

Die Umsetzung erfolgt zweckmässig in einem geeigneten Lösungsmittel, insbesondere einem polaren Lösungsmittel, z.B. Dimethylformamid, Dimethylsulfoxid oder Hexamethylphosphortriamid. Die Reaktion kann bei Raumtemperatur oder bei erhöhter Temperatur, z.B. bei 20-70 °C, durchgeführt werden, wobei durch Variation von Reaktionsdauer und -temperatur der Sulfatierungsgrad beeinflusst werden kann. Der jeweils erreichte Sulfatierungsgrad kann durch HPLC abgeschätzt werden. In einer bevorzugten Ausführungsform werden durch geeignete Wahl von Reaktionsdauer und -temperatur alle oder praktisch alle freien Hydroxygruppen sulfatiert. Die Aufarbeitung des Reaktiongemisches bzw. die Isolierung des Reaktionprodukts kann nach an sich bekannten Methoden erfolgen, z.B. durch Gelfiltration oder Ultrafiltration. Zweckmässig wird das Reaktionsgemisch vor der Aufarbeitung mit einer zur Salzbildung mit den Sulfonsäuregruppen in den eingangs definierten Verbindungen befähigten, hinreichend basischen Verbindung, z.B. mit einem Alkali-metallazetat, wie Natriumazetat, versetzt und die Verbindung der Formel Ia und Ib in Salzform, z.B. als Natriumsalz, isoliert.

Die Ausgangsstoffe für das erfindungsgemässe Verfahren, d.h. die den Verbindungen der Formeln Ia und Ib entsprechenden, nicht sulfatierten Verbindungen, können wie in den nachstehenden Beispielen beschrieben oder in Analogie dazu hergestellt werden. Die unterschiedlichen Zwischenstufen, über welche Substanzen der Formeln Ia und Ib hergestellt werden, können allgemein beginnend mit bekannten oder in Analogie zu bekannten leicht zugänglichen Grundkörpern stufenweise wie folgt hergestellt werden:

Reste von Zuckeralkoholen, Didesoxyglycopyranosiden oder der Tris-(hydroxymethyl)-methyl-Rest, sowie Derivate von Zucker-Mono und Di-carbonsäuren, deren Hydroxyfunktionen frei oder durch übliche Schutzgruppen wie Acetonide, Methylenacetale, Arylmethyläther, Alkyl- oder Arylester etc. geschützt, mit Desoxystellen zu Oxiran-Ringen oder mit Carbonsäurefunktionen zu 5 oder 6 gliedrigen Laktonen geschlossen sein können, und die eine resp. zwei für die Nachfolgereaktion geeignete funktionelle Gruppen tragen, können nach unterschiedlichen Verknüpfungsmustern und in variabler Reihenfolge durch gleichzeitige Bildung einer oder mehrerer neuer Bindungen pro Reaktionsschritt, mit ebenfalls geeignet funktionalisierten aromatischen Einheiten $Y^1$-$Y^9$ oder dem Aromaten W oder bei deren Fehlen mit anderen geeigneten mono- oder difunktionellen Derivaten von Polyolen verknüpft werden. Vor der Wiederholung der Verknüpfungsreaktion müssen jeweils die zur Verknüpfungsreaktion nötigen funktionellen Gruppen durch Entfernung einer Schutzgruppe, wie auch die Reduktion einer Nitrogruppe, oder durch Einführung einer geeigneten Aktivierungsfunktion für die nächste Verknüpfungsreaktion vorbereitet werden, es sei denn, bifunktionelle Verbindungen werden selektiv nur an einer funktionellen Stelle umgesetzt. Bei Wiederholung der Verknüpfungsreaktion ist zumindest eine der zu verknüpfenden Einheiten bereits eine komplexere Verbindung, die sowohl Derivate von Polyolen als auch aromatische Untereinheiten besitzt; gegebenenfalls kann nun die erneute Verknüpfungsreaktion auch zwischen zwei aromatischen Untereinheiten durchgeführt werden. Nach Abschluss des Gerüstaufbaues werden nach Entfernung von Schutzgruppen die unsulfatierten Vorstufen der Formeln Ia und Ib, erhalten.

Ist die Verknüpfungsreaktion die Bildung einer Amidbindung, so kann diese dadurch gebildet werden, dass eine Komponente mit freier Aminofunktion mit der zweiten Komponente in Form eines aliphatischen Esters, Lactons, eines Säurechlorides oder einer nach bekannten Methoden der Peptidchemie aktivierten Säurefunktion (gemischtes Anhydrid oder Aktivester) zur Reaktion gebracht wird.

Ist die Verknüpfungsreaktion die Bildung einer Etherfunktion, so können freie Hydroxyfunktionen an einem Zuckerrest mit einem Phenol oder einem benzylischen oder allylischen Alkohol nach bekannten Methoden verknüpft werden entweder ohne vorgängige Aktivierung der Alkoholfunktion nach Mitsunobu (O. Mitsunobu, Synthesis 12, 1 (1981)), oder nach Aktivierung der Alkoholfunktion. Eine solche Aktivierung kann erfolgen durch Ueberführung in Halogenide oder Alkyl- resp. Arylsulfonate oder durch Bildung eines Epoxides mit einer benachbarten Hydroxyfunktion.

Ist die Verknüpfungsreaktion die Bildung eines Arylamines, so können diese durch bekannte Methoden der Umsetzung von Aminoglycitolen mit aktivierten Aromaten erhalten werden.

Der Aufbau aromatischer Reste mit Synthonen, die bereits-gegebenenfalls an den Hydroxyfunktionen geschützte - Derivate von Polyolen enthalten, kann auch unter Verwendung von C-C-bindungsbildenden Reaktionen durchgeführt werden. Dabei werden vorzugsweise Reaktionen verwendet, die unter milden Reaktionsbedingungen ablaufen können, wie durch Palladiumverbindungen katalysierte Vinylierungs- und Acetylenylierungs-Reatkionen von Typ der "Heck" Reaktion (R. F. Heck, Organic Reactions 27, 345 (1982)).

Die Entfernung allfällig nach dem Aufbau des Gerüstes noch vorhandener Schutzgruppen erfolgt nach der allgemein üblichen Methodik.

Zum Aufbau unsymmetrischer Verbindungen ist die Technologie der Reaktionen an einem festen Träger besonders geeignet. Dabei wird eine erste Syntheseeinheit über eine labile Funktion, z.B. eine labile Amidfunktion, an ein geeignet funktionalisiertes Harz gebunden. Nach anschliessender Freisetzung einer geeigneten funktionellen Gruppe durch Entfernung einer allfällig vorhandenen Schutzgruppe kann nun nicht nur mit einem mono- sondern auch mit einem difunktionellen Synthon im Ueberschuss selektiv eine Monoreaktion erzielt werden. Dabei bleibt in der sich am Harz aufbauenden Verbindung eine funktionelle Gruppe erhalten, und eine weitere Reaktion mit einer zweiten bifunktionellen Einheit kann direkt angeschlossen werden. Dieser Vorgang lässt sich mehrere Male wiederholen. Ein monofunktionelles Derivat eines Polyoles kann schliesslich zum Kettenabbruch verwendet werden. Eine mögliche Verknüpfungsreaktion, auf die sich dieses Prinzip anwenden lässt, ist die Bildung von Etherfunktionen. Dabei können die oben erwähnten Reaktionsbedingungen zur Herstellung von Etherbindungen Verwendung finden. Die am Harz aufgebaute Verbindung kann schliesslich durch eine spezifische Abspaltungsreaktion, z.B. unter sauren Bedingungen, vom Harz abgespalten werden; so wird nach Entfernung allfällig noch vorhandener Schutzgruppen eine unsulfatierte Vorstufe der eingangs definierten Verbindungen erhalten.

Die erfindungsgemässen Verbindungen hemmen die Migration und Proliferation von glatten Muskelzellen der Gefässwand. Sie können also zur Behandlung und/oder Prophylaxe von Krankheiten, die durch exzessive oder destruktive Proliferation von glatten Muskelzellen gekennzeichnet sind, sowie von arteriosklerotischen Gefässwandveränderungen, insbesondere zur Verhinderung von Restenose nach koronarer oder peripherer Angioplastie oder nach Bypass-Operationen, verwendet werden. Im Prinzip können diese Substanzen zur Behandlung und/oder Prophylaxe von allen Erkrankungen, bei denen Migration oder Proliferation von glatten Muskelzellen eine Rolle spielen, eingesetzt werden.

Im Gegensatz zu Heparin haben diese Verbindungen aber keine $AT_{III}$-Aktivität (Antithrombin III) und somit keine inhibierende Wirkung auf die Gerinnungsfaktoren IIa und Xa. Demzufolge ist ihre blutgerinnungshemmende Aktivität sehr viel geringer als die von Heparin, und damit ist das Blutungsrisiko bei der Therapie mit diesen Substanzen minimal.

Da Heparin-bindende Proteine bei verschiedenen Krankheiten eine wichtige Rolle spielen, können Heparin-ähnliche Substanzen, wie die erfindungsgemässen Verbindungen, zusätzlich auch zur Behandlung dieser Krankheiten eingesetzt werden: z.B. wird die Aufnahme von verschiedenen Viren (Herpes, HIV) durch solche Substanzen inhibiert, die arterielle Thrombose (vWF, Plättchenadhäsion) wird durch solche Substanzen gehemmt, die Aktivierung des Komplementsystem (z.B. bei Reperfusion) kann gemindert werden, und verschiedene Wachstumsfaktoren oder Cytokine (z.B. bFGF in Tumoren) können inhibiert werden.

Die pharmakologischen Aktivitäten der erfindungsgemässen Verbindungen können in den nachstehend beschriebenen Versuchsanordnungen gezeigt werden:

Antiproliferative Aktivität

Die antiproliferative Aktivität einer Substanz wird als $r_i$-Wert ausgedrückt, der einen Vergleichswert zu der

entsprechenden Aktivität von Heparin darstellt und der in Zellkulturen wie folgt ermittelt wurde: Glatte Muskelzellen der Ratte wurden mit einer Dichte von $8x10^3$ Zellen/Loch auf Zellkulturplatten ausgebracht , Medium: DMEM (Dulbecco modified Eagle medium) mit 10% FCS ( fetal calf serum); Kultivation bei 37°C und 5% $CO_2$. Nach 4 Stunden wurde die Zahl der adhärierten Zellen bestimmt und die zu testenden Substanzen (100 µg/ml, gelöst in $H_2O$) zugegeben. Als Kontrolle dienten a) Zellen, denen nichts zugegeben wurde, und b) Zellen, die mit Heparin (100 µg/ml) versetzt wurden. Anschliessend wurden die Zellen für 48 h inkubiert, und danach wurde wieder die Zellzahl bestimmt.

Aus diesen Werten wurde die Inhibition i des Zellwachstums, d.h. die Erniedrigung der Wachstumsrate der Zellen in Prozenten im Vergleich zur Kontrolle ausgerechnet.

$$i = 100 - \frac{\mu \text{ Substanz}}{\mu \text{ Kontrolle}} \cdot 100$$

wobei die Wachstumsrate µ errechnet wurde als

$$\mu = \frac{\Delta \ln Z}{\Delta t_{[d]}} = \ln \frac{Z_{(t2)}}{Z_{(t1)}} * \frac{1}{\Delta t_{[d]}} \qquad [d^{-1}]$$

wobei Z die Anzahl der Zellen und d die Zeit in Tagen ist.

Schliesslich wurde ri - die relative inhibitorische Aktivität - welche die Aktivität von einer Substanz (bei 100 µg/ml) im Vergleich zur Aktivität von Heparin bei gleicher Konzentration im gleichen Experiment ausdrückt, errechnet:

$$r_i = \frac{i_{\text{Substanz}}}{i_{\text{Heparin}}}$$

Blutgerinnungshemmung

Die blutgerinnungshemmende Wirkung wurde wie folgt ermittelt:

Hemmung von Thrombin oder Faktor Xa im Chromogen Substrat Test (Teien et al., Thrombosis Research 10, 399-410 (1977)): Die Bestimmung erfolgte im Cobas-Bio Centrifugal Automatic Spectrophotometer. Die verwendete Pufferlösung bestand aus 50 mM Tris-Puffer, 180 mM NaCl, 7.5 mM EDTA-$Na_2$, 1% PEG 6000 und 0.02% Tween-80, pH 8.4. Die Testlösung bestand aus 50 µl Puffer, 30 µl Antithrombin III (1U/ml, Kabi-Diagnostica) und 20 µl Plasma, das verschiedene Konzentrationen der Versuchsverbindungen enthielt. Im Analyseautomaten wurden 30 µl Probenlösung und 20 µl Wasser mit 180 µl Thrombin (1U/ml, Thrombin Reagent Roche Basel) in die Testküvette gegeben. Nach 240 Sekunden Inkubation bei 37 °C wurden 60 µl S-2238 (H-D-Phe-Pip-Arg-NH.pNA, Kabi Diagnostica, Möndal, Schweden, 0.75 mM in Wasser) und 20µl Wasser zugesetzt. Die Freisetzung von pNA (p-Nitro-anilin) wurde während 60 Sekunden bei 405 nm in 10-Sekunden-Intervallen im Vergleich zu Wasser als Blindwert verfolgt. Die Hemmwirkung wird als $IC_{50}$, d.h. als Konzentration [µg/ml] angegeben, bei der die amidolytische Aktivität von Thrombin im Vergleich zum Plasma-Kontrollwert um 50% vermindert wird.

In gleicher Weise wurde die Hemmung von Faktor Xa gemessen, wobei eine Lösung von Faktor Xa (2.8 nkat/ml und 2 mM S-2222 (Bz-CO-Ile-Glu-Arg-NH.pNA, Kabi Diagnostica) in Wasser anstelle von Thrombin bzw. S-2238 verwendet wurde.

In der nachstehenden Tabelle sind die in den vorstehend beschriebenen Versuchsanordnungen mit einer repräsentativen Anzahl Verbindungen der Formel I erhaltenen Aktivitätsdaten angeführt:

| Beispiel | Antiproliferative Aktivität ri | Antikoagulative Aktivität $IC_{50}$ [µg/ml] | |
|---|---|---|---|
| | | Thrombin | Faktor Xa |
| 6 | 0,7 | > 1000 | > 1000 |
| 11 | 2,2 | > 1000 | > 1000 |
| 14 | 1,5 | > 1000 | > 1000 |
| 16 | 2,1 | > 1000 | > 1000 |
| 17 | 1,5 | > 1000 | > 1000 |
| 22 | 2,1 | > 1000 | > 1000 |
| 40 | 1,4 | > 1000 | > 1000 |
| 43 | 1,3 | > 1000 | > 1000 |

(fortgesetzt)

| Beispiel | Antiproliferative Aktivität ri | Antikoagulative Aktivität $IC_{50}$ [μg/ml] | |
|---|---|---|---|
| | | Thrombin | Faktor Xa |
| 45 | 1,4 | > 1000 | > 1000 |
| 48 | 2,7 | > 1000 | > 1000 |
| 52 | 0,8 | > 1000 | > 1000 |
| 56 | 1,7 | > 1000 | > 1000 |
| 58 | 1,8 | > 1000 | > 1000 |
| 59 | 1,8 | > 1000 | > 1000 |
| 61 | 2,4 | >1000 | > 1000 |
| Heparin | 1,0 | 1,9 | 2,7 |

<u>In vivo Assay zur Bestimmung der antiproliferativen Aktivität der erfindungsgemässen Verbindungen in verletzten Carotiden der Ratte</u>

Die linken Carotiden von männlichen Wistar Kyoto Ratten (300 - 400g) wurden nach erfolgter Narkose mit einem 2F Embolektomiekatheter verletzt, indem der Katheter im aufgepumpten Zustand dreimal durch das Gefäß gezogen wurde. Nach der Wundversorgung wurden die Tiere mit Standardfutter und Wasser ad libidum paarweise gehalten.

Die Verbindungen wurden in Konzentrationen von 0.3 - 1mg/kg/h i.v. appliziert. Hierzu wurde den Tieren während der Narkose unter die Rückenhaut eine osmotische Minipumpe implantiert, die mit der Vena jugularis verbunden wurde. So konnten die Verbindungen während der gesamten Versuchsdauer von 14 Tagen konstant verabreicht werden.

Nach 14 Tagen hatte sich proliferatives Gewebe gebildet (Neointima), dessen Größe sich morphometrisch an histologischen Querschnitten bestimmen ließ. Hierzu wurden die Ratten getötet und mit Glutaraldehyd perfusionsfixiert.

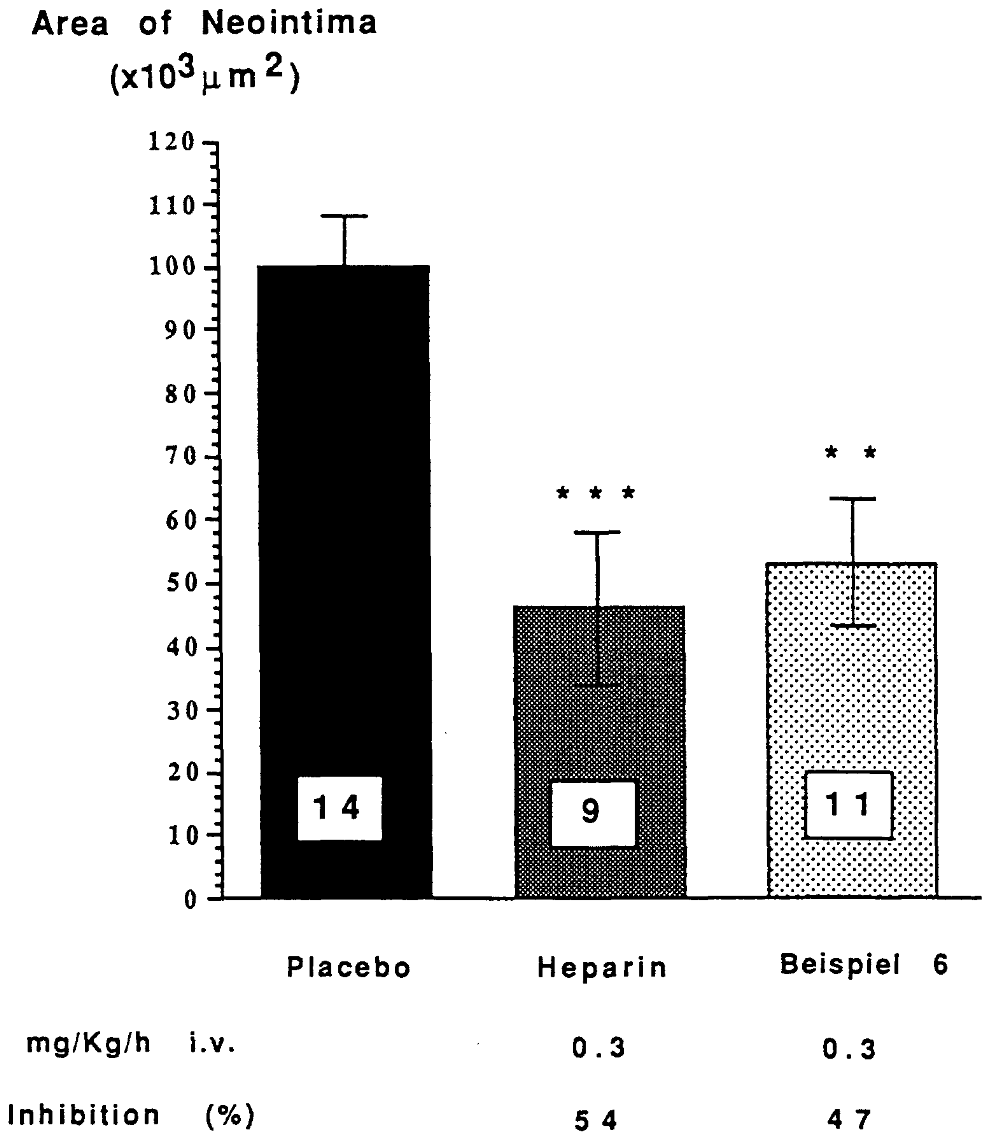


Die Querschnittsfläche der Neointima in Rattencarotiden 14 Tage nach Ballonisierung war nach i.v. Applikation von Beispiel 6 (0.3mg/kg/h) signifikant reduziert (p<0.01, t-Test). Damit erreichte Beispiel 6 einen vergleichbaren Effekt wie gleich konzentriertes Heparin (Die Anzahl der Tiere n ist in der Abbildung angegeben; Mittelwerte ± SEM).

Die Versuchsresultate zeigen, dass die erfindungsgemässen Verbindungen eine antiproliferative Wirkung aufweisen, die derjenigen von Heparin entspricht (oder nahekommt) oder grösser ist, im Gegensatz zu Heparin jedoch keine oder eine viel geringere anti-Koagulationswirkung zeigen.

Die Arzneimittel auf der Basis der erfindungsgemässen Verbindungen können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, verabreicht werden. Die Verabreichung erfolgt jedoch vorzugsweise parenteral, z.B. in Form von Injektionslösungen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z. B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Bei enteraler Verabreichung kann die Resorption des Wirkstoffs mit Hilfe von Liposomen erhöht werden.
Die Dosierung des Wirkstoffes kann innerhalb weiter Grenzen variieren und ist natürlich in jedem einzelnen Fall den individuellen Gegebenheiten anzupassen. Im allgemeinen dürfte bei parenteraler Verabreichung eine Dosis von etwa 0,1 bis 100 mg/kg, vorzugsweise von etwa 1,5 bis 15 mg/kg, pro Tag für den Erwachsenen angemessen sein, wobei aber die soeben angegebene obere Grenze auch überschritten werden kann, wenn sich dies als angezeigt erweisen sollte.
Die Erfindung wird durch nachstehende Beispiele weiter erläutert.

Beispiel 1

A. 1,024 g 4,4'-Dioxo-5,5'-(2-hydroxy-propan-1,3-diyldioxy)-di-4H-1-benzopyran-2-carbonsäure Dinatriumsalz) (Neth. Appl. NL 6,603,997, Preparation of dichromonyl derivatives, Fisons Pharmaceuticals Ltd., 26. September 1966, CA 67:100002d) wurden in 15 ml Acetonitril und 5 ml Dimethylformamid suspendiert, anschliessend unter Feuchtigkeitsausschluss bei 0-5 °C mit 0,5 ml 4-Methylmorpholin versetzt und während 10 Minuten intensiv gerührt. Hierauf wurden 0,704 g 2-Chlor-4,6-dimethoxy-1,3,5-triazin fest hinzugefügt und weitere 2 Stunden bei 0-5 °C gerührt. Anschliessend wurden 0,73 g D-Glucamin gefolgt von 10 ml Dimethylformamid zugesetzt und weitere 18 Stunden bei Raumtemperatur gerührt. Hierauf wurde das Reaktionsgemisch mehrmals mit Wasser verdünnt und mehrmals unter vermindertem Druck bei 40-50 °C eingedampft und der Rückstand mit 20 ml Pyridin und 20 ml Acetanhydrid versetzt und weitere 60 Stunden bei Raumtemperatur gerührt. Dann wurde dieses Reaktionsgemisch erneut eingeengt, mit Eiswasser und 1 N Salzsäure versetzt und mit Methylenchlorid extrahiert. Die vereinigten Methylenchloridphasen wurden über Magnesiumsulfat getrocknet, eingeengt und mit Essigester an Kieselgel chromatographiert. Die dabei erhaltene Produktfraktion wurde in 11 ml Methanol und 3 ml Tetrahydrofuran gelöst und mit 0,75 ml 1 molarer Natriummethylat-Lösung in Methanol versetzt. Das Reaktionsgemisch wurde während 2 Stunden bei Raumtemperatur gerührt. Der dabei gebildete Niederschlag wurde abfiltriert, mit Methanol und Ether gewaschen und getrocknet. So wurde 5-[2-Hydroxy-3-[2-(D-glucit-1-ylcarbamoyl)-4-oxo-4H-1-benzopyran-5-yloxy]-propoxy]-4-oxo-4H-1-benzopyran-2-carbonsäure D-glucit-1-ylamid als farbloses Pulver erhalten, MS: *m*/*z* 795,4 ($[M+H]^+$).
B. Eine Suspension von 3,2 g 5-[2-Hydroxy-3-[2-(D-glucit-1-ylcarbamoyl)-4-oxo-4H-1-benzopyran-5-yloxy]-propoxy]-4-oxo-4H-1-benzopyran-2-carbonsäure D-glucit-1-ylamid und 12,0 g Schwefeltrioxidtrimethylamin-komplex in 40 ml absolutem Dimethylformamid wurde 18 Stunden bei 70 °C gerührt. Nach Abkühlen wurde mit 10 g Natriumacetat und 50 ml Wasser versetzt, eingedampft, mehrmals mit Wasser versetzt und erneut eingedampft. Der so erhaltene Rückstand wurde in 300 ml absolutem Methanol aufgeschlämmt und intensiv gemischt. Der unlösliche Rückstand wurde abfiltriert, im Vakuum getrocknet, dann in Wasser aufgenommen und an SP Sephadex® C-25 chromatographiert. Die natriumsulfatfreien Produktfraktionen wurden lyophilisiert und ergaben 4-Oxo-5-[3-[4-oxo-2-(2,3,4,5,6-penta-O-sulfo-D-glucit-1-ylcarbamoyl)-4H-1-benzopyran-5-yloxy]-2-sulfooxypropoxy]-4H-1-benzopyran-2-carbonsäure 2,3,4,5,6-penta-O-sulfo-D-glucit-1-ylamid Undekanatriumsalz, $[\alpha]_D^{20}$ -4,8° (*c* 0,6; Wasser).

Beispiel 2

A. 184 mg Cyanurchlorid, 543,5 mg D-Glucamin und 450 mg Kaliumkarbonat wurden während dreier Tage in 10 ml Wasser bei Raumtemperatur gerührt. Das so erhaltene Rohprodukt wurde hierauf an LiChroprep RP-18 Kieselgel mit Wasser und anschliessend mit Wasser mit ansteigendem Anteil an Methanol chromatographiert. Die relevanten Fraktionen wurden lyophilisiert; so wurde N,N',N"-(1,3,5-Triazin-2,4,6-triyl)-tris-(1-amino-1-desoxy-D-glucitol) als farbloses Pulver erhalten, MS: *m*/*z* 619,2 ($[M+H]^+$).
B. Das oben erhaltene N,N',N"-(1,3,5-Triazin-2,4,6-triyl)-tris-(1-amino-1-desoxy-D-glucitol) wurde analog Beispiel 1.B. zu N,N',N"-(1,3,5-Triazin-2,4,6-triyl)-tris-(1-amino-1-desoxy-2,3,4,5,6-penta-O-sulfo-D-glucitol) Pentadekanatriumsalz umgesetzt, $[\alpha]_D^{20}$ -1,4° (*c* 0,9; Wasser), MS: *m*/*z* 2126,0 (rekonstruiertes M), (Tetradekanatriumsalzmonosulfonsäure).

Beispiel 3

A. 2,5 g 2,3:4,5-Di-O-isopropyliden-1,6-bis-O-(4-methyl-phenylsulfonyl)-galactitol (E.P. 56575 A1, Baeyer AG/ CA 98(7)(1982):54399d), 2,4 g (E)-3-(4-Hydroxy-phenyl)-acrylsäuremethylester und 1,85 g fein gemahlenes wasserfreies Kaliumkarbonat wurden in 30 ml Dimethylformamid suspendiert und während 18 Stunden bei 130 °C unter Argon gerührt. Anschliessend wurde das Reaktionsgemisch mit Wasser verdünnt. Die gebildeten Kristalle wurden abfiltriert und anschliessend mit 5% Ether in Methylenchlorid an Kieselgel chromatographert. So wurde 2,3:4,5-Di-O-isopropyliden-1,6-bis-O-[(E)-4-(2-methoxycarbonyl-vinyl)-phenyl]-galactitol in Form farbloser Kristalle erhalten. MS: *m*/*z* 582 ((M]$^+$).

B. 1,89 g 2,3:4,5-Di-O-isopropyliden-1,6-bis-O-[(E)-4-(2-methoxycarbonyl-vinyl)-phenyl]-galactitol, 30 ml Methanol und 30 ml konzentrierte wässrige Natronlauge wurden bei 110 °C 18 Stunden unter Rückfluss erwärmt. Hierauf wurde der grösste Teil des Methanol unter vermindertem Druck abdestilliert. Der Rückstand wurde mit Eiswasser verdünnt und mit IN wässriger Salzsäure angesäuert. Die dabei gebildeten Kristalle wurden abfiltriert und ergaben 1,6-Bis-O-[4-[(E)-2-carboxy-vinyl]-phenyl]-2,3 :4,5-di-O-isopropyliden-galactitol in Form farbloser Kristalle , MS: *m*/*z* 554 ([M]$^+$).

C. 0,28 g 1,6-Bis-O-[4-[(E)-2-carboxy-vinyl]-phenyl]-2,3:4,5-di-O-isopropyliden-galactitol in 5 ml Dimethylformamid wurden unter Feuchtigkeitsaus-schluss bei 0 - 5 °C mit 0,1 ml 4-Methylmorpholin versetzt und während 10 Minuten intensiv gerührt. Hierauf wurden 0,20 g 2-Chlor-4,6-dimethoxy-1,3,5-triazin fest hinzugefügt und weitere 2 Stunden bei 0 - 5 °C gerührt. Anschliessend wurde 0,19 g D-Glucamin zugesetzt und es wurde weitere 18 Stunden bei Raumtemperatur gerührt. Hierauf wurde das Reaktionsgemisch mehrmals mit Wasser verdünnt und unter vermindertem Druck bei 40 - 50 °C eingedampft, mit 6 ml Wasser und 0,5 ml Triäthylamin versetzt, erwärmt und filtriert; dabei wurde 1,6-Bis-[4-[(E)-2-D-glucit-1-ylcarbamoyl-vinyl]-phenyl]-2,3:4,5-di-O-isopropyliden-galactitol als farbloses Pulver erhalten, MS: *m*/*z* 881,6 ([M+H]$^+$).

D. 1,0 g 1,6-Bis-[4-[(E)-2-D-glucit-1-ylcarbamoyl-vinyl]-phenyl]-2,3:4,5-di-O-isopropyliden-galactitol wurden in 17 ml Dioxan, 2 ml Trifluoressigsäure und 5 ml Wasser suspendiert und 4 Stunden bei 110 °C unter Rückfluss erwärmt. Hierauf wurde das Reaktionsgemisch zur Trockene eingeengt; dabei resultierte 1,6-Bis-[(E)-4-(2-D-glucit-1-ylcarbamoyl-vinyl)-phenyl]-galactitol als farbloser Festkörper, welcher direkt in die nächste Stufe eingesetzt wurde.

E. Das oben erhaltene 1,6-Bis-[(E)-4-(2-D-glucit-1-ylcarbamoyl-vinyl)-phenyl]-galactitol wurde analog Beispiel 1.B. zu 1,6-Bis-[4-[(E)-2-(2,3,4,5,6-penta-O-sulfo-D-glucit-1-ylcarbamoyl)-vinyl]-phenyl]-2,3,4,5-tetra-O -sulfo-galactitol Tetradekanatriumsalz, $[\alpha]_D^{20}$ -1,7° (*c* 0,7; Wasser), MS: *m*/*z* 2229,0 (rekonstruiertes M), umgesetzt.

Beispiel 4

A. 0,69 g 4'-Hydroxy-biphenyl-4-carbonsäuremethylester und 0,44 g fein gemahlenes Kaliumkarbonat, suspendiert in 2,5 ml Dimethylformamid wurden mit 0,54 g 3,4-O-Methoxymethylen-2,5-O-methylen-1,6-bis-O-(4-methylphenylsulfonyl)-D-mannitol (B. Lamm et al., Acta Chem. Scand. B 41, 202 (1987)) versetzt. Das Reaktionsgemisch wurde bei 130 °C während 18 Stunden unter Argon gerührt. Anschliessend wurde die Reaktionslösung auf Wasser gegossen. Der ausgefallene Niederschlag wurde abfiltriert und mit Methylenchlorid/Methanol an Kieselgel chromatographiert. Dabei resultierte 1,6-Bis-O-(4'-methoxycarbonyl-biphenyl-4-yl)-3,4-O-methoxymethylen-2,5-O-methylen-D-mannitol als farbloser Festkörper, MS: *m*/*z* 656 ([M]$^+$).

B. 1,0 g 1,6-Bis-O-(4'-methoxycarbonyl-biphenyl-4-yl)-3,4-O-methoxymethylen-2,5-O-methylen-D-mannitol, gelöst in 20 ml Methanol, wurden mit 6 ml konz. Schwefelsäure versetzt und 16 Stunden unter Rückfluss erhitzt. Anschliessend wurde das Reaktionsgemisch auf Wasser gegossen, und die ausgefallenen Kristalle wurden abfiltriert. Dabei resultierte 1,6-Bis-O-(4'-methoxycarbonyl-biphenyl-4-yl)-2,5-O-methylen-D-mannitol als farbloser Festkörper, MS: *m*/*z* 614 ([M]$^+$).

C. 0,67 g 1,6-Bis-O-(4'-methoxycarbonyl-biphenyl-4-yl)-2,5-O-methylen-D-mannitol wurden in 15 ml konzentrierter Natriumhydroxidlösung und 15 ml Methanol während 16 Stunden unter Rückfluss erwärmt; hierauf wurde eingedampft, mit verdünnter Salzsäure angesäuert. Der dabei gebildete Niederschlag von 1,6-Bis-O-(4'-carboxy-biphenyl-4-yl)-2,5-O-methylen-D-mannitol wurde abfiltriert, in 5 ml Essigsäureanhydrid und 5 ml Pyridin gelöst und 16 Stunden bei Raumtemperatur gerührt. Nun wurde erneut eingedampft, auf verdünnte Salzsäure gegossen und abfiltriert; dabei resultierte 3,4-Di-O-acetyl-1,6-bis-O-(4'-carboxy-biphenyl-4-yl)-2,5-0-methylen-D-mannitol in Form farbloser Kristalle, MS: *m*/*z* 669,4 ([M-H]$^-$).

D. 0,78 g 3,4-Di-O-acetyl-1,6-bis-O-(4'-carboxy-biphenyl-4-yl)-2,5-O-methylen-D-mannitol in 15 ml Dimethylformamid wurden unter Feuchtigkeitsausschluss bei 0 - 5 °C mit 0,26 ml 4-Methylmorpholin versetzt und während 10 Minuten intensiv gerührt. Hierauf wurde 0,47 g 2-Chlor-4,6-dimethoxy-1,3,5-triazin fest hinzugefügt, und das Reaktionsgemisch wurde weitere 2 Stunden bei 0 - 5 °C gerührt. Anschliessend wurde 0,49 g D-Glucamin zugesetzt, und das Reaktionsgemisch wurde weitere 18 Stunden bei Raumtemperatur gerührt. Hierauf wurde mehrmals mit Wasser verdünnt und unter vermindertem Druck bei 40 - 50 °C eingedampft, mit 15 ml Pyridin und 15 ml Acetanhydrid versetzt und weitere 18 Std. gerührt. Dann wurde das Reaktionsgemisch erneut eingeengt, mit Eiswasser und 1 N Salzsäure versetzt. Der so erhaltene Niederschlag wurde abfiltriert, in Methylenchlorid gelöst, mit Wasser gewaschen, über Magnesiumsulfat getrocknet, eingeengt und mit Methylenchlorid/Methanol an Kieselgel chromatographiert. Dabei resultierte 3,4-Di-O-acetyl-1,6-bis-[4'-(2,3,4,5,6-penta-0-acetyl-D-glucit-1-ylcarbamoyl)-biphenyl-4-yl]-2,5-O-methylen-D-mannitol als farbloser Festkörper, MS: *m*/*z* 1417,4 ([M+H]$^+$).

E. 1,2 g 3,4-Di-O-acetyl-1,6-bis-[4'-(2,3,4,5,6-penta-O-acetyl-D-glucit-1-ylcarbamoyl)-biphenyl-4-yl]-2,5-O-methylen-D-mannitol wurden in 25 ml Methanol und 5 ml Tetrahydrofuran gelöst und mit 1,2 ml 1 molarer Natriummethylat-Lösung in Methanol versetzt. Das Reaktionsgemisch wurde während 2,5 Stunden bei Raumtemperatur gerührt. Der dabei gebildete Niederschlag wurde abfiltriert und mit Methanol gewaschen. So wurde 1,6-Bis-O-(4'-D-glucit-1-ylcarbamoyl-biphenyl-4-yl)-2,5-O-methylen-D-mannitol in Form farbloser Kristalle erhalten und direkt in die folgende Reaktionsstufe eingesetzt.

F. Das oben erhaltene 1,6-Bis-O-(4'-D-glucit-1-ylcarbamoyl-biphenyl-4-yl)-2,5-O-methylen-D-mannitol wurde analog Beispiel 1.B. zu 1,6-Bis-[4'-(2,3,4,5,6-penta-O-sulfo-D-glucit-1-ylcarbamoyl)-biphenyl-4-yl]-2,5-O-methylen-3,4-di-0-sulfo-D-mannitol Dodekanatriumsalz, $[\alpha]_D^{20}$ -7,6° (c 0,7; Wasser), MS: *m*/*z* 2137,0 (rekonstruiertes M), umgesetzt.

Beispiel 5

A. 2,92 g 4'-Hydroxy-biphenyl-4-carbonsäuremethylester und 1,77 g fein gemahlenes Kaliumkarbonat, suspendiert in 25 ml Dimethylformamid, wurden mit 2,0 g 2,3-O-Isopropyliden-1,4-bis-O-(4-methyl-phenylsulfonyl)-L-threitol versetzt. Das Reaktionsgemisch wurde bei 130 °C während 18 Stunden unter Argon gerührt. Anschliessend wurde die Reaktionslösung auf Eiswasser gegossen, der ausgefallene Niederschlag wurde abfiltriert und mit Methylenchlorid/Methanol an Kieselgel chromatographiert. Dabei resultierte 2,3-O-Isopropyliden-1,4-bis-O-(4'-methoxycarbonyl-biphenyl-4-yl)-L-threitol als farbloser Festkörper, MS: *m*/*z* 582 ($[M]^+$).

B. 1,77 g 2,3-O-Isopropyliden-1,4-bis-O-(4'-methoxycarbonyl-biphenyl-4-yl)-L-threitol wurden in 40 ml Methanol und 40 ml konzentrierter Natriumhydroxidlösung während 18 Stunden unter Rückfluss erwärmt. Anschliessend wurde das Methanol abdestilliert. Der Rückstand wurde mit Eiswasser versetzt, angesäuert, und der gebildete Niederschlag wurde abfiltriert; dabei wurde 1,4-Bis-O-(4'-carboxy-biphenyl-4-yl)-2,3-O-isopropyliden-L-threitol als farbloser Festkörper erhalten, MS: *m*/*z* 554 ($[M]^+$).

C. 1,5 g 1,4-Bis-O-(4'-carboxy-biphenyl-4-yl)-2,3-O-isopropyliden-L-threitol in 30 ml Dimethylformamid wurden unter Feuchtigkeitsausschluss bei 0 - 5 °C mit 0,6 ml 4-Methylmorpholin versetzt und während 10 Minuten intensiv gerührt. Hierauf wurden 1,04 g 2-Chlor-4,6-dimethoxy-1,3,5-triazin fest hinzugefügt und weitere 2 Stunden bei 0 - 5 °C gerührt. Anschliessend wurden 1,07 g D-Glucamin zugesetzt und weitere 18 Stunden bei Raumtemperatur gerührt. Hierauf wurde das Reaktionsgemisch mehrmals mit Wasser verdünnt und unter vermindertem Druck bei 40 - 50 °C eingedampft, mit 1 ml Triethylamin und 5 ml Wasser versetzt, kurz aufgekocht, und der unlösliche Niederschlag wurde abfiltriert. Dabei resultierte 1,4-Bis-O-(4'-D-glucit-1-ylcarbamoyl-biphenyl-4-yl)-2,3-O-isopropyliden-L-threitol als farbloser Festkörper, MS: *m*/*z* 881,2 ($[M+H]^+$).

D. 1,0 g 1,4-Bis-O-(4'-D-glucit-1-ylcarbamoyl-biphenyl-4-yl)-2,3-O-isopropyliden-L-threitol wurden in 15 ml Dioxan, 1,5 ml Trifluoressigsäure und 5 ml Wasser suspendiert und 4 Stunden bei 110 °C unter Rückfluss erwärmt. Anschliessend wurde das Reaktionsgemisch zur Trockene eingeengt. Dabei resultierte 1,4-Bis-O-(4'-D-glucit-1-ylcarbamoyl-biphenyl-4-yl)-L-threitol als farbloser Festkörper, MS: *m*/*z* 841,6 ($[M+H]^+$).

E. Das oben erhaltene 1,4-Bis-O-(4'-D-glucit-1-ylcarbamoyl-biphenyl-4-yl)-L-threitol wurde analog Beispiel 1.B. zu 1,4-Bis-O-[4-(2,3,4,5,6-penta-O-sulfo-D-glucit-1-ylcarbamoyl)-biphenyl-4-yl]-2,3-di-O-sulfo-L-threitol Dodekanatrium-salz , $[\alpha]_D^{20}$ +0,85° (*c* 0,7; Wasser), MS: *m*/*z* 2065,0 (rekonstruiertes M), umgesetzt.

Beispiel 6

A. 0,6 g 1,6-Bis-O-[4-[(E)-2-(2,3,4,5,6-penta-O-sulfo-D-glucit-1-ylcarbamoyl)-vinyl]-phenyl]-2,3,4,5-tetra-O-sulfo-galactitol Tetradekanatriumsalz (Beispiel 3.E.) wurden in 9 ml destilliertem Wasser unter Zusatz von 80 mg Palladium auf Kohle (10%) in einer Wasserstoffatmosphäre erschöpfend hydriert. Anschliessend wurde das Reaktionsgemisch über einen 0,8 μ Cellulosefilter filtriert und das Filtrat lyophilisiert; dabei wurde 1,6-Bis-O-[4-[2-(2,3,4,5,6-penta-O-sulfo-D-glucit-1-ylcarbamoyl)-ethyl]-phenyl]-2,3,4,5-tetra-O-sulfo-galactitol Tetradekanatriumsalz , $[\alpha]_D^{20}$ -3,2° (*c* 0,7; Wasser), MS: *m*/*z* 2233,0 (rekonstruiertes M), erhalten.

Beispiel 7

A. 2,0 g 2,3-O-Isopropyliden-1,4-bis-O-(4-methyl-phenylsulfonyl)-L-threitol, 2,3 g (E)-3-(4-Hydroxy-phenyl)-acrylsäuremethylester und 1,77 g fein gemahlenes wasserfreies Kaliumkarbonat wurden in 30 ml Dimethylformamid suspendiert. Das Reaktionsgemisch wurde während 18 Stunden bei 130 °C unter Argon gerührt. Anschliessend wurde das Reaktionsgemisch mit Wasser verdünnt und mit Methylenchlorid extrahiert. Die vereinigten Methylenchloridphasen wurden über Magnesiumsulfat getrocknet und eingeengt. Der Rückstand wurde mit 5% Ether in Methylenchlorid an Kieselgel chromatographert. So wurde 2,3-O-Isopropyliden-1,4-bis-[4-[(E)-2-methoxycarbonyl-vinyl]-phenyl]-L-threitol in Form farbloser Kristalle erhalten, MS: *m*/*z* 482 ($[M]^+$).

B. 0,24 g 2,3-O-Isopropyliden-1,4-bis-[4-[(E)-2-methoxycarbonyl-vinyl]-phenyl]-L-threitol wurde in 2 ml Acetonitril und 3 ml 2 N Natriumhydroxidlösung während 70 Stunden bei Raumtemperatur gerührt. Anschliessend wurde

das Acetonitril abdestilliert. Der Rückstand wurde mit Eiswasser versetzt, angesäuert, und der gebildete Niederschlag wurde abfiltriert. Dabei wurde 1,4-O-Bis-[4-[(E)-2-carboxy-vinyl]-phenyl]-2,3-O-isopropyliden-L-threitol als farbloser Festkörper erhalten, MS: *m/z* 454 ([M]$^+$).

C. 1,0 g 1,4-O-Bis-[4-[(E)-2-carboxy-vinyl]-phenyl]-2,3-O-isopropyliden-L-threitol in 20 ml Dimethylformamid wurde unter Feuchtigkeitsausschluss bei 0 - 5 °C mit 0,5 ml 4-Methylmorpholin versetzt und während 10 Minuten intensiv gerührt. Hierauf wurde 0,85 g 2-Chlor-4,6-dimethoxy-1,3,5-triazin fest hinzugefügt und weitere 2 Stunden bei 0 - 5 °C gerührt. Anschliessend wurde 0,87 g D-Glucamin zugesetzt und weitere 18 Stunden bei Raumtemperatur gerührt. Hierauf wurde das Reaktionsgemisch mehrmals mit Wasser verdünnt und unter vermindertem Druck bei 40 - 50 °C eingedampft, mit 17 ml Pyridin und 17 ml Acetanhydrid versetzt und weitere 18 Stunden gerührt. Dann wurde das Reaktionsgemisch erneut eingeengt, mit Eiswasser und 1 N Salzsäure versetzt und mit Methylenchlorid extrahiert. Die vereinigten Methylenchloridphasen wurden über Magnesiumsulfat getrocknet, eingeengt und mit Methylenchlorid/Methanol an Kieselgel chromatographiert. Die Produktfraktion wurde in 30 ml Methanol und 7 ml Tetrahydrofuran gelöst, mit 2 ml 1 molarer Natriummethylat-Lösung in Methanol versetzt und während 18 Stunden bei Raumtemperatur gerührt. Der dabei gebildete Niederschlag wurde nach Zusatz von wenig Essigsäure und teilweise Abdestillieren des Methanols abfiltriert. Dabei resultierte 1,4-Bis-O-[4-[(E)-2-D-glucit-1-ylcarbamoyl-vinyl]-phenyl]-2,3-O-isopropyliden-L-threitol als farbloser Festkörper, MS: *m/z* 781,5 ([M+H]$^+$).

D. 0,33 g 1,4-Bis-O-[4-[(E)-2-D-glucit-1-ylcarbamoyl-vinyl]-phenyl]-2,3-0-isopropyliden-L-threitol wurde in 5 ml Dioxan, 1 ml Trifluoressigsäure und 1,5 ml Wasser suspendiert und 1,5 Stunden bei 110 °C unter Rückfluss erwärmt. Anschliessend wurde das Reaktionsgemisch zur Trockene eingeengt. Dabei resultierte 1,4-Bis-O-[4-[(E)-2-D-glucit-1-ylcarbamoyl-vinyl]-phenyl]-L-threitol als farbloser Festkörper, MS: *m/z* 741,6 ([M+H]$^+$).

E. Das oben erhaltene 1,4-Bis-O-[4-[(E)-2-D-glucit-1-ylcarbamoyl-vinyl]-phenyl]-L-threitol wurde analog Beispiel 1.B. zu 1,4-Bis-O-[4-[(E)-2-(2,3,4,5,6-penta-O-sulfo-D-glucit-1-ylcarbamoyl)-vinyl]-phenyl]-2,3-di-O-sulfo-L-threitol Dodekanatriumsalz, $[\alpha]_D^{20}$ -0,86° (*c* 0,7; Wasser), MS: *m/z* 1966,0 (rekonstruiertes M), umgesetzt.

Beispiel 8

A. 0,2 g 1,4-Bis-O-[4-[(E)-2-(2,3,4,5,6-penta-O-sulfo-D-glucit-1-ylcarbamoyl)-vinyl]-phenyl]-2,3-di-O-sulfo-L-threitol Dodekanatriumsalz wurde in 10 ml destilliertem Wasser unter Zusatz von 40 mg Palladium auf Kohle (10%) in einer Wasserstoffatmosphäre erschöpfend hydriert. Anschliessend wurde das Reaktionsgemisch über einen 0,8 μ Cellulosefilter filtriert und das Filtrat lyophilisiert; dabei wurde 1,4-Bis-O-[4-[2-(2,3,4,5,6-penta-O-sulfo-D-glucit-1-ylcarbamoyl)-ethyl]-phenyl]-di-O-sulfo-L-threitol Dodekanatrium-salz, $[\alpha]_D^{20}$ -0,17° (*c* 0,6; Wasser), MS: *m/z* 1970,0 (rekonstruiertes M), erhalten.

Beispiel 9

A. Eine Lösung von 4,91 g (4-Methoxycarbonyl-benzyl)-triphenylphosphoniumbromid in 50 ml Tetrahydrofuran wurde unter Feuchtigkeitsausschluss und unter Argon bei 0 - 5 °C mit 1,91 g 4-Benzyloxy-benzaldehyd und anschliessend langsam mit 10 ml 1 N Natriummethylat-Lösung in Methanol versetzt. Nach 30 Minuten Rühren bei Raumtemperatur wurde das Reaktionsgemisch über Dicalit filtriert, eingeengt und mit Hexan/Essigester (3:1) an Kieselgel chromatographiert. Dabei wurde (Z)-4-[2-(4-Benzyloxy-phenyl)-vinyl]-benzoesäure methylester als gelblicher Festkörper erhalten, MS: *m/z* 344 ([M]$^+$).

B. 1,5 g (Z)-4-[2-(4-Benzyloxy-phenyl)-vinyl]-benzoesäure methylester wurde in 20 ml Methanol und 20 ml Aethylacetat unter Zusatz von 150 mg Palladium auf Kohle (10%) in einer Wasserstoffatmosphäre erschöpfend hydriert. Anschliessend wurde das Reaktionsgemisch über einen 0,8 μ Cellulosefilter filtriert und das Filtrat eingeengt; dabei wurde 4-[2-(4-Hydroxy-phenyl)-ethyl]-benzoesäure methylester als farbloser Festkörper erhalten, MS: *m/z* 256 ([M]$^+$).

C. 0,71 g 2,3-O-Isopropyliden-1,4-bis-O-(4-methyl-phenylsulfonyl)-L-threitol, 1,0 g 4-[2-(4-Hydroxy-phenyl)-ethyl]-benzoesäuremethylester und 0,54 g fein gemahlenes wasserfreies Kaliumkarbonat wurden in 20 ml Dimethylformamid suspendiert und während 18 Stunden bei 130 °C unter Argon gerührt. Anschliessend wurde das Reaktionsgemisch mit Wasser verdünnt und mit Ethylacetat extrahiert. Die vereinigten Ethylacetatphasen wurden über Magnesiumsulfat getrocknet und eingeengt. Der Rückstand wurde mit 5% Ether in Methylenchlorid an Kieselgel chromatographiert. So wurde 2,3-O-Isopropyliden-1,4-bis-O-[4-[2-(4-methoxycarbonyl-phenyl)-ethyl]-phenyl]-L-threitol in Form farbloser Kristalle erhalten, MS: *m/z* 638 ([M]$^+$).

D. 0,7 g 2,3-O-Isopropyliden-1,4-bis-O-[4-[2-(4-methoxycarbonyl-phenyl)-ethyl]-phenyl]-L-threitol wurde in 20 ml Methanol und 20 ml konzentrierter Natriumhydroxidlösung während 18 Stunden unter Rückfluss gerührt. Anschliessend wurde das Methanol abdestilliert. Der Rückstand wurde mit Eiswasser versetzt, mit verdünnter Salzsäure angesäuert und der gebildete Niederschlag abfiltriert; dabei wurde 1,4-Bis-O-[4-[2-(4-carboxy-phenyl)-ethyl]-phenyl]-2,3-O-isopropyliden-L-threitol als farbloser Festkörper erhalten, MS: *m/z* 609,4 ([M-H]$^-$).

E. 0,52 g 1,4-Bis-O-[4-[2-(4-carboxy-phenyl)-ethyl]-phenyl]-2,3-O-isopropyliden-L-threitol in 10 ml Dimethylformamid wurde unter Feuchtigkeitsausschluss bei 0 - 5 °C mit 0,21 ml 4-Methylmorpholin versetzt und während 10 Minuten intensiv gerührt. Hierauf wurde 0,33 g 2-Chlor-4,6-dimethoxy-1,3,5-triazin fest hinzugefügt und weitere 2 Stunden bei 0 - 5 °C gerührt. Anschliessend wurde 0,34 g D-Glucamin zugesetzt und weitere 18 Stunden bei Raumtemperatur gerührt. Hierauf wurde das Reaktionsgemisch mit Wasser verdünnt, eingeengt, mit 10 ml Wasser und 0,5 ml Triäthylamin versetzt, kurz aufgekocht und filtriert. Der dabei erhaltene Rückstand wurde in 20 ml Methanol und 20 ml Methylenchlorid verrührt und erneut filtriert. Dabei resultierte 1,4-Bis-O-[4-[2-(4-D-glucit-1-ylcarbamoyl-phenyl)-ethyl]-phenyl]-2,3-O-isopropyliden-L-threitol als farbloser Festkörper, MS: *m/z* 959,6 ($[M+Na]^+$).

F. 0,52 g 1,4-Bis-O-[4-[2-(4-D-glucit-1-ylcarbamoyl-phenyl)-ethyl]-phenyl]-2,3-O-isopropyliden-L-threitol wurde in 7 ml Dioxan, 2 ml Trifluoressigsäure und 3 ml Wasser suspendiert und 18 Stunden bei 110 °C unter Rückfluss erwärmt. Anschliessend wurde das Reaktionsgemisch zur Trockene eingeengt, mit 6 ml Essigsäureanhydrid und 6 ml Pyridin versetzt und 18 Stunden bei Raumtemperatur gerührt. Dann wurde das Reaktionsgemisch erneut eingeengt und mit Eiswasser und 1 N Salzsäure versetzt. Der dabei gebildete Niederschlag wurde abfiltriert und mit Methylenchlorid/ Isopropanol an Kieselgel chromatographiert. Dabei resultierte 2,3-Di-O-acetyl-1,4-bis-O-[4-[2-[4-(2,3,4,5,6-penta-O-acetyl-D-glucit-1-ylcarbamoyl)-phenyl]-ethyl]-phenyl]-L-threitol als farbloser Festkörper, MS: *m/z* 1424,9 ($[M+Na]^+$).

G. 0,36 g 2,3-Di-O-acetyl-1,4-bis-O-[4-[2-[4-(2,3,4,5,6-penta-O-acetyl-D-glucit-1-ylcarbamoyl)-phenyl]-ethyl]-phenyl]-L-threitol wurde in 5 ml Methanol und 5 ml Tetrahydrofuran gelöst, mit 0,4 ml 1 molarer Natriummethylatlösung in Methanol versetzt und während 18 Stunden bei Raumtemperatur gerührt. Der dabei gebildete Niederschlag wurde abfiltriert. Dabei resultierte 1,4-Bis-O-[4-[2-(4-D-glucit-1-ylcarbamoyl-phenyl)-ethyl]-phenyl]-L-threitol als farbloser Festkörper, MS: *m/z* 919,3 ($[M+Na]^+$).

H. Das oben erhaltene 1,4-Bis-O-[4-[2-(4-D-glucit-1-ylcarbamoyl-phenyl)-ethyl]-phenyl]-L-threitol wurde analog Beispiel 1.B. zu 1,4-Bis-O-[4-[2-[4-(2,3,4,5,6-penta-O-sulfo-D-glucit-1-ylcarbamoyl)-phenyl]-ethyl]-phenyl]-2,3-di-O-sulfo-L-threitol Dodekanatriumsalz, $[\alpha]_D^{20}$ -0,57° (*c* 0,7; Wasser) umgesetzt.

<u>Beispiel 10</u>

A. Analog Beispiel 5.A. - D. wurde aus 4'-Hydroxy-biphenyl-4-carbonsäuremethylester und 2,3-O-Isopropyliden-1,4-bis-O-(4-methyl-phenylsulfonyl)-D-threitol an Stelle von 2,3-O-Isopropyliden-1,4-bis-O-(4-methylphenylsulfonyl)-L-threitol über folgende Zwischenstufen:

2,3-O-Isopropyliden-1,4-bis-O-(4'-methoxycarbonyl-biphenyl-4-yl)-D-threitol, farbloser Festkörper, MS: *m/z* 582 ($[M]^+$);

1,4-Bis-O-(4'-carboxy-biphenyl-4-yl)-2,3-O-isopropyliden-D-threitol, farbloser Festkörper, MS: *m/z* 553,5 ($[M-H]^-$);

1,4-Bis-O-(4'-D-glucit-1-ylcarbamoyl-biphenyl-4-yl)-2,3-O-isopropyliden-D-threitol, farbloser Festkörper, MS: *m/z* 881,5 ($[M+H]^+$);

1,4-Bis-O-(4'-D-glucit-1-ylcarbamoyl-biphenyl-4-yl)-D-threitol als farbloser Festkörper erhalten, MS: *m/z* 864,6 ($[M+Na]^+$).

B. Das oben erhaltene 1,4-Bis-O-(4'-D-glucit-1-ylcarbamoyl-biphenyl-4-yl)-D-threitol wurde analog Beispiel 1.B. zu 1,4-Bis-O-[4-(2,3,4,5,6-penta-O-sulfo-D-glucit-1-ylcarbamoyl)-biphenyl-4-yl]-2,3-di-O-sulfo-D-threitol Dodekanatriumsalz , $[\alpha]_D^{20}$ -9,8° (*c* 0,8; Wasser), MS: *m/z* 2066,0 (rekonstruiertes M) umgesetzt.

<u>Beispiel 11</u>

A. Analog Beispiel 9.C. - 9.G. wurde aus 4-[2-(4-Hydroxy-phenyl)-ethyl]-benzoesäure methylester und 2,3:4,5-Di-O-isopropyliden-1,6-bis-O-(4-methyl-phenylsulfonyl)-galactitol an Stelle von 2,3-O-Isopropyliden-1,4-bis-O-(4-methyl-phenylsulfonyl)-L-threitol über folgende Zwischenstufen:

2,3:4,5-Di-O-isopropyliden-1,6-bis-O-[4-[2-(4-methoxycarbonyl-phenyl)-ethyl]-phenyl]-galactitol, farbloser Festkörper, MS: *m/z* 738 ($[M]^+$);

1,6-Bis-O-[4-[2-(4-carboxy-phenyl)-ethyl]-phenyl]-2,3:4,5-di-O-isopropylidengalactitol, farbloser Festkörper, MS: *m/z* 709,4 ([M-H]-);

1,6-Bis-O-[4-[2-(4-D-glucit-1-ylcarbamoyl-phenyl)-ethyl]-phenyl]-2,3:4,5-di-O-isopropyliden-galactitol, farbloser

Festkörper, MS: *m/z* 1037,6 ($(M+H]^+$);

2,3,4,5-Tetra-O-acetyl-1,6-bis-O-[4-[2-[4-(2,3,4,5,6-penta-O-acetyl-D-glucit-1-ylcarbamoyl)-phenyl]-ethyl]-phenyl]-galactitol, farbloser Festkörper, MS: *m/z* 773,8 ($[M+2H]^{2+}$);

1,6-Bis-O-[4-[2-(4-D-glucit-1-ylcarbamoyl-phenyl)-ethyl]-phenyl]-galactitol als farbloser Festkörper erhalten, MS: *m/z* 979,5 ($[M+Na]^+$).

B. Das oben erhaltene 1,6-Bis-O-[4-[2-(4-D-glucit-1-ylcarbamoyl-phenyl)-ethyl]-phenyl]-galactitol wurde analog Beispiel 1.B. zu 1,6-Bis-O-[4-[2-[4-(2,3,4,5,6-penta-O-sulfo-D-glucit-1-ylcarbamoyl)-phenyl]-ethyl]-phenyl]-2,3,4,5-tetra-O-sulfo-galactitol Tetradekanatriumsalz, $[\alpha]_D^{20}$ -3,3° (*c* 0,6; Wasser), MS: *m/z* 2385,0 (rekonstruiertes M) umgesetzt.

Beispiel 12

A. 1,33 g 2,3:4,5-Di-O-isopropyliden-1,6-bis-O-(4-methyl-phenylsulfonyl)-galactitol, 1,6 g 4'-Hydroxy-biphenyl-4-carbonsäuremethylester und 0,97 g fein gemahlenes wasserfreies Kaliumkarbonat wurden in 20 ml Dimethylformamid suspendiert und während 18 Stunden bei 130 °C unter Argon gerührt. Anschliessend wurde das Reaktionsgemisch mit Wasser verdünnt und die so gebildeten Kristalle wurden abfiltriert. So wurde 2,3:4,5-Di-O-isopropyliden-1,6-bis-(4'-methoxycarbonyl-biphenyl-4-yl)-galactitol in Form farbloser Kristalle erhalten, MS: *m/z* 682 ($[M]^+$).

B. 1,2 g 2,3:4,5-Di-O-isopropyliden-1,6-bis-(4'-methoxycarbonyl-biphenyl-4-yl)-galactitol, 25 ml Methanol und 25 ml konzentrierte wässrige Natronlauge wurden bei 110 °C 18 Stunden unter Rückfluss erwärmt. Hierauf wurde der grösste Teil des Methanol unter vermindertem Druck abdestilliert, der Rückstand mit Eiswasser verdünnt, mit verdünnter wässriger Salzsäure angesäuert, und die dabei gebildeten Kristalle wurden abfiltriert. So wurde 1,6-Bis-(4'-carboxy-biphenyl-4-yl)-2,3:4,5-di-O-isopropyliden-galactitol in Form farbloser Kristalle erhalten, MS: *m/z* 653,5 ($[M-H]^-$).

C. 1,0 g 1,6-Bis-(4'-carboxy-biphenyl-4-yl)-2,3:4,5-di-O-isopropyliden-galactitol wurde in 17 ml Dioxan, 4 ml Trifluoressigsäure und 5 ml Wasser suspendiert und 17 Stunden bei 110 °C unter Rückfluss erwärmt. Hierauf wurde das Reaktionsgemisch eingeengt, mit Methanol versetzt und filtriert. Dabei resultierte 1,6-Bis-(4'-carboxy-biphenyl-4-yl)-galactitol als farbloser Festkörper, Elementaranalyse berechnet für $C_{32}H_{30}O_{10}x0.31H_2O$: C =66,25%, H = 5,32%: gefunden: C = 66,12%, H = 5,24%.

D. 0,8 g 1,6-Bis-(4'-carboxy-biphenyl-4-yl)-galactitol wurde in einem Gemisch von 20 ml Essigsäureanhydrid und 20 ml Pyridin während 5 Stunden bei 100 °C gerührt. Hierauf wurde das Reaktionsgemisch eingeengt, mit Eiswasser und verdünnter Salzsäure versetzt, und der so gebildete Niederschlag wurde abfiltriert. Dabei resultierte 2,3,4,5-Tetra-O-acetyl-1,6-bis-O-(4'-carboxy-biphenyl-4-yl)-galactitol als farbloser Festkörper, MS: *m/z* 741,4 ($[M-H]^-$).

E. 0,84 g 2,3,4,5-Tetra-O-acetyl-1,6-bis-O-(4'-carboxy-biphenyl-4-yl)-galactitol in 20 ml Dimethylformamid wurde unter Feuchtigkeitsausschluss bei 0 - 5 °C mit 0,3 ml 4-Methylmorpholin versetzt und während 10 Minuten intensiv gerührt. Hierauf wurde 0,44 g 2-Chlor-4,6-dimethoxy-1,3,5-triazin fest hinzugefügt und weitere 2 Stunden bei 0 - 5 °C gerührt. Anschliessend wurden 0,45 g D-Glucamin zugesetzt und weitere 18 Stunden bei Raumtemperatur gerührt. Hierauf wurde das Reaktionsgemisch mehrmals mit Wasser verdünnt und unter vermindertem Druck bei 40 - 50 °C eingedampft, mit 12 ml Wasser und 0,5 ml Triäthylamin versetzt, erwärmt und filtriert. Das so erhaltene rohe 2,3,4,5-Tetra-O-acetyl-1,6-bis-O-(4'-D-glucit-1-ylcarbamoyl-biphenyl-4-yl)-galactitol wurde zur weiteren Reinigung mit 10 ml Pyridin und 10 ml Acetanhydrid versetzt und 24 Stunden bei Raumtemperatur gerührt. Dann wurde dieses Reaktionsgemisch eingeengt, mit Eiswasser und 1 N Salzsäure versetzt und mit Methylenchlorid extrahiert. Die vereinigten Methylenchloridphasen wurden über Magnesiumsulfat getrocknet, eingeengt und mit Methylenchlorid/Isopropanol an Kieselgel chromatographiert. So wurde 2,3,4,5-Tetra-O-acetyl-1,6-bis-O-[4'-(2,3,4,5,6-penta-O-acetyl-D-glucit-1-ylcarbamoyl)-biphenyl-4-yl]-galactitol als farbloser Festkörper erhalten, MS: *m/z* 1489,4 ($[M+H]^+$).

F. 0,6 g 2,3,4,5-Tetra-O-acetyl-1,6-bis-O-[4'-(2,3,4,5,6-penta-O-acetyl-D-glucit-1-ylcarbamoyl)-biphenyl-4-yl]-galactitol wurde in 5 ml Methanol und 5 ml Tetrahydrofuran gelöst, mit 0,4 ml 1 molarer Natriummethylat-Lösung in Methanol versetzt und während 2 Stunden bei 60 °C und 18 Stunden bei Raumtemperatur gerührt. Der dabei gebildete Niederschlag wurde abfiltriert. So resultierte 1,6-Bis-O-(4'-D-glucit-1-ylcarbamoyl-biphenyl-4-yl)-galactitol als farbloser Festkörper, MS: *m/z* 923,2 ($[M+Na]^+$).

G. Das oben erhaltene 1,6-Bis-O-(4'-D-glucit-1-ylcarbamoyl-biphenyl-4-yl)-galactitol wurde analog Beispiel 1.B. zu 1,6-Bis-O-[4'-(2,3,4,5,6-penta-O-sulfo-D-glucit-1-ylcarbamoyl)-biphenyl-4-yl]-2,3,4,5-tetra-O-sulfo-galactitol Tetradekanatriumsalz, $[\alpha]_D^{20}$-2,4° (*c* 0,7; Wasser), MS: *m/z* 2330,0 (rekonstruiertes M), umgesetzt.

Beispiel 13

A. Analog Beispiel 5.A. - D. wurde aus 3-Hydroxy-naphthalin-2-carbon-säuremethylester und 2,3-O-Isopropyliden-1,4-bis-O-(4-methyl-phenyl-sulfonyl)-L-threitol über folgende Zwischenstufen:

2,3-O-Isopropyliden-1,4-bis-O-(3-methoxycarbonyl-naphthalin-2-yl)-L-threitol, farbloser Festkörper, MS: *m*/*z* 530 ($[M]^+$);

1,4-Bis-O-(3-carboxy-naphthalin-2-yl)-2,3-O-isopropyliden-L-threitol, farbloser Festkörper, MS: *m*/*z* 501,4 ($[M-H]^-$);

1,4-Bis-O-(3-D-glucit-1-ylcarbamoyl-naphthalin-2-yl)-2,3-O-isopropyliden-L-threitol, farbloser Festkörper, MS: *m*/*z* 830,0($[M+H]^+$);

1,4-Bis-O-(3-D-glucit-1-ylcarbamoyl-naphthalin-2-yl)-L-threitol als farbloser Festkörper erhalten, MS: *m*/*z* 789,7 ($[M+H]^+$).

B. Das oben erhaltene 1,4-Bis-O-(3-D-glucit-1-ylcarbamoyl-naphthalin-2-yl)-L-threitol wurde analog Beispiel 1.B. zu 1,4-Bis-O-[3-(2,3,4,5,6-penta-O-sulfo-D-glucit-1-ylcarbamoyl)-naphtalin-2-yl]-2,3-di-O-sulfo-L-threitol Dodekanatriumsalz, $[\alpha]_D^{20}$ -3,9° (*c* 0,7; Wasser), MS: *m*/*z* 2013,5 (rekonstruiertes M) umgesetzt.

Beispiel 14

A. Analog Beispiel 12. A. - F. wurde aus 3-Hydroxy-naphthalin-2-carbonsäuremethylester und 2,3:4,5-Di-O-isopropyliden-1,6-bis-O-(4-methyl-phenylsulfonyl)-galactitol über folgende Zwischenstufen:

2,3:4,5-Di-O-isopropyliden-1,6-bis-O-(2-methoxycarbonyl-naphthalin-2-yl)-galactitol, gelblicher Festkörper, MS: *m*/*z* 630 ($[M]^+$);

1,6-Bis-O-(3-carboxy-naphthalin-2-yl)-2,3:4,5-di-O-isopropyliden-galactitol, farbloser Festkörper, MS: *m*/*z* 601,3 ($[M-H]^-$);

1,6-Bis-O-(3-carboxy-naphthalin-2-yl)-galactitol, farbloser Festkörper, MS: *m*/*z* 545,3 ($[M+Na]^+$);

2,3,4,5-Tetra-O-acetyl-1,6-bis-O-(3-carboxy-naphthalin-2-yl)-galactitol, farbloser Festkörper, MS: *m*/*z* 713,4 ($[M+Na]^+$);

2,3,4,5-Tetra-O-acetyl-1,6-bis-O-(3-D-glucit-1-ylcarbamoyl-naphthalin-2-yl)-galactitol, farbloser Festkörper, MS: *m*/*z* 1017,6 ($[M+H]^+$);

1,6-Bis-O-(3-D-glucit-1-ylcarbamoyl-naphthalin-2-yl)-galactitol als farbloser Festkörper erhalten, MS: *m*/*z* 849,7 ($[M+H]^+$).

B. Das oben erhaltene 1,6-Bis-O-(3-D-glucit-1-ylcarbamoyl-naphthalin-2-yl)-galactitol wurde analog Beispiel 1.B. zu 1,6-Bis-O-[3-(2,3,4,5,6-penta-O-sulfo-D-glucit-1-ylcarbamoyl)-naphthalin-2-yl]-2,3,4,5-tetra-O-sulfo-galactitol Tetradekanatriumsalz, $[\alpha]_D^{20}$ -6,3° (*c* 0,7; Wasser), MS: *m*/*z* 2277,5 (rekonstruiertes M) umgesetzt.

Beispiel 15

A. 0,4 g Bis-(4-hydroxy-phenyl)-methan gelöst in 8 ml Acetonitril wurde unter Argon mit 0,3 g gepulvertem Kaliumkarbonat und 0,4 g tert.-Butyl bromoacetat versetzt und 2,5 Stunden bei Raumtemperatur gerührt. Anschliessend wurde nochmal 0,13 g tert.-Butyl bromoacetat hinzugefügt und während 70 Stunden bei Raumtemperatur gerührt. Hierauf wurde das Reaktionsgemisch eingeengt, und der Rückstand wurde mit Methylenchlorid/Ether (95:5) an Kieselgel chromatographiert; dabei wurde 4-(4-Hydroxy-benzyl)-phenoxyessigsäure tert.-butyl ester als farbloser Festkörper erhalten, MS: *m*/*z* 314 ($[M]^+$).

B. Analog Beispiel 12. A. - F. wurde aus 4-(4-Hydroxy-benzyl)-phenoxy-essigsäure tert.-butyl ester und 2,3-O-Isopropyliden-1,4-di-O-(4-methyl-phenylsulfonyl)-L-threitol über folgende Zwischenstufen:

1,4-Bis-O-[4-(4-tert.-butoxycarbonylmethoxy-benzyl)-phenyl]-2,3-0-isopropyliden-L-threitol, farbloser Festkörper, welcher ohne weitere Charakterisierung weiter verarbeitet wurde;

1,4-Bis-O-[4-(4-carboxymethoxy-benzyl)-phenyl)-2,3-O-isopropyliden-L-threitol, farbloser Festkörper, MS: *m/z* 660,5 ($[M+NH_4]^+$);

1,4-Bis-O-[4-(4-carboxymethoxy-benzyl)-phenyl]-L-threitol, farbloser Festkörper, MS: *m/z* 625,5 ($[M+Na]^+$);

2,3-Di-O-acetyl-1,4-bis-O-[4-(4-carboxymethoxy-benzyl)-phenyl]-L-threitol, bräunlicher, amorpher Festkörper, MS: *m/z* 685,4 ($[M-H]^-$);

2,3-Di-O-acetyl-1,4-bis-O-[4-[4-(2,3,4,5,6-penta-O-acetyl-D-glucit-1-ylcarbamoylmethoxy)-benzyl]-phenyl]-L-threitol, farbloser amorpher Festkörper, MS: *m/z* 1456,8 ($[M+Na]^+$);

1,4-Bis-O-[4-(4-D-glucit-1-ylcarbamoylmethoxy-benzyl)-phenyl]-L-threitol als farbloser Festkörper erhalten, MS: *m/z* 952,4 ($[M+Na]^+$).

C. Das oben erhaltene 1,4-Bis-O-[4-(4-D-glucit-1-ylcarbamoylmethoxy-benzyl)-phenyl]-L-threitol wurde analog Beispiel 1.B. zu 1,4-Bis-O-[4-[4-(2,3,4,5,6-penta-O-sulfo-D-glucit-1-ylcarbamoylmethoxy)-benzyl]-phenyl]-2,3-di-O-sulfo-L-threitol Dodekanatriumsalz , $[\alpha]_D^{20}$ +0,50° (*c* 0,6; Wasser), MS: *m/z* 2154,0 (rekonstruiertes M), umgesetzt.

Beispiel 16

A. Analog Beispiel 3.A. - D. wurde aus 6-Hydroxy-naphthalin-2-carbonsäuremethylester und 2,3:4,5-Di-O-isopropyliden-1,6-bis-O-(4-methyl-phenylsulfonyl)-galactitol über folgende Zwischenstufen:

2,3:4,5-Di-O-isopropyliden-1,6-bis-O-(6-methoxycarbonyl-naphthalin-2-yl)-galactitol, farbloser Festkörper, MS: *m/z* 630 ($[M]^+$);

1,6-Bis-O-(6-carboxy-naphthalin-2-yl)-2,3:4,5-di-O-isopropyliden-galactitol, farbloser Festkörper, MS: *m/z* 601,3 ($[M-H]^-$);

1,6-Bis-O-(6-D-glucit-1-ylcarbamoyl-naphthalin-2-yl)-2,3:4,5-di-O-isopropyliden-galactitol, farbloser Festkörper, MS: *m/z* 929,7 ($[M+H]^+$);

1,6-Bis-O-(6-D-glucit-1-ylcarbamoyl-naphthalin-2-yl)-galactitol als rötlicher Festkörper erhalten, MS: *m/z* 871,5 ($[M+Na]^+$).

B. Das oben erhaltene 1,6-Bis-O-(6-D-glucit-1-ylcarbamoyl-naphthalin-2-yl)-galactitol wurde analog Beispiel 1.B. zu 1,6-Bis-O-[6-(2,3,4,5,6-penta-O-sulfo-D-glucit-1-ylcarbamoyl)-naphthalin-2-yl]-2,3,4,5-tetra-O-sulfo-galactitol Tetradekanatriumsalz , $[\alpha]_D^{20}$ -4,1° (*c* 0,7; Wasser) umgesetzt.

Beispiel 17

A. Zu 16,82 g 3,5-Dihydroxy-benzoesäuremethylester, gelöst in 120 ml Methanol wurde unter Rühren und unter Feuchtigkeitsausschluss 100 ml Natriummethylat-Lösung in Methanol (hergestellt mit 2,6 g Natrium) gefolgt von 22,29 g 4-Chlormethyl-biphenyl zugegeben und während 4 Stunden bei 90 °C gerührt. Hierauf wurde das Reaktionsgemisch eingeengt, zwischen Methylenchlorid und verdünnter Salzsäure verteilt. Die vereinigten Methylenchloridphasen wurden über Magnesiumsulfat getrocknet, eingeengt und mit Methylenchlorid/Methanol an Kieselgel chromatographiert. Die so erhaltene Produktfraktion wurde aus Methylenchorid umkristallisiert. Dabei wurde 3-Biphenyl-4-ylmethoxy-5-hydroxy-benzoesäuremethylester in Form farbloser Kristalle erhalten, MS: *m/z* 334 ($[M]^+$).

B. Analog Beispiel 3.A. - C. wurde aus 3-Biphenyl-4-ylmethoxy-5-hydroxy-benzoesäuremethylester und 2,3:4,5-Di-O-isopropyliden-1,6-bis-O-(4-methyl-phenylsulfonyl)-galactitol über folgende Zwischenstufen:

1,6-Bis-O-(3-biphenyl-4-ylmethoxy-5-methoxycarbonyl-phenyl)-2,3:4,5-di-O-isopropyliden-galactitol, farbloser Festkörper, welcher ohne weitere Charakterisierung weiter verarbeitet wurde;

1,6-Bis-O-(3-biphenyl-4-ylmethoxy-5-carboxy-phenyl)-2,3:4,5-di-O-isopropyliden-galactitol, farbloser Festkörper, MS: *m/z* 865,2 ($[M-H]^-$);

1,6-Bis-O-(3-biphenyl-4-ylmethoxy-5-D-glucit-1-ylcarbamoyl-phenyl)-2,3:4,5-di-O-isopropyliden-galactitol in

Form farbloser Kristalle erhalten, MS: *m/z* 1215,3 ([M+Na]$^+$).

C. 0,2 g 1,6-Bis-O-(3-biphenyl-4-ylmethoxy-5-D-glucit-1-ylcarbamoyl-phenyl)-2,3:4,5-di-O-isopropyliden-galactitol wurde in 2 ml Dioxan, 0,5 ml Trifluoressigsäure und 0,7 ml Wasser suspendiert und 72 Stunden bei Raumtemperatur gerührt. Hierauf wurde das Reaktionsgemisch eingeengt, mit Methanol versetzt und filtriert. Dabei resultierte 1,6-Bis-O-(3-biphenyl-4-ylmethoxy-5-D-glucit-1-ylcarbamoyl-phenyl)-galactitol als farbloser Festkörper, MS: *m/z* 1114,4 ([M+H]$^+$).

D. Das oben erhaltene 1,6-Bis-O-(3-biphenyl-4-ylmethoxy-5-D-glucit-1-ylcarbamoyl-phenyl)-galactitol wurde analog Beispiel 1.B. zu 1,6-Bis-O-[3-biphenyl-4-ylmethoxy-5-(2,3,4,5,6-penta-O-sulfo-D-glucit-1-ylcarbamoyl)-phenyl]-2,3,4,5-tetra-O-sulfo-galactitol Tetradekanatriumsalz , $[\alpha]_D^{20}$ -2,4° (*c* 0,7; Wasser), MS: *m/z* 2512,0 (rekonstruiertes [M-Na]), umgesetzt.

Beispiel 18

A. Analog Beispiel 3. A. - D. wurde aus 4-Hydroxy-benzoesäuremethylester und 2,3-O-Isopropyliden-1,4-bis-O-(4-methyl-phenylsulfonyl)-L-threitol über folgende Zwischenstufen:

2,3-O-Isopropyliden-1,4-bis-O-(4-methoxycarbonyl-phenyl)-L-threitol, farbloser Festkörper, MS: *m/z* 430 ([M]$^+$);

1,4-Bis-O-(4-carboxy-phenyl)-2,3-O-isopropyliden-L-threitol, farbloser Festkörper, MS: *m/z* 402 ([M]$^+$);

1,4-Bis-O-(4-D-glucit-1-ylcarbamoyl-phenyl)-2,3-O-isopropyliden-L-threitol, farbloser Festkörper, MS: *m/z* 729,4 ([M+H]$^+$);

1,4-Bis-O-(4-D-glucit-1-ylcarbamoyl-phenyl)-L-threitol als farbloser Festkörper erhalten, MS: *m/z* 689,3 ([M+H]$^+$).

B. Das oben erhaltene 1,4-Bis-O-(4-D-glucit-1-ylcarbamoyl-phenyl)-L-threitol wurde analog Beispiel 1.B. zu 1,4-Bis-O-[4-(2,3,4,5,6-penta-O-sulfo-D-glucit-1-ylcarbamoyl)-phenyl]-2,3-di-O-sulfo-L-threitol Dodekanatriumsalz , $[\alpha]_D^{20}$ -3,1° (*c* 0,7; Wasser), MS: *m/z* 1913,5 (rekonstruiertes M), umgesetzt.

Beispiel 19

A. Analog Beispiel 3. A. - D. wurde aus 4-Hydroxy-naphthalin-2-carbonsäureethylester und 2,3:4,5-Di-O-isopropyliden-1,6-bis-O-(4-methyl-phenylsulfonyl)-galactitol über folgende Zwischenstufen:

2,3:4,5-Di-O-isopropyliden-1,6-bis-O-(3-ethoxycarbonyl-naphthalin-1-yl)-galactitol, farbloser Festkörper, MS: *m/z* 658 ([M]$^+$);

1,6-Bis-O-(3-carboxy-naphthalin-1-yl)-2,3:4,5-di-O-isopropyliden-galactitol, farbloser Festkörper, MS: *m/z* 602 ([M]$^+$);

1,6-Bis-O-(3-D-glucit-1-ylcarbamoyl-naphthalin-1-yl)-2,3:4,5-di-O-isopropyliden-D-galactitol, farbloser Festkörper, MS: *m/z* 951,8 ([M+Na]$^+$);

1,6-Bis-O-(3-D-glucit-1-ylcarbamoyl-naphthalin-1-yl)-galactitol als farbloser Festkörper erhalten, MS: *m/z* 872,4 ([M+Na]$^+$).

B. Das oben erhaltene 1,6-Bis-O-(3-D-glucit-1-ylcarbamoyl-naphthalin-1-yl)-galactitol wurde analog Beispiel 1.B. zu 1,6-Bis-O-[3-(2,3,4,5,6-penta-O-sulfo-D-glucit-1-ylcarbamoyl)-naphthalin-1-yl]-2,3,4,5-tetra-O -sulfo-galactitol Tetradekanatriumsalz , $[\alpha]_D^{20}$ -1,2° (*c* 0,5; Wasser), MS: *m/z* 2277,0 (rekonstruiertes M) umgesetzt.

Beispiel 20

A. 6,55 g 3-Hydroxy-7-methoxy-naphthalin-2-carbonsäure wurden in 20 ml Dimethylformamid gelöst, mit 5,5 g Natriumhydrogenkarbonat und 4,0 ml Dimethylsulfat versetzt und während 10 Minuten bei 90 °C unter Argon gerührt. Anschliessend wurde das Reaktionsgemisch auf Eiswasser gegossen und mit Ether extrahiert. Die vereinigten Etherphasen wurden mit Wasser gewaschen und über Magnesiumsulfat getrocknet und eingeengt. Der so erhaltene Rückstand wurde aus Essigsäureethylester/ Hexan umkristallisiert. Dabei wurde 3-Hydroxy-7-methoxy-naphthalin-2-carbonsäuremethylester mit Smp. 138 °C erhalten.

B. Analog Beispiel 12. A. - F. wurde aus 3-Hydroxy-7-methoxy-naphthalin-2-carbonsäuremethylester und 2,3:4,5-Di-O-isopropyliden-1,6-bis-O-(4-methyl-phenylsulfonyl)-galactitol über folgende Zwischenstufen:

1,6-Bis-O-(6-methoxy-3-methoxycarbonyl-naphthalin-2-yl)-2,3:4,5-di-O-isopropyliden-galactitol, farbloser Festkörper, MS: *m*/*z* 690 ($[M]^+$);

1,6-Bis-O-(3-carboxy-6-methoxy-naphthalin-2-yl)-2,3:4,5-di-O-isopropyliden-galactitol, farbloser Festkörper, MS: *m*/*z* 661,2 ($[M-H]^-$);

1,6-Bis-O-(3-carboxy-6-methoxy-naphthalin-2-yl)-galactitol, farbloser Festkörper, MS: *m*/*z* 581,2 ($[M-H]^-$);

2,3,4,5-Tetra-O-acetyl-1,6-bis-O-(3-carboxy-6-methoxy-naphthalin-2-yl)-galactitol, farbloser Festkörper, MS: *m*/*z* 749,1 ($[M-H]^-$);

2,3,4,5-Tetra-O-acetyl-1,6-bis-O-(3-D-glucit-1-ylcarbamoyl-6-methoxy-naphthalin-2-yl)-galactitol, farbloser Festkörper, MS: *m*/*z* 1077,4 ($[M+H]^+$);

1,6-Bis-O-(3-D-glucit-1-ylcarbamoyl-6-methoxy-naphthalin-2-yl)-galactitol als farbloser Festkörper erhalten, MS: *m* /*z* 909,3 ($[M+H]^+$).

C. Das oben erhaltene 1,6-Bis-O-(3-D-glucit-1-ylcarbamoyl-6-methoxy-naphthalin-2-yl)-galactitol wurde analog Beispiel 1.B. zu 1,6-Bis-O-[6-methoxy-3-(2,3,4,5,6-penta-O-sulfo-D-glucit-1-ylcarbamoyl)-naphthalin-2-yl]-2,3,4,5-tetra-O-sulfo-galactitol Tetradekanatriumsalz , $[\alpha]_D^{20}$ -5,7° (*c* 0,6; Wasser), MS: *m*/*z* 2337,5 (rekonstruiertes M) umgesetzt.

Beispiel 21

A. Analog Beispiel 20. A. wurde aus 3-Hydroxy-5-methoxy-naphthalin-2-carbonsäure 3-Hydroxy-5-methoxy-naphthalin-2-carbonsäuremethylester mit Smp. 182 °C erhalten.

B. Analog Beispiel 12. A. - F. wurde aus 3-Hydroxy-5-methoxy-naphthalin-2-carbonsäuremethylester und 2,3:4,5-Di-O-isopropyliden-1,6-bis-O-(4-methyl-phenylsulfonyl)-galactitol über folgende Zwischenstufen:

2,3:4,5-Di-O-isopropyliden-1,6-bis-O-(8-methoxy-3-methoxycarbonyl-naphthalin-2-yl)-galactitol, farbloser Festkörper, MS: *m*/*z* 690 ($[M]^+$);

1,6-Bis-O-(3-carboxy-8-methoxy-naphthalin-2-yl)-2,3:4,5-di-O-isopropyliden-galactitol, MS: *m*/*z* 661,1 ($[M-H]^-$);

1,6-Bis-O-(3-carboxy-8-methoxy-naphthalin-2-yl)-galactitol, farbloser Festkörper, MS: *m*/*z* 581,2 ($[M-H]^-$);

2,3,4,5-Tetra-O-acetyl-1,6-bis-O-(3-carboxy-5-methoxy-naphthalin-2-yl)-galactitol, farbloser Festkörper, MS: *m*/*z* 749,1 ($[M-H]^-$);

2,3,4,5-Tetra-O-acetyl-1,6-bis-O-[8-methoxy-3-(2,3,4,5,6-penta-O-acetyl-D-glucit-1-ylcarbamoyl)-naphthalin-2-yl]-galactitol, farbloser Festkörper, MS: *m*/*z* 1497,2 ($[M+H]^+$);

1,6-Bis-O-(3-D-glucit-1-ylcarbamoyl-8-methoxy-naphthalin-2-yl)-galactitol als farbloser Festkörper erhalten, MS: *m*/*z* 909,2 ($[M+H]^+$).

C. Das oben erhaltene 1,6-Bis-O-(3-D-glucit-1-ylcarbamoyl-8-methoxy-naphthalin-2-yl)-galactitol wurde analog Beispiel 1.B. zu 1,6-Bis-O-[8-methoxy-3-(2,3,4,5,6-penta-O-sulfo-D-glucit-1-ylcarbamoyl)-naphthalin-2-yl]-2,3,4,5-tetra-O-sulfo-galactitol Tetradekanatriumsalz , $[\alpha]_D^{20}$ -9,3° (*c* 0,7; Wasser), MS: *m*/*z* 2337,0 (rekonstruiertes M) umgesetzt.

Beispiel 22

A. 25,0 g 3,7-Dihydroxy-naphthalin-2-carbonsäure und 22,5 g Natriumhydrogencarbonat wurden in 125 ml Dimethylformamid gelöst, mit 16,5 ml Dimethylsulfat versetzt und bei 85 °C während 20 Minuten gerührt. Hierauf wurde

das Reaktionsgemisch auf Eiswasser gegossen. Die gebildeten Kristalle wurden abfiltriert, dabei wurde 3,7-Dihydroxy-naphthalin-2-carbonsäuremethylester als gelblicher Festkörper erhalten, MS *m/z* 218 ([M]$^+$).

B. 6,5 g 3,7-Dihydroxy-naphthalin-2-carbonsäuremethylester und 11,3 g Toluol-4-sulfonsäure-(R)-2,2-dimethyl-[1,3]dioxolan-4-ylmethylester wurden in 50 ml Acetonitril unter Zusatz von 5,4 g Kaliumkarbonat während 60 Stunden bei 80 °C gerührt. Das Reaktionsgemisch wurde auf Eiswasser gegossen und mit Ether extrahiert. Die vereinigten Etherphasen wurden mit Wasser gewaschen, über Magnesiumsulfat getrocknet, filtriert und eingedampft; der so erhaltene Rückstand wurde an Kieselgel mit Hexan/Aether chromatographiert, dabei erhielt man 7-[(S)-2,2-Dimethyl-[1,3]dioxolan-4-ylmethoxy]-3-hydroxy-naphthalin-2-carbonsäuremethylester als gelblicher Festkörper, MS: *m/z* 332 ([M]$^+$).

C. Analog Beispiel 3.A. - D. wurde aus 7-[(S)-2,2-Dimethyl-[1,3]dioxolan-4-ylmethoxy]-3-hydroxy-naphthalin-2-carbonsäuremethylester und 2,3:4,5-Di-O-isopropyliden-1,6-bis-O-(4-methyl-phenylsulfonyl)-galactitol über folgende Zwischenstufen:

1,6-Bis-O-[6-[(S)-2,2-dimethyl-[1,3]dioxolan-4-ylmethoxy]-3-methoxycarbonyl-naphthalin-2-yl]-2,3:4,5-di-O-isopropyliden-galactitol, farbloser Festkörper, MS *m/z* 891,3 ([M+H]$^+$);

1,6-Bis-O-[3-carboxy-6-[(S)-2,2-dimethyl-[1,3]dioxolan-4-yl]-naphthalin-2-yl]-2,3:4,5-di-O-isopropyliden-galactitol, farbloser Festkörper, MS *m/z* 861,4 ([M-H]$^-$);

1,6-Bis-O-[6-[(S)-2,2-dimethyl-[1,3]dioxolan-4-yl]-3-D-glucit-1-ylcarbamoyl-naphthalin-2-yl]-2,3:4,5-di-O-isopropyliden-galactitol, farbloser Festkörper, MS *m/z* 1211,6 ([M+Na]$^+$);

1,6-Bis-O-[6-[(R)-2,3-dihydroxy-propoxy]-3-D-glucit-1-ylcarbamoyl-naphthalin-2-yl]-galactitol als farbloser Festkörper erhalten, MS *m/z* 1051,3 ([M+Na]$^+$).

D. Das oben erhaltene 1,6-Bis-O-[6-[(R)-2,3-dihydroxy-propoxy]-3-D-glucit-1-ylcarbamoyl-naphthalin-2-yl]-galactitol wurde analog Beispiel 1.B. umgesetzt zu 1,6-Bis-O-[6-[(S)-2,3-bis-sulfooxy-propoxy]-3-(2,3,4,5,6-penta-O-sulfo-D-glucit-1-ylcarbamoyl)-naphthalin-2-yl]-2,3,4,5-tetra-O-sulfo-galactitol Octadekanatriumsalz, $[\alpha]_D^{20}$ -4,6° (*c* 0,7; Wasser), MS: *m/z* 2864,0 (rekonstruiertes M).

<u>Beispiel 23</u>

A. Analog Beispiel 22.A. wurde aus 7-Bromo-3-hydroxy-naphthalin-2-carbonsäure (R.A. Murphy et al., J. Med. Chem. <u>33</u>, 171 (1990)) 7-Bromo-3-hydroxy-naphthalin-2-carbonsäuremethylester als gelblicher Festkörper erhalten, MS *m/z* 280,282 ([M]$^+$).

B. Analog Beispiel 3.A. - D. wurde aus 7-Bromo-3-hydroxy-naphthalin-2-carbonsäuremethylester und 2,3:4,5-Di-O-isopropyliden-1,6-bis-O-(4-methyl-phenylsulfonyl)-galactitol über folgende Zwischenstufen:

1,6-Bis-O-(6-bromo-3-methoxycarbonyl-naphthalin-2-yl)-2,3:4,5-di-O-isopropyliden-galactitol, farbloser Festkörper, MS *m/z* 773 ([M-$CH_3$]$^+$);

1,6-Bis-O-(6-bromo-3-carboxy-naphthalin-2-yl)-2,3:4,5-di-O-isopropyliden-galactitol, farbloser Festkörper, MS *m/z* 783,2 ([M+Na]$^+$);

1,6-Bis-O-(6-bromo-3-D-glucit-1-ylcarbamoyl-naphthalin-2-yl)-2,3:4,5-di-O-isopropyliden-galactitol, farbloser Festkörper, welcher ohne weitere Reinigung weiterverarbeitet wurde;

1,6-Bis-O-(6-bromo-3-D-glucit-1-ylcarbamoyl-naphthalin-2-yl)-galactitol als farbloser Festkörper erhalten, MS *m/z* 1029,1 ([M+Na]$^+$).

C. Das oben erhaltene 1,6-Bis-O-(6-bromo-3-D-glucit-1-ylcarbamoyl-naphthalin-2-yl)-galactitol wurde analog Beispiel 1.B. zu 1,6-Bis-O-[6-bromo-3-(2,3,4,5,6-penta-O-sulfo-D-glucit-1-ylcarbamoyl)-naphthalin-2-yl]-2,3,4,5-tetra-O-sulfo-galactitol Tetradekanatriumsalz , $[\alpha]_D^{20}$ -15,4° (*c* 0,7; Wasser), umgesetzt.

<u>Beispiel 24</u>

A. Eine Suspension von 1,78 g L-Weinsäuredimethylester und 4,85 g Tris-hydroxymethyl-methylamine in 50 ml Methanol wurde 6 Tage unter Rückfluss erhitzt und dann eingeengt. Der Rückstand wurde chromatographisch mit

Essigester/ Methanol/ Wasser als Eluens über Kieselgel gereinigt und ergab N,N'-Bis-(2-hydroxy-1,1-bis-hydroxymethyl-ethyl)-L-tartaramid, $[\alpha]_D^{20}$ +101,0° (*c* 0,4; Dimethylsulfoxid), MS: *m*/*z* 357,3 ([M + H]$^+$).

B. Eine Suspension von 0,36 g N,N'-Bis-(2-hydroxy-1,1-bis-hydroxymethyl-ethyl)-L-tartaramid und 2,23 g Schwefeltrioxid-Trimethylaminkomplex in 10 ml absolutem Dimethylformamid wurde 24 Stunden bei 70 °C gerührt. Nach Abkühlen wurde mit 2,63 g Natriumacetat in 50 ml Methanol versetzt. Der Niederschlag wurde abgenutscht, mit Methanol gewaschen und getrocknet. Der Rückstand wurde in Wasser aufgenommen und an Sephadex® LH20 und SP Sephadex® C-25 chromatographiert. Die Produktfraktionen wurden lyophilisiert und ergaben 2,3-Di-O-sulfo-N,N'-bis-(2-sulfooxy-1,1,-bis-sulfooxymethyl-ethyl)-L-tartaramid Oktanatriumsalz, $[\alpha]_D^{20}$ +31,2° (*c* 0,5; Wasser), MS: *m*/*z* 1172 (rekonstruiertes M).

Beispiel 25

A. L-Weinsäuredimethylester und D-Glucamin wurden miteinander umgesetzt wie unter Bsp. 24.A. beschrieben und ergaben L-Weinsäure-N,N'-bis-D-glucit-1-yl-amid, $[\alpha]_D^{20}$ +52,0° (*c* 0,5; Dimethylsulfoxide), MS: *m*/*z* 477,6 ([M + H]$^+$).

B. Eine Suspension von 0,48 g L-Weinsäure-N,N'-bis-D-glucit-1-yl-amid und 3,34-g Schwefeltrioxid-Trimethylaminkomplex in 15 ml absolutem Dimethylformamid wurde 18 Stunden bei 70 °C gerührt. Nach Abkühlen wurde die obere Phase abdekantiert. Der Rückstand wurde mit 1,97 g Natriumacetat in 20 ml Wasser und eingedampft und dann mehrfach in Wasser aufgenommen und eingedampft. Der so erhaltene Rückstand wurde in Wasser aufgenommen und an Sephadex® LH20 und SP Sephadex® C-25 chromatographiert. Die Produktfraktionen wurden lyophilisiert und ergaben N,N'-Bis-(2,3,4,5,6-penta-O-sulfo-D-glucit-1-yl)-di-O-sulfo-L-tartaramid Dodekanatriumsalz, $[\alpha]_D^{20}$ +18,6° (*c* 0,5; Wasser), MS: *m*/*z* 1705 (rekonstruiertes M).

Beispiel 26

A. Eine Suspension von 2,0 g D-Weinsäuredimethylester und 4,47 g D-Glucamin in 50 ml Methanol wurde 8 Tage unter Rückfluss erhitzt, abgenutscht und mit Methanol gewaschen. Das Nutschgut wurde getrocknet und mit 110 ml Acetanhydrid in 220 ml Pyridin während 18 Stunden bei Raumtemperatur acetyliert. Das Reaktionsprodukt wurde eingeengt, aus Wasser gefällt, getrocknet und ergab 2,3-Di-O-acetyl-N,N'-bis-(2,3,4,5,6-penta-O-acetyl-D-glucit-1-yl)-D-tartaramid, $[\alpha]_D^{20}$ +10,4° (*c* 0,5; Dimethylsulfoxid), MS: *m*/*z* 1003,5 ([M + Na]$^+$).

B. Eine Lösung von 4,8 g 2,3-Di-O-acetyl-N,N'-bis-(2,3,4,5,6-penta-O-acetyl-D-glucit-1-yl)-D-tartaramid in 50 ml Methanol und 50 ml Tetrahydrofuran wurde mit 4,8 ml einer 2 %igen methanolischen Natriummethanolatlösung versetzt und 5 Stunden bei Raumtemperatur gerührt. Der entstandene Niederschlag wurde abgenutscht, mit Methanol gewaschen, am Vakuum bei 60 °C getrocknet und ergab N,N'-Di-D-glucit-1-yl-D-tartaramid, MS: *m*/*z* 499,6 ([M + Na]$^+$).

C. Eine Suspension von 1,0 g N,N'-Di-D-glucit-1-yl-D-tartaramid und 9,07 g Schwefeltrioxid-Triethylaminkomplex in 50 ml absolutem Dimethylformamid wurde 20 Stunden bei 45 °C gerührt. Nach Abkühlen wurde am Hochvakuum eingeengt und der Rückstand mit einer Lösung von 8,2 g Natriumacetat in 90 ml Wasser versetzt, eingedampft, mehrmals mit Wasser versetzt und erneut eingedampft. Der so erhaltene Rückstand wurde in Wasser aufgenommen und an Sephadex® LH20 und SP Sephadex® C-25 chromatographiert. Die Produktfraktionen wurden lyophilisiert und ergabenN,N'-Bis-(2,3,4,5,6-penta-O-sulfo-D-glucit-1-yl)-2,3-di-O -sulfo-D-tartaramid Dodekanatriumsalz, $[\alpha]_D^{20}$ -28,8° (*c* 0,5; Wasser), MS: *m*/*z* 1700,5 (rekonstruiertes M).

Beispiel 27

A. Umsetzung von meso-Weinsäuredimethylester und D-Glucamin wie in Bsp. 26.A. beschrieben ergab 2,3-Di-O-acetyl-N,N'-bis-(2,3,4,5,6-penta-O-acetyl-D-glucit-1-yl)-meso-tartaramid, $[\alpha]_D^{20}$ +6,2° (*c* 0,5; Dimethylsulfoxid), MS: *m*/*z* 1003,8 ([M + Na]$^+$).

B. Deacetylierung von 2,3-Di-O-acetyl-N,N'-bis-(2,3,4,5,6-penta-O-acetyl-D-glucit-1-yl)-meso-tartaramid wie in Bsp. 26.B. beschrieben ergab N,N'-Di-D-glucit-1-yl-meso-tartaramid, MS: *m*/*z* 499,4 ([M + Na]$^+$).

C. Sulfatierung von N,N'-Di-D-glucit-1-yl-meso-tartaramid wie in Bsp. 26.C. beschrieben ergab N,N'-Bis-(2,3,4,5,6-penta-O-sulfo-D-glucit-1-yl)-2,3-di-O-sulfo-meso-tartaramid Dodekanatriumsalz, $[\alpha]_D^{20}$ -4,6° (*c* 0,5; Wasser), MS: *m*/*z* 1700,5 (rekonstruiertes M).

Beispiel 28

A. Umsetzung von Galactarsäuredimethylester und Tris-hydroxymethyl-methylamin wie in Bsp. 25.A. beschrieben ergab N,N'-Bis-(2-hydroxy-1,1-bis-hydroxymethyl-ethyl)-galactaramid, MS: *m*/*z* 417,1 ([M + H]$^+$).

B. Sulfatierung von N,N'-Bis-(2-hydroxy-1,1-bis-hydroxymethyl-ethyl)-galactaramid wie in Bsp. 25.B. beschrieben ergab 2,3,4,5-Tetra-O-sulfo-N,N'-bis-(2-sulfooxy-1,1-bis-sulfooxymethyl-ethyl)-galactaramid Dekanatriumsalz, MS: *m*/*z* 1436,0 (rekonstruiertes M).

Beispiel 29

A. Umsetzung von Galactarsäuredimethylester und D-Glucamin wie in Bsp. 25.A. beschrieben ergab N,N'-Di-D-glucit-1-yl-galactaramid, $[\alpha]_D^{20}$ -14,4° (*c* 0,5; Wasser), MS: *m*/*z* 537,2 ([M + H]$^+$).

B. Sulfatierung von N,N'-Di-D-glucit-1-yl-galactaramid wie in Bsp. 25.B. beschrieben ergab N,N'-Bis-(2,3,4,5,6-penta-O-sulfo-D-glucit-1-yl)-2,3,4,5-tetra-O-sulfo-galactaramid Tetradekanatriumsalz, MS: *m*/*z* 1963,0 (rekonstruiertes M).

Beispiel 30

A. 15 g 4-Aminomethyl-benzylamin wurden in 600 ml Methylenchlorid gelöst und innerhalb vom 2,5 Stunden mit einer Lösung von 8,0 g Di-tert.-butyl-dikarbonat in 80 ml Methylenchlorid versetzt. Hierauf wurde der ausgefallene Niederschlag abfiltriert, verworfen, die Mutterlauge eingedampft und der so erhaltene Rückstand an Kieselgel mit Hexan/ Essigester/Methanol chromatographiert; dabei wurde (4-Aminomethyl-benzyl)-carbamidsäure tert.-butyl ester erhalten, MS *m*/*z* 179 ([M-tert.-Butyl]$^+$).

B. 0,6 ml D-Weinsäurediethylester und 1,81 g (4-Aminomethyl-benzyl)-carbamidsäure tert.-butyl ester wurden in 20 ml Ethanol gelöst und während 44 Stunden bei 85 °C unter Rückfluss erwärmt. Nach Abkühlen auf 0 °C wurden die ausgefallenen Kristalle abfiltriert und im Hochvakuum getrocknet; dabei wurde N,N'-Bis-(4-tert.-butoxycarbonylaminomethyl-benzyl)-D-tartaramid erhalten, MS *m*/*z* 587,1 ([M+H]$^+$).

C. 0,594 g N,N'-Bis-(4-tert.-butoxycarbonylaminomethyl-benzyl)-D-tartaramid wurde bei 0 °C in 0,8 ml Trifluoressigsäure gelöst und anschliessend während 5 Stunden bei Raumtemperatur gerührt; anschliessend wurde eingedampft und mit Methylenchlorid/Ether (1:1) versetzt. Der gebildete Niederschlag wurde abfiltriert. So wurde N,N'-Bis-(4-aminomethyl-benzyl)-D-tartaramid Di-(trifluoracetat) erhalten, MS *m*/*z* 387,4 ([M+H]$^+$).

D. 0,69 g N,N'-Bis-(4-aminomethyl-benzyl)-D-tartaramid Di-(trifluoracetat) wurde in 40 ml Ethanol gelöst, mit 0,47 ml Triethylamin und 0,48 g D-Glukonsäure-δ-lacton versetzt und während 16 Stunden bei 85 °C gerührt. Nach Abkühlen wurde der gebildete Niederschlag abfiltriert, mit Ether gewaschen und im Hochvakuum getrocknet. So wurde N,N'-Bis-(4-D-glucit-1-ylcarbamoylmethyl-benzyl)-D-tartaramid erhalten, MS *m*/*z* 743,4 ([M+H]$^+$).

E. Das oben erhaltene N,N'-Bis-(4-D-glucit-1-ylcarbamoylmethyl-benzyl)-D-tartaramid wurde analog Beispiel 1.B. zu N,N'-Bis-[4-(2,3,4,5,6-penta-O-sulfo-D-glucit-1-ylcarbamoylmethyl)-benzyl]-di-O-sulfo-D-tartaramid Dodekanatriumsalz umgesetzt, MS: *m*/*z* 1967,0 (rekonstruiertes M).

Beispiel 31

A. 0,97 g 1-O-(4-Amino-phenyl)-2,3:4,5-di-O-isopropyliden-D-arabinitol hergestellt wie in der europäischen Patentanmeldung 95 100 180.9 vom 9.1.95 beschrieben ) und 0,27 g Benzol-1,3,5-tricarbonsäure-trichlorid wurden in 22 ml Methylenchlorid gelöst, bei 5 °C mit 1,0 ml Triethylamin versetzt und während 60 Stunden bei Raumtemperatur gerührt. Nach Einengen wurde das Rohprodukt an Kieselgel mit Methylenchlorid und Methanol chromatographiert. Man erhielt Benzol-1,3,5-tricarbonsäure-tris-[4-(2,3:4,5-di-O-isopropyliden-D-arabinit-1-yloxy)-phenylamid], MS: *m*/*z* 1127 ([M+H]$^+$).

B. 0,95 g Benzol-1,3,5-tricarbonsäure-tris-[4-(2,3:4,5-di-O-isopropyliden-D-arabinit-1-yloxy)-phenylamid] wurde in 20 ml Dioxan gelöst, mit 3 ml Trifluoressigsäure (35%ig) versetzt und während 7 Stunden bei Rückfluss gerührt. Anschliessend wurde zweimal 50 ml Toluol zugesetzt und jeweils im Wasserstrahlvakuum eingeengt. Der so erhaltene Rückstand wurde während 4 Stunden im Hochvakuum bei Raumtemperatur über Phosphorpentoxid getrocknet. Dabei erhielt man Benzol-1,3,5-tricarbonsäure-tris-(4-D-arabinit-1-yloxy-phenylamid), welches direkt in die nächste Stufe eingesetzt wurde.

C. 0,98 g Benzol-1,3,5-tricarbonsäure-tris-(4-D-arabinit-1-yloxy-phenylamid) wurden in Analogie zu Beispiel 1.B. mit Schwefeltrioxid-trimethylamin-komplex umgesetzt. Dabei wurde Benzol-1,3,5-tricarbonsäure tris-[4-(2,3,4,5-tetra-O-sulfo-D-arabinit-lyloxy)-phenylamid] Dodekanatriumsalz erhalten, MS: *m*/*z* 2110,0 (rekonstruiertes M).

Beispiel 32

A. 18,2 g 5-Hydroxyisophthalsäure wurden in 300 ml Methanol unter Zusatz von 5 ml Schwefelsäure (96%) während 18 Stunden bei Rückfluss gerührt. Nach dem Abkühlen auf 5 °C wurde mit gesättigter NatriumbikarbonatLösung auf pH 8 gestellt, dann das Methanol im Wasserstrahlvakuum abdestilliert. Die heterogene Wasserphase wurde mit Methylenchlorid erschöpfend extrahiert; die organische Phase wurde über Magnesiumsulfat getrocknet, filtriert und

eingeengt. Man erhielt 5-Hydroxy-isophthalsäure-dimethylester , welcher aus Methylenchlorid/n-Hexan umkristallisiert wurde, Smp. 159-161 °C.

B. 2,1 g 5-Hydroxy-isophthalsäure-dimethylester und 2,9 g Toluol-4-sulfonsäure-(R)-2,2-dimethyl-[1,3]dioxolan-4-ylmethylester wurden in 120 ml Dimethylformamid unter Zusatz von 6,9 g Kaliumkarbonat während 3 Stunden bei Rückfluss gerührt. Das Reaktionsgemisch wurde auf Eiswasser gegossen, mit Methylenchlorid extrahiert, die vereinigten Methylenchloridphasen wurden mit Wasser gewaschen, über Magnesiumsulfat getrocknet, filtriert und eingedampft. Man erhielt (S)-5-(2,2-Dimethyl-[1,3]dioxolan-4-ylmethoxy)-isophthalsäure-dimethylester, MS: *m*/*z* 324 ($[M]^+$).

C. 1,40 g (S)-5-(2,2-Dimethyl-[1,3]dioxolan-4-ylmethoxy)-isophthalsäure-dimethylester wurde mit 4 ml Natronlauge (28%ig) in 50 ml Methanol während 16 Stunden bei Raumtemperatur gerührt. Nach Neutralisation mit 6N Salzsäure wurde das Methanol im Wasserstrahlvakuum abdestilliert. Der Rückstand wurde auf Eiswasser gegossen, mit Methylenchlorid extrahiert, die vereinigten Methylenchloridphasen wurden mit Wasser gewaschen, über Magnesiumsulfat getrocknet, filtriert und eingedampft. Man erhielt (S)-5-(2,2-Dimethyl-[1,3]dioxolan-4-ylmethoxy)-isophthalsäure, MS: *m*/*z* 296 ($[M]^+$).

D. 0,59 g (S)-5-(2,2-Dimethyl-[1,3]dioxolan-4-ylmethoxy)-isophthalsäure, gelöst 10 ml Acetonitril, wurde unter Feuchtigkeitsausschluss bei 0 - 5 °C mit 0,44 ml 4-Methylmorpholin versetzt und während 10 Minuten intensiv gerührt. Hierauf wurde 0,70 g 2-Chlor-4,6-dimethoxy-1,3,5-triazin fest hinzugefügt und weitere 2 Stunden bei 0 - 5 °C gerührt. Anschliessend wurde 1,29 g 1-O-(4-Amino-phenyl)-2,3:4,5-di-O-isopropyliden-D-arabinitol, gelöst in 30 ml Acetonitril/Dimethylformamid (2:1), zugefügt und weitere 18 Stunden bei Raumtemperatur gerührt. Nach dem Abdestillieren der Lösungsmittel im Hochvakuum wurde der Rückstand an Kieselgel mit Methylenchlorid und Methanol chromatographiert. Man erhielt (S)-N,N'-Bis-[4-(2,3:4,5-di-O-isopropyliden-D-arabinit-1-yloxy)-phenyl]-5-(2,2-dimethyl-[1,3]dioxolan-4-ylmethoxy)-isophthalamid, MS: *m*/*z* 907 ($[M+H]^+$).

E. 2,05 g (S)-N,N'-Bis-[4-(2,3:4,5-di-O-isopropyliden-D-arabinit-1-yloxy)-phenyl]-5-(2,2-dimethyl-[1,3]dioxolan-4-ylmethoxy)-isophthalamid wurde in 50 ml Dioxan gelöst, mit 15 ml Trifluoressigsäure (70% in Wasser) versetzt und während 12 Stunden bei Rückfluss gerührt. Anschliessend wurde zweimal 50 ml Toluol zugesetzt und jeweils im Wasserstrahlvakuum eingeengt. Der so erhaltene Rückstand wurde während 4 Stunden im Hochvakuum bei Raumtemperatur über Phosphorpentoxid getrocknet. Man erhielt (R)-N,N'-Bis-(4-D-arabinit-1-yloxy-phenyl)-5-(2,3-dihydroxy-propoxy)-isophthalamid, MS: *m*/*z* 707 ($[M+H]^+$).

F. 1,05 g (R)-N,N'-Bis-(4-D-arabinit-1-yloxy-phenyl)-5-(2,3-dihydroxy-propoxy)-isophthalamid wurde in Analogie zu Beispiel 1.B. mit Schwefeltrioxid-trimethylaminkomplex umgesetzt. Dabei wurde (S)-N,N'-Bis-[4-(2,3,4,5-tetra-O-sulfo-D-arabinit-lyloxy)-phenyl]-5-(2,3-bis-sulfooxy-propoxy)-isophthalamid Dekanatriumsalz erhalten, MS: *m*/*z* 1727,0 (rekonstruiertes M).

Beispiel 33

A. 2,35 g 2,3-O-Isopropyliden-1,4-di-O-(4-methyl-phenylsulfonyl)-L-threitol und 1,52 g 4-Hydroxy-benzoesäuremethylester wurden in 75 ml Dimethylformamid unter Zusatz von 3,46 g Kaliumkarbonat während 3 Stunden bei Rückfluss gerührt. Das Reaktionsgemisch wurde auf Eiswasser gegossen, mit Methylenchlorid extrahiert, die vereinigten Methylenchloridphasen wurden mit Wasser gewaschen, über Magnesiumsulfat getrocknet, filtriert und eingedampft. Das Rohprodukt wurde an Kieselgel mit Methylenchlorid und Methanol chromatographiert. Man erhielt 2,3-O-Isopropyliden-1,4-bis-O-(4-methoxycarbonyl-phenyl)-L-threitol, MS: *m*/*z* 430 ($[M]^+$).

B. 1,81 g 2,3-O-Isopropyliden-1,4-bis-O-(4-methoxycarbonyl-phenyl)-L-threitol wurde in 50 ml Methanol unter Zusatz von 5 ml Natronlauge (28%ig) während 20 Stunden bei 50 °C gerührt. Das Reaktionsgemisch wurde danach auf 10 °C gekühlt, mit Salzsäure auf einen pH-Wert von 2 gestellt, und das Methanol wurde im Wasserstrahlvakuum abdestilliert. Zum Rückstand wurde 100 ml Wasser gegeben, 1 Stunde im Eisbad gerührt, dann filtriert und getrocknet. Man erhielt 1,4-Bis-O-(4-carboxy-phenyl)-L-threitol, MS: *m*/*z* 361 ($[M-H]^-$).

C. 1,33 g 1,4-Bis-O-(4-carboxy-phenyl)-L-threitol wurde in 20 ml Pyridin mit 3,8 ml Acetanhydrid während 20 Stunden bei Raumtemperatur gerührt. Das Reaktionsgemisch wurde auf Eiswasser gegossen, mit Methylenchlorid extrahiert, die vereinigten Methylenchloridphasen wurden mit Wasser gewaschen, über Magnesiumsulfat getrocknet, filtriert und eingedampft. Man erhielt 2,3-Di-O-acetyl-1,4-bis-O-(4-carboxy-phenyl)-L-threitol, welches aus Methanol/Methylenchlorid umkristallisiert wurde, MS: *m*/*z* 387 ($[M-AcO]^-$).

D. 0,89 g 2,3-Di-O-acetyl-1,4-bis-O-(4-carboxy-phenyl)-L-threitol, gelöst 10 ml Acetonitril, wurde unter Feuchtigkeitsausschluss bei 0 - 5 °C mit 0,44 ml 4-Methylmorpholin versetzt und während 10 Minuten intensiv gerührt. Hierauf wurde 0,70 g 2-Chlor-4,6-dimethoxy-1,3,5-triazin fest hinzugefügt und weitere 2 Stunden bei 0 - 5 °C gerührt. Anschliessend wurde 1,29 g 1-0-(4-Amino-phenyl)-2,3:4,5-di-O-isopropyliden-D-arabinitol, gelöst in 30 ml Acetonitril/ Dimethylformamid (2:1), zugefügt und weitere 18 Stunden bei Raumtemperatur gerührt. Nach dem Abdestillieren der Lösungsmittel im Hochvakuum wurde der Rückstand an Kieselgel mit Methylenchlorid und Methanol chromatographiert. Man erhielt 2,3-Di-O-acetyl-1,4-bis-O-[4-[4-(2,3:4,5-di-O-isopropyliden-D-arabinit-1-yloxy)-phenylcarbamoyl]-phenyl]-L-threitol, MS: *m*/*z* 1057 ($[M+H]^+$).

E. 2,9 g 2,3-Di-O-acetyl-1,4-bis-O-[4-[4-(2,3:4,5-di-O-isopropyliden-D-arabinit-1-yloxy)-phenylcarbamoyl]-phenyl]-L-threitol wurde in 40 ml Dioxan gelöst, mit 2,8 ml Trifluoressigsäure und 5,6 ml destilliertem Wasser versetzt und während 3 Stunden unter Rückfluss gerührt; anschliessend wurde zweimal 100 ml Toluol zugesetzt und jeweils im Wasserstrahlvakuum eingeengt. Der so erhaltene Rückstand wurde während 4 Stunden im Hochvakuum bei Raumtemperatur über Phosphorpentoxid getrocknet. Man erhielt dabei 2,3-Di-O-acetyl-1,4-bis-O-[4-(4-D-arabinit-1-yloxy-phenylcarbamoyl)-phenyl]-L-threitol, welches direkt in die nächste Stufe eingesetzt wurde.

F. 2,08 g 2,3-Di-O-acetyl-1,4-bis-O-[4-(4-D-arabinit-1-yloxy-phenylcarbamoyl)-phenyl]-L-threitol wurde in 50 ml Methanol mit 1,0 g Kaliumkarbonat unter Zusatz von 15 ml Wasser/ Dimethylformamid (2:1) während 18 Stunden bei Raumtemperatur gerührt. Die Lösungsmittel wurden im Hochvakuum abdestilliert, dann wurde der Rückstand in 50 ml Wasser aufgenommen, filtriert und getrocknet. Man erhielt 1,4-Bis-O-[4-(4-D-arabinit-1-yloxy-phenylcarbamoyl)-phenyl]-L-threitol. Elementaranalyse berechnet für $C_{40}H_{48}N_2O_{16}$: C = 59,11%, H = 5,95%, N = 3,45%; gefunden: C = 58,69%, H = 6,07%, N = 3,10%.

G. 1,20 g 1,4-Bis-O-[4-(4-D-arabinit-1-yloxy-phenylcarbamoyl)-phenyl]-L-threitol wurde in Analogie zu Beispiel 1.B. mit Schwefeltrioxid-trimethylaminkomplex umgesetzt. Dabei wurde 1,4-Bis-O-[4-[4-(2,3,4,5-tetra-O-sulfo-D-arabinit-1-yloxy)-phenyl-carbamoyl]-phenyl]-2,3-di-O-sulfo-L-threitol Dekanatriumsalz erhalten, MS: *m/z* 1833,0 (rekonstruiertes M).

Beispiel 34

A. 16,7 g 2,5-O-Methylen-1,6-bis-O-(4-methyl-phenylsulfonyl)-D-mannitol (B. Lamm et al., Acta Chem. Scand. B 41, 202 (1987)) wurde unter Zusatz von 0,1 g p-Toluolsulfonsäure in 80 ml Trimethyl-orthoformiat unter Argon während 16 Stunden bei Raumtemperatur gerührt. Nach Neutralisation mit Kaliumkarbonat und anschliessender Filtration wurde das Filtrat eingeengt. Der Rückstand wurde aus Methanol umkristallisiert. Man erhielt 3,4-O-Methoxymethylen-2,5-O-methylen-1,6-bis-O-(4-methyl-phenylsulfonyl)-D-mannitol, MS: *m/z* 545 ($[M+H]^+$).

B. 4,36 g 3,4-O-Methoxymethylen-2,5-O-methylen-1,6-bis-O-(4-methyl-phenylsulfonyl)-D-mannitol wurde in Analogie zu Beispiel 33.A. mit 2,44 g 4-Hydroxy-benzoesäuremethylester umgesetzt und aufgearbeitet. Das Rohprodukt wurde an Kieselgel mit Methylenchlorid und Methanol chromatographiert. Man erhielt 1,6-Bis-O-(4-methoxycarbonyl-phenyl)-3,4-0-methoxymethylen-2,5-O-methylen-D-mannitol, MS: *m/z* 504 ($[M]^+$).

C. 3,12 g 1,6-Bis-O-(4-methoxycarbonyl-phenyl)-3,4-O-methoxymethylen-2,5-O-methylen-D-mannitol wurde in 100 ml Methanol mit 6 ml Natronlauge (28%) während 6 Stunden bei Rückfluss gerührt. Nach dem Abkühlen auf 10 °C wurde mit verdünnter Salzsäure der pH-Wert des Reaktionsgemisches auf pH 2 gestellt, dann das Methanol im Wasserstrahlvakuum abdestilliert. Zum Rückstand wurden 100 ml Wasser gegeben, 1 Stunde im Eisbad gerührt, dann filtriert, und der Rückstand wurde getrocknet. So erhielt man 1,6-Bis-O-(4-carboxy-phenyl)-2,5-O-methylen-D-mannitol, MS: *m/z* 433 ($[M-H]^-$).

D. 2,05 g 1,6-Bis-O-(4-carboxy-phenyl)-2,5-O-methylen-D-mannitol wurde in 55 ml 20%iger Schwefelsäure in Methanol während 18 Stunden bei Rückfluss gerührt. Dann wurden nach dem Abkühlen auf Raumtemperatur 50 ml Eiswasser zugesetzt, und das Methanol wurde im Wasserstrahlvakuum abdestilliert. Die verbleibende heterogene Wasserphase wurde filtriert, der Rückstand getrocknet. Man erhielt 1,6-Bis-O-(4-methoxycarbonyl-phenyl)-D-mannitol, MS: *m/z* 449 ($[M-H]^-$).

E. 2,33 g 1,6-Bis-O-(4-methoxycarbonyl-phenyl)-D-mannitol wurde in Analogie zu Beispiel 34. C. umgesetzt und aufgearbeitet. Man erhielt 1,6-Bis-O-(4-carboxy-phenyl)-D-mannitol, MS: *m/z* 421 ($[M-H]^-$).

F. 2,10 g 1,6-Bis-O-(4-carboxy-phenyl)-D-mannitol wurde in 30 ml Pyridin mit 5,1 ml Acetanhydrid während 20 Stunden bei Raumtemperatur gerührt. Das Reaktionsgemisch wurde extrahierend mit Eiswasser und Methylenchlorid aufgearbeitet. Die organische Phase wurde über Magnesiumsulfat getrocknet, filtriert und eingeengt. Der Rückstand wurde aus Methanol und Methylenchlorid umkristallisiert. Man erhielt 2,3,4,5-Tetra-O-acetyl-1,6-bis-O-(4-carboxy-phenyl)-D-mannitol, MS: *m/z* 589 ($[M-H]^-$).

G. 1,18 g 2,3,4,5-Tetra-O-acetyl-1,6-bis-O-(4-carboxy-phenyl)-D-mannitol wurde in Analogie zu Beispiel 33.D. mit 1-O-(4-Amino-phenyl)-2,3:4,5-di-O-isopropyliden-D-arabinitol umgesetzt. Das Rohprodukt wurde an Kieselgel mit Methylenchlorid und Methanol chromatographiert. Man erhielt 2,3,4,5-Tetra-O-acetyl-1,6-bis-O-[4-[4-(2,3:4,5-di-O-isopropyliden-D-arabinit-1-yloxy)-phenylcarbamoyl]-phenyl]-D-mannitol, MS: *m/z* 1201 ($[M+H]^+$).

H. 2,28 g 2,3,4,5-Tetra-O-acetyl-1,6-bis-O-[4-[4-(2,3:4,5-di-O-isopropyliden-D-arabinit-1-yloxy)-phenylcarbamoyl]-phenyl]-D-mannitol wurde mit 2,0 g Kaliumkarbonat in 60 ml Methanol unter Zusatz von 10 ml Wasser während 18 Stunden bei Raumtemperatur gerührt. Das Methanol wurde im Wasserstrahlvakuum abdestilliert, zum Rückstand wurde 50 ml Wasser zugegeben und es wurde filtriert. Nach Trocknung über Phosphorpentoxid erhielt man 1,6-O-Bis-[4-[4-(2,3:4,5-di-O-isopropyliden-D-arabinit- lyloxy)-phenylcarbamoyl]-phenyl]-D-mannitol, MS: *m/z* 1033 ($[M+H]^+$).

I. 1,67 g 1,6-Bis-O-[4-[4-(2,3:4,5-di-O-isopropyliden-D-arabinit-lyloxy)-phenylcarbamoyl]-phenyl]-D-mannitol wurde in 50 ml Dioxan gelöst, mit 15 ml Trifluoressigsäure (70% in Wasser) versetzt und während 3 Stunden bei Rückfluss gerührt. Anschliessend wurde zweimal 50 ml Toluol zugesetzt und jeweils im Wasserstrahlvakuum eingeengt. Der so erhaltene Rückstand wurde während 4 Stunden im Hochvakuum bei Raumtemperatur über Phosphorpentoxid getrocknet. Dabei erhielt man 1,6-O-Bis-[4-(4-D-arabinit-1-yloxy-phenylcarbamoyl)-phenyl]-D-mannitol, IR: (KBr, $cm^{-1}$): 3307, 1638, 1607, 1538, 1249, 1076, 826.

K. 1,31 g 1,6-Bis-O-[4-(4-D-arabinit-1-yloxy-phenylcarbamoyl)-phenyl]-D-mannitol wurde in Analogie zu Beispiel 1.B. mit Schwefeltrioxid-trimethylamin-komplex umgesetzt. Dabei wurde 1,6-Bis-O-[4-[4-(2,3,4,5-tetra-O-sulfo-D-arabinit-1-yloxy)-phenylcarbamoyl]-phenyl]-2,3,4,5-tetra-O-sulfo-D-mannitol Dodekanatriumsalz erhalten, MS: *m*/*z* 2097,0 (rekonstruiertes M).

Beispiel 35

A. 10,01 g meso-Weinsäure Monohydrat wurde in 35 ml Acetanhydrid unter Zusatz von 0,3 ml konzentrierter Schwefelsäure 15 Minuten unter Argon bei Rückfluss gerührt; nach dem Abkühlen des Reaktionsgemisches auf 5 °C wurde unter Rühren 150 ml Diethylether zugegeben, dann wurde filtriert und das Filtrat eingeengt. Man erhielt so Di-O-acetyl-meso-weinsäureanhydrid, MS: *m*/*z* 235,2 ($[M+H]^+$) (=Dicarbonsäure), IR: (KBr, $cm^{-1}$): 1800,1752,1214.

B. 2,16 g Di-O-acetyl-meso-weinsäureanhydrid und 2,60 g 1-O-(4-Amino-phenyl)-2,3:4,5-di-O-isopropyliden-D-arabinitol wurden in 120 ml Methylenchlorid während 16 Stunden bei Raumtemperatur gerührt. Nach dem Abdestillieren des Lösungsmittels wurde das Produkt an Kieselgel mit Methylenchlorid und Methanol chromatographiert. Man erhielt ein Gemisch von (2R,3S)- und (2S,3R)-Di-O-acetyl-N-[4-(2,3:4,5-di-O-isopropyliden-D-arabinit-1-yloxy)-phenyl]-meso-tartaramsäure, MS: *m*/*z* 538,4 ($[M-H]^-$).

C. 0,54 g Gemisch von (2R,3S)- und (2S,3R)-Di-O-acetyl-N-[4-(2,3:4,5-di-O-isopropyliden-D-arabinit-1-yloxy)-phenyl]-meso-tartaramsäure wurden in 15 ml Methylenchlorid mit 0,10 ml Oxalylchlorid unter Zusatz von 1 Tropfen Dimethylformamid bei -20 °C zur Reaktion gebracht; nach 2 Stunden wurden zuerst 0,28 ml Triethylamin, dann 0,32 g 1-O-(4-Amino-phenyl)-2,3:4,5-di-O-isopropyliden-D-arabinitol gelöst in 5 ml Methylenchlorid bei -25 °C zugetropft. Nach dem Aufwärmen auf Raumtemperatur wurde das Lösungsmittel abdestilliert, und der Rückstand wurde an Kieselgel mit Methylenchlorid und Methanol chromatographiert. Man erhielt Di-O-acetyl-N,N'-bis-[4-(2,3:4,5-di-O-isopropyliden-D-arabinit-1-yloxy)-phenyl]-meso-tartaramid, MS: *m*/*z* 845,6 ($[M+H]^+$).

D. 2,32 g Di-O-acetyl-N,N'-bis-[4-(2,3:4,5-di-O-isopropyliden-D-arabinit-1-yloxy)-phenyl]-meso-tartaramid wurde in Analogie zu Beispiel 34. H. mit 2,78 g Kaliumkarbonat in Methanol/Wasser während 3 Stunden bei Raumtemperatur gerührt. Das Reaktionsgemisch wurde extrahierend mit Wasser und Essigester aufgearbeitet. Das Rohprodukt wurde an Kieselgel mit Methylenchlorid und Acetonitril chromatographiert. Man erhielt N,N'-Bis-[4-(2,3:4,5-di-O-isopropyliden-D-arabinit-lyloxy)-phenyl]-meso-tartaramid, MS: *m*/*z* 761,3 ($[M+H]^+$).

E. 1,40 g N,N'-Bis-[4-(2,3:4,5-di-O-isopropyliden-D-arabinit-lyloxy)-phenyl]-meso-tartaramid wurde in 45 ml Dioxan gelöst, mit 15 ml Trifluoressigsäure (70% in Wasser) versetzt und während 3 Stunden bei Rückfluss gerührt. Anschliessend wurde zweimal 50 ml Toluol zugesetzt und jeweils im Wasserstrahlvakuum eingeengt. Der so erhaltene Rückstand wurde während 16 Stunden im Hochvakuum bei bei 50 °C über Phosphorpentoxid getrocknet. Man erhielt N,N'-Bis-(4-D-arabinit-1-yloxy-phenyl)-meso-tartaramid, IR: (KBr, $cm^{-1}$): 3390, 3304, 1661, 1604, 1539, 1513, 1241, 1077, 1042,824.

F. 0,90 g N,N'-Bis-(4-D-arabinit-1-yloxy-phenyl)-meso-tartaramid wurde in Analogie zu Beispiel 1.B. mit Schwefeltrioxid-trimethylamin-komplex umgesetzt. Dabei wurde Di-O-sulfo-N,N'-bis-[4-(2,3,4,5-tetra-O-sulfo-D-arabinit-1-yloxy)-phenyl]-meso-tartaramid Dekanatriumsalz erhalten, MS: *m*/*z* 1621,0 (rekonstruiertes M).

Beispiel 36

A. 14,6 g 2,3:4,5-Di-O-isopropyliden-D-arabinitol (European Patent Application 247721 A1, 21. April 1987, DOW CHEMICAL COMPANY) wurde in 55 ml Pyridin mit 12,7 g p-Toluolsulfonylchlorid unter Zusatz von 0,05 g 4-(N, N-Dimethylamino)-pyridin umgesetzt. Nach dem Abdestillieren des Lösungsmittels wurde das Reaktionsgemisch extrahierend mit verdünnter Salzsäure/Eis und Essigester aufgearbeitet. Man erhielt 2,3:4,5-Di-O-isopropyliden-1-O-(4-methyl-phenylsulfonyl)-D-arabinitol, welches aus Essigester/n-Hexan umkristallisiert wurde, Elementaranalyse berechnet für $C_{18}H_{26}O_7S$: C = 55,94%, H = 6,78%; gefunden: C = 56,02%, H = 6,77%.

B. 11,60 g 2,3:4,5-Di-O-isopropyliden-1-O-(4-methyl-phenylsulfonyl)-D-arabinitol wurde in Analogie zu Beispiel 33.A. mit 8,50 g 4'-Benzyloxy-biphenyl-4-ol (H. Kapitza & R. Zentel, Makromol. Chem. 192, 1859 (1991)) und 20,7 g Kaliumkarbonat in 350 ml N,N-Dimethylformamid umgesetzt. Nach dem Abdestillieren des Lösungsmittels wurde das Rohprodukt an Kieselgel mit Methylenchlorid und Methanol chromatographiert. Man erhielt 1-O-(4'-Benzyloxy-biphenyl-4-yl)-2,3:4,5-di-O-isopropyliden-D-arabinitol, MS: *m*/*z* 490 ($[M]^+$).

C. 9,80 g 1-O-(4'-Benzyloxy-biphenyl-4-yl)-2,3:4,5-di-O-isopropyliden-D-arabinitol wurde durch katalytische Hydrierung mit 2,5 g Pd-Kohle (10%) in Essigester debenzyliert. Man erhielt 1-O-(4'-Hydroxy-biphenyl-4-yl)-2,3:4,5-di-O-isopropyliden-D-arabinitol, Elementaranalyse berechnet für $C_{23}H_{28}O_6$: C = 68,98%, H = 7,05%; gefunden: C = 68,76%, H = 7,10%.

D. 2,00 g 1-O-(4'-Hydroxy-biphenyl-4-yl)-2,3:4,5-di-O-isopropyliden-D-arabinitol und 1,18 g 2,3-O-Isopropyliden-1,4-bis-O-(4-methyl-phenylsul-fonyl)-L-threitol wurden wie in Beispiel 33.A. beschrieben umgesetzt und aufgearbeitet. Das Rohprodukt wurde an Kieselgel mit n-Hexan, Methylenchlorid und Acetonitril chromatographiert. Man erhielt 1,4-Bis-O-[4'-(2,3:4,5-di-O-isopropyliden-D-arabinit-1-yloxy)-biphenyl-4-yl]-2,3-0-isopropyliden-L-threitol, MS: *m*/*z* 927,4 ($[M+H]^+$).

E. 1,63 g 1,4-Bis-O-[4'-(2,3:4,5-di-O-isopropyliden-D-arabinit-1-yloxy)-biphenyl-4-yl]-2,3-O-isopropyliden-L-threitol wurde in 50 ml Dioxan gelöst, mit 15 ml Trifluoressigsäure (70% in Wasser) versetzt und während 3 Stunden bei Rückfluss gerührt. Nach dem Abkühlen auf Raumtemperatur wurde eingeengt, der Rückstand wurde zweimal mit 50 ml Toluol versetzt und azeotrop abdestilliert. Der so erhaltene Rückstand wurde in 50 ml Wasser aufgenommen, 1 Stunde bei Raumtemperatur gerührt, dann auf 10 °C gekühlt und filtriert. Nach dem Trocknen über Phosphorpentoxid bei 50 °C erhielt man 1,4-Bis-O-(4'-D-arabinit-1-yloxy-biphenyl-4-yl)-L-threitol, Elementaranalyse berechnet für $C_{38}H_{46}O_{14}$: C = 62,80%, H = 6,38%; gefunden: C = 63,12%, H = 6,21%.

F. 1,09 g 1,4-Bis-O-(4'-D-arabinit-1-yloxy-biphenyl-4-yl)-L-threitol wurde in Analogie zu Beispiel 1.B. mit Schwefeltrioxid-trimethylamin-Komplex umgesetzt. Dabei wurde 2,3-Di-O-sulfo-1,4-bis-O-[4'-(2,3,4,5-tetra-O-sulfo-D-arabinit-1-yloxy)-biphenyl-4-yl]-L-threitol Dekanatriumsalz erhalten, MS: *m*/*z* 1746,0 (rekonstruiertes M).

Beispiel 37

A. 1,37 g 2,3:4,5-Di-O-isopropyliden-1,6-bis-O-(4-methyl-phenylsulfonyl)-galactitol und 1,96 g 1-O-(4'-Hydroxy-biphenyl-4-yl)-2,3:4,5-di-O-isopropyliden-D-arabinitol (Beispiel 36.C.) wurden in Analogie zu Beispiel 33.A. umgesetzt. Das Rohprodukt wurde an Kieselgel mit n-Hexan und Methylenchlorid chromatographiert. Man erhielt 1,6-Bis-O-[4'-(2,3:4,5-di-O-isopropyliden-D-arabinit-1-yloxy)-biphenyl-4-yl]-2,3:4,5-di-O-isopropyliden-galactitol, MS: *m*/*z* 1026,5 ($[M]^+$).

B. 1,70 g 1,6-Bis-O-[4'-(2,3:4,5-di-O-isopropyliden-D-arabinit-1-yloxy)-biphenyl-4-yl]-2,3:4,5-di-O-isopropyliden-galactitol wurde in Analogie zu Beispiel 35.E. mit Trifluoressigsäure (75%ig) in Dioxan umgesetzt und analog aufgearbeitet. Man erhielt 1,4-Bis-O-(4'-D-arabinit-1-yloxy-biphenyl-4-yl)-galactitol, Elementaranalyse berechnet für $C_{40}H_{50}O_{16}$: C = 61,06%, H = 6,41%; gefunden: C = 60,99%, H = 6,50%.

C. 1,10 g 1,4-Bis-O-(4'-D-arabinit-1-yloxy-biphenyl-4-yl)-galactitol wurde in Analogie zu Beispiel 1.B. mit Schwefeltrioxid-trimethylamin-komplex umgesetzt. Dabei wurde 2,3,4,5-Tetra-O-sulfo-1,6-bis-O-[4'-(2,3,4,5-tetra-O-sulfo-D-arabinit-1-yloxy)-biphenyl-4-yl]-galactitol Dodekanatriumsalz erhalten, MS: *m*/*z* 2011,0 (rekonstruiertes M).

Beispiel 38

A. 1,96 g 1-O-(4'-Hydroxy-biphenyl-4-yl)-2,3:4,5-di-O-isopropyliden-D-arabinitol (Beispiel 36.C.) und 0,56 g 1,2:5,6-Dianhydro-3,4-O-isopropyliden-D-mannitol (Y. Le Merrer et al. Heterocyles 25, 541 (1987)) wurden in 130 ml Dimethylformamid unter Zusatz von 4,15 g Kaliumkarbonat während 6 Stunden unter Argon bei 100 °C gerührt. Das Reaktionsgemisch wurde auf Eiswasser gegossen, mit Methylenchlorid extrahiert, die vereinigten Methylenchloridphasen wurden mit Wasser gewaschen, über Magnesiumsulfat getrocknet, filtriert und eingedampft. Das so erhaltene Rohprodukt wurde an Kieselgel mit Hexan und Essigester chromatographiert. Man erhielt dabei 1,6-Bis-O-[4'-(2,3:4,5-di-O-isopropyliden-D-arabinit-1-yloxy)-biphenyl-4-yl]-3,4-O-isopropyliden-D-mannitol, MS: *m*/*z* 1010,6 ($[M+Na]^+$).

B. 1,60 g 1,6-Bis-O-[4'-(2,3:4,5-di-O-isopropyliden-D-arabinit-1-yloxy)-biphenyl-4-yl]-3,4-O-isopropyliden-D-mannitol wurde in Analogie zu Beispiel 35.E. mit Trifluoressigsäure (50 %ig) in Dioxan umgesetzt. Man erhielt 1,6-Bis-O-(4'-D-arabinit-1-yloxy-biphenyl-4-yl)-D-mannitol, IR: (KBr, $cm^{-1}$): 3381, 1607, 1499, 1242, 1176, 1045, 823.

C. 1,18 g 1,6-Bis-O-(4'-D-arabinit-1-yloxy-biphenyl-4-yl)-D-mannitol wurde in Analogie zu Beispiel 1.B. mit Schwefeltrioxid-trimethylaminkomplex umgesetzt. Dabei wurde 2,3,4,5-Tetra-O-sulfo-1,6-bis-O-[4'-(2,3,4,5-tetra-O-sulfo-D-arabinit-1-yloxy)-biphenyl-4-yl]-D-mannitol Dodekanatriumsalz erhalten, MS: *m*/*z* 2011,0 (rekonstruiertes M).

Beispiel 39

A. 30,1 g Anisol und 26,0 g 1,1-Dichlor-2,2-diethoxy-ethan wurden in 56 ml Eisessig unter Zusatz von 42 ml konzentrierter Schwefelsäure bei 5 °C unter Argon umgesetzt. Das Reaktionsgemisch wurde auf 350 ml Eiswasser gegossen und danach abgenutscht; der Kristallbrei wurde mit Wasser gewaschen und getrocknet. Das so erhaltene Rohprodukt wurde aus Methylenchlorid und n-Hexan umkristallisiert. Man erhielt so 4,4'-Dimethoxy-1,1'-(2,2-dichlor-ethyliden)-dibenzol, Smp.: 113-116 °C.

B. 6,0 g 4,4'-Dimethoxy-1,1'-(2,2-dichlor-ethyliden)-dibenzol wurde mit 9,7 g Kalium-tert.-butylat bei 190 °C erhitzt. Nach der Zugabe von Eiswasser wurde filtriert, mit Wasser gewaschen und aus Methylenchlorid und n-Hexan umkristallisiert. Man erhielt 4,4'-Dimethoxy-1,1'-ethindiyl-dibenzol, Smp.: 140-142 °C.

C. 16,4 g 4,4'-Dimethoxy-1,1'-ethindiyl-dibenzol wurden in 240 ml Methylenchlorid unter Argon bei -75 °C mit

12,6 ml Bortribromid umgesetzt. Nach der Hydrolyse wurde das Produkt extrahierend mit Ether isoliert. Die organische Phase wurde über Magnesiumsulfat getrocknet, filtriert und eingeengt. Das Rohprodukt wurde an Kieselgel mit n-Hexan und Essigester chromatographiert. Dabei wurde 4,4'-Ethyndiyl-diphenol erhalten, MS: *m*/*z* 210,0 ($[M]^+$).

D. 2,56 g 4,4'-Ethyndiyl-diphenol und 4,71 g 2,3:4,5-Di-O-isopropyliden-1-O-(4-methyl-phenylsulfonyl)-D-arabinitol (Beispiel 36.A.) wurden in 500 ml Dimethylformamid unter Zusatz von 8,43 g Kaliumkarbonat während 70 Stunden bei 60 °C umgesetzt. Das Reaktionsgemisch wurde anschliessend auf Eiswasser gegossen, mit Methylenchlorid extrahiert, die vereinigten Methylenchloridphasen wurden mit Wasser gewaschen, über Magnesiumsulfat getrocknet, filtriert und eingeengt. Das so erhaltene Rohprodukt wurde an Kieselgel mit Methylenchlorid und Acetonitril chromatographiert. Man erhielt 1-O-[4-(4-Hydroxy-phenylethynyl)-phenyl]-2,3:4,5-di-O-isopropyliden-D-arabinitol, MS: *m*/*z* 424,0 ($[M]^+$).

E. 2,12 g 1-O-[4-(4-Hydroxy-phenylethynyl)-phenyl]-2,3:4,5-di-O-isopropyliden-D-arabinitol und 1,43 g 2,3:4,5-Di-O-isopropyliden-1,6-bis-O-(4-methyl-phenylsulfonyl)-galactitol wurden in Analogie zu Beispiel 33.A. unter Zusatz von 1,73 g Kaliumkarbonat umgesetzt und aufgearbeitet. Das Rohprodukt wurde an Kieselgel mit n-Hexan und Methylenchlorid chromatographiert. Man erhielt 1,6-Bis-O-[4-[4-(2,3:4,5-di-O-isopropyliden-D-arabinit-1-yloxy)-phenylethynyl]-phenyl]-2,3:4,5-di-O-isopropyliden-galactitol, IR: (KBr, $cm^{-1}$): 2230, 1608, 1516, 1246, 1173, 1064, 1025, 836.

F. 1,80 g 1,6-Bis-O-[4-[4-(2,3:4,5-di-O-isopropyliden-D-arabinit-1-yloxy)-phenylethynyl]-phenyl]-2,3:4,5-di-O-isopropyliden-galactitol wurde in Analogie zu Beispiel 35.E. während 7 Stunden bei Rückfluss umgesetzt und anschliessend analog aufgearbeitet. Man erhielt 1,6-Bis-O-[4-(4-D-arabinit-1-yloxy-phenylethynyl)-phenyl]-galactitol, welches direkt in die nächste Stufe eingesetzt wurde.

G. 1,40 g 1,6-Bis-O-[4-(4-D-arabinit-1-yloxy-phenylethynyl)-phenyl]-galactitol wurde in Analogie zu Beispiel 1.B. mit Schwefeltrioxid-trimethylamin-komplex umgesetzt. Dabei wurde 2,3,4,5-Tetra-O-sulfo-1,6-bis-O-[4-[4-(2,3,4,5-tetra-O-sulfo-D-arabinit-1-yloxy)-phenyl-ethynyl]-phenyl]-galactitol Dodekanatriumsalz erhalten, MS: *m*/*z* 2060,0 (rekonstruiertes M).

Beispiel 40

A. 9,61 g 2,3-Dihydroxy-naphthalin wurden in 70 ml 0,95 M Natriumethylat-Lösung in Ethanol gelöst, mit 6,92 ml Benzylchlorid versetzt und während 4 Stunden bei Rückfluss gerührt. Nach dem Abkühlen auf Raumtemperatur wurde extrahierend mit Wasser, verdünnter Salzsäure und Methylenchlorid aufgearbeitet. Das Rohprodukt wurde an Kieselgel mit n-Hexan und Methylenchlorid chromatographiert. Dabei wurde 3-Benzyloxy-naphthalin-2-ol erhalten, MS: *m*/*z* 250,0 ($[M]^+$).

B. 2,80 g 3-Benzyloxy-naphthalin-2-ol und 4,33 g 2,3:4,5-Di-O-isopropyliden-1-0-(4-methyl-phenylsulfonyl)-D-arabinitol (Beispiel 36.A.) wurde in Analogie zu Beispiel 33.A. während 6 Stunden bei 100 °C umgesetzt und analog aufgearbeitet. Das so erhaltene Rohprodukt wurde an Kieselgel mit Methylenchlorid chromatographiert. Das Produkt wurde aus n-Hexan umkristallisiert. Man erhielt 1-O-(3-Benzyloxy-naphthalin-2-yl)-2,3:4,5-di-O-isopropyliden-D-arabinitol, MS: *m*/*z* 464,0 ($[M]^+$).

C. 4,80 g 1-O-(3-Benzyloxy-naphthalin-2-yl)-2,3:4,5-di-O-isopropyliden-D-arabinitol wurde mit 1,0 g Pd-Kohle (10%) als Katalysator in Methanol bei Raumtemperatur und Normaldruck während 5 Stunden hydriert. Nach dem Abfiltrieren des Katalysators wurde das Lösungsmittel im Wasserstrahlvakuum abdestilliert, und der Rückstand wurde an Kieselgel mit Methylenchlorid und Methanol chromatographiert. Man erhielt 1-O-(3-Hydroxy-naphthalin-2-yl)-2,3:4,5-di-O-isopropyliden-D-arabinitol, MS: *m*/*z* 374,0 ($[M]^+$).

D. 1,10 g 1-O-(3-Hydroxy-naphthalin-2-yl)-2,3:4,5-di-O-isopropyliden-D-arabinitol und 0,84 g 2,3:4,5-Di-O-isopropyliden-1,6-bis-O-(4-methyl-phenylsulfonyl)-galactitol wurden in Analogie zu Beispiel 33.A. während 6 Stunden bei 100 °C umgesetzt und analog aufgearbeitet. Das Rohprodukt wurde an Kieselgel mit n-Hexan und Essigester chromatographiert. Dabei erhielt man 1,6-Bis-O-[3-(2,3:4,5-di-O-isopropyliden-D-arabinit-1-yloxy)-naphthalin-2-yl]-2,3:4,5-di-O-isopropyliden-galactitol, IR: (Film, $cm^{-1}$): 1628, 1510, 1256, 1216, 1176, 1096, 1017, 747.

E. 0,90 g 1,6-Bis-O-[3-(2,3:4,5-di-O-isopropyliden-D-arabinit-1-yloxy)-naphthalin-2-yl]-2,3:4,5-di-O-isopropyliden-galactitol wurde in Analogie zu Beispiel 35.E. mit 10 ml Trifluoressigsäure (70% in Wasser) in 25 ml Dioxan umgesetzt. Nach der Aufarbeitung wurde 1,6-Bis-O-(3-D-arabinit-1-yloxy-naphthalin-2-yl)-galactitol erhalten, welches direkt in die nächste Stufe eingesetzt wurde.

F. 0,83 g 1,6-Bis-O-(3-D-arabinit-1-yloxy-naphthalin-2-yl)-galactitol wurde in Analogie zu Beispiel 1.B. mit Schwefeltrioxid-trimethylaminkomplex umgesetzt. Dabei wurde 1,6-Bis-O-[3-(2,3,4,5-tetra-O-sulfo-D-arabinit-1-yloxy)-naphthalin-2-yl]-2,3,4,5-tetra-O-sulfo-galactitol Dodekanatriumsalz erhalten, MS: *m*/*z* 1960,0 (rekonstruiertes M).

Beispiel 41

A. 1,53 g 1-O-(3-Hydroxy-naphthalin-2-yl)-2,3:4,5-di-O-isopropyliden-D-arabinitol (Beispiel 40.C.) und 0,97

g 2,3-O-Isopropyliden-1,4-di-O-(4-methyl-phenylsulfonyl)-L-threitol wurden analog Beispiel 33.A. während 21 Stunden bei 100 °C gerührt und analog aufgearbeitet. Das Rohprodukt wurde an Kieselgel mit Toluol und Essigester chromatographiert. So erhielt man 1,4-Bis-O-[3-(2,3:4,5-di-O-isopropyliden-D-arabinit-1-yloxy)-naphthalin-2-yl]-2,3-O-isopropyliden-L-threitol, welches direkt in die nächste Stufe eingesetzt wurde.

B. 0,65 g 1,4-Bis-O-[3-(2,3:4,5-di-O-isopropyliden-D-arabinit-1-yloxy)-naphthalin-2-yl]-2,3-O-isopropyliden-L-threitol wurde in Analogie zu Beispiel 35.E. während 7 Stunden bei Rückfluss gerührt und analog aufgearbeitet. Man erhielt 1,4-Bis-O-(3-D-arabinit-1-yloxy-naphthalin-2-yl)-L-threitol, welches direkt in die nächste Stufe eingesetzt wurde.

C. 0,50 g 1,4-Bis-O-(3-D-arabinit-1-yloxy-naphthalin-2-yl)-L-threitol wurde in Analogie zu Beispiel 1.B. mit Schwefeltrioxid-trimethylaminkomplex umgesetzt. Dabei wurde 2,3-Di-O-sulfo-1,4-bis-O-[3-(2,3,4,5-tetra-O-sulfo-D-arabinit-1-yloxy)-naphthalin-2-yl]-L-threitol Dekanatriumsalz erhalten, MS: *m*/*z* 1695,0 (rekonstruiertes M).

Beispiel 42

0,50 g 2,3,4,5-Tetra-O-sulfo-1,6-bis-O-[4-[4-(2,3,4,5-tetra-O-sulfo-D-arabinit-1-yloxy)-phenylethynyl]-phenyl]-galactitol Dodekanatriumsalz (Beispiel 39.G.) wurde mit 0,35 g Palladium auf Kohle (10%) in 5 ml Wasser bei Normaldruck hydriert. Nach dem Abfiltrieren des Katalysators wurde das Filtrat lyophilisiert. Man erhielt so 2,3,4,5-Tetra-O-sulfo-1,6-bis-O-[4-[2-[4-(2,3,4,5-tetra-O-sulfo-D-arabinit-1-yloxy)-phenyl]-ethyl]-phenyl]-galactitol Dodekanatriumsalz, MS: *m*/*z* 2067,0 (rekonstruiertes M).

Beispiel 43

A. 30,00 g Bis-(4-Hydroxy-phenyl)-methan wurde in 240 ml 0,82 M Natriumethylat-Lösung in Ethanol gelöst, mit 19,80 ml Benzylbromid versetzt und während 4 Stunden bei Rückfluss gerührt. Nach dem Abkühlen auf Raumtemperatur wurde extrahierend mit Eiswasser, verdünnter Salzsäure und Methylenchlorid aufgearbeitet. Das dabei erhaltene Rohprodukt wurde an Kieselgel mit Methylenchlorid und Aether chromatographiert. So erhielt man 4-(4-Benzyloxy-benzyl)-phenol, MS: *m*/*z* 290,0 ($[M]^+$).

B. 2,98 g 1,2:5,6-Dianhydro-3,4-O-isopropyliden-D-mannitol und 9,30 g 4-(4-Benzyloxy-benzyl)-phenol wurde in Analogie zu Beispiel 33.A. während 17 Stunden bei 100 °C gerührt und analog aufgearbeitet. Das Rohprodukt wurde an Kieselgel mit n-Hexan und Essigester chromatographiert. Das reine Produkt wurde aus Essigester und n-Hexan umkristallisiert. Dabei erhielt man 1,6-Bis-O-[4-(4-Benzyloxy-benzyl)-phenyl]-3,4-O-isopropyliden-D-mannitol, MS: *m*/*z* 784,4 ($[M+NH_4]^+$).

C. 6,07 g 1,6-Bis-O-[4-(4-Benzyloxy-benzyl)-phenyl]-3,4-O-isopropyliden-D-mannitol wurden unter Zusatz von 2,0 g Palladium auf Kohle (5%) in 250 ml Essigester bei Normaldruck und Raumtemperatur hydriert. Nach dem Abfiltrieren des Katalysators wurde das Lösungsmittel im Wasserstrahlvakuum abdestilliert, und der Rückstand wurde an Kieselgel mit Methylenchlorid und Methanol chromatographiert. Man erhielt 1,6-Bis-O-[4-(4-hydroxy-benzyl)-phenyl]-3,4-O-isopropyliden-D-mannitol, MS: *m*/*z* 604,4 ($[M+NH_4]^+$).

D. 1,20 g 1,6-Bis-O-[4-(4-hydroxy-benzyl)-phenyl]-3,4-O-isopropyliden-D-mannitol und 1,58 g 2,3:4,5-Di-O-isopropyliden-1-O-(4-methyl-phenyl-sulfonyl)-D-arabinitol (Beispiel 36.A.) wurden analog Beispiel 33.A. während 21 Stunden bei 100 °C gerührt und analog aufgearbeitet. Das Rohprodukt wurde an Kieselgel mit Methylenchlorid und Methanol chromatographiert. Dabei erhielt man 1,6-Bis-O-[4-[4-(2,3:4,5-di-O-isopropyliden-D-arabinit-1-yloxy)-benzyl]-phenyl]-3,4-O-isopropyliden-D-mannitol, MS: *m*/*z* 1032,5 ($[M+NH_4]^+$).

E. 1,30 g 1,6-Bis-O-[4-[4-(2,3:4,5-di-O-isopropyliden-D-arabinit-1-yloxy)-benzyl]-phenyl]-3,4-O-isopropyliden-D-mannitol wurde analog Beispiel 35.E. während 3 Stunden bei Rückfluss gerührt. Nach dem Abkühlen auf Raumtemperatur wurden die ausgefallenen Kristalle abgenutscht und im Hochvakuum bei 50 °C über Phosphorpentoxid getrocknet. So erhielt man 1,6-Bis-O-[4-(4-D-arabinit-1-yloxy-benzyl)-phenyl]-D-mannitol, MS: *m*/*z* 832,4 ($[M+NH_4]^+$).

F. 0,81 g 1,6-Bis-O-[4-(4-D-arabinit-1-yloxy-benzyl)-phenyl]-D-mannitol wurde in Analogie zu Beispiel 1.B. mit Schwefeltrioxid-trimethylamin-komplex umgesetzt. Dabei wurde 2,3,4,5-Tetra-O-sulfo-1,6-bis-O-[4-[4-(2,3,4,5-tetra-O-sulfo-D-arabinit-1-yloxy)-benzyl]-phenyl]-D-mannitol Dodekanatriumsalz erhalten, MS: *m*/*z* 2039,0 (rekonstruiertes M).

Beispiel 44

A. 3,65 g 4-Hydroxy-benzoesäuremethylester und 7,73 g 2,3:4,5-Di-O-isopropyliden-1-O-(4-methyl-phenylsulfonyl)-D-arabinitol (Beispiel 36.A.) wurden analog Beispiel 33.A. unter Zusatz von 13,82 g Kaliumkarbonat in 300 ml Dimethylformamid während 3 Stunden bei 100 °C umgesetzt und aufgearbeitet. Das Rohprodukt wurde an Kieselgel mit Methylenchlorid und Methanol chromatographiert. Man erhielt so 2,3:4,5-Di-O-isopropyliden-1-O-(4-methoxycarbonyl-phenyl)-D-arabinitol, MS: *m*/*z* 366,0 ($[M]^+$).

B. 7,37 g 2,3:4,5-Di-O-isopropyliden-1-O-(4-methoxycarbonyl-phenyl)-D-arabinitol wurde mit 20 ml Natronlauge (28%ig) in 300 ml Methanol während 16 Stunden bei 50 °C gerührt. Nach Neutralisation mit 6N Salzsäure wurde das Methanol im Wasserstrahlvakuum abdestilliert. Der Rückstand wurde auf Eiswasser gegossen, mit Methylenchlorid extrahiert, die vereinigten Methylenchloridphasen wurden mit Wasser gewaschen, über Magnesiumsulfat getrocknet, filtriert und eingedampft. Das Rohprodukt wurde an Kieselgel mit Methylenchlorid und Methanol chromatographiert. Man erhielt dabei 1-O-(4-Carboxy-phenyl)-2,3:4,5-di-O-isopropyliden-D-arabinitol, MS: *m/z* 352,0 ($[M]^+$).

C. 17,60 g 2,3:4,5-Di-O-isopropyliden-1,6-bis-O-(4-methyl-phenylsulfonyl)-galactitol und 16,00 g Natriumazid wurden in 300 ml Dimethylformamid während 1 Stunde bei 100 °C gerührt. Das Reaktionsgemisch wurde anschliessend extrahierend mit Eiswasser und Essigester aufgearbeitet. Das so erhaltene Rohprodukt wurde aus Methylenchlorid und n-Hexan umkristallisiert. Dabei erhielt man 1,6-Diazido-1,6-didesoxy-2,3:4,5-di-0-isopropyliden-galactitol, MS: *m/z* 297,0 ($[M-CH_3]^+$).

D. 9,20 g 1,6-Diazido-1,6-didesoxy-2,3:4,5-di-O-isopropyliden-galactitol wurde unter Zusatz von 2,50 g Palladiumkohle (10%) in 250 ml Methanol bei Raumtemperatur und Normaldruck hydriert. Nach dem Abfiltrieren des Katalysators wurde das Lösungsmittel im Wasserstrahlvakuum abdestilliert. Der Rückstand wurde in Ethanol aufgenommen. Zwei Equivalente Salzsäure (in Ethanol) wurden zugegeben, es wurde nochmals eingeengt, und das Produkt wurde aus Methanol und Ether umkristallisiert. So erhielt man 1,6-Diamino-1,6-didesoxy-2,3:4,5-di-O-isopropyliden-galactitol Di-hydrochlorid, MS: *m/z* 245,0 ($[M-CH_3]^+$).

E. 1,41 g 1-O-(4-Carboxy-phenyl)-2,3:4,5-di-O-isopropyliden-D-arabinitol und 0,70 g 2-Chloro-4,6-dimethoxy-1,3,5-triazin wurde unter Zusatz von 0,88 ml 4-Methyl-morpholin in 40 ml Acetonitril/ Dimethylformamid (3:1) während 2 Stunden bei 0-5 °C gerührt. Anschliessend wurde 0,67 g 1,6-Diamino-1,6-didesoxy-2,3:4,5-di-O-isopropyliden-galactitol Di-hydrochlorid zugesetzt und weitere 18 Stunden bei Raumtemperatur gerührt. Das Reaktionsgemisch wurde extrahierend mit Eiswasser und Methylenchlorid aufgearbeitet. Das Rohprodukt wurde an Kieselgel mit Methylenchlorid und Methanol chromatographiert. Durch Umkristallisation aus Methylenchlorid und n-Hexan erhielt man 1,6-Didesoxy-2,3:4,5-di-O-isopropyliden-1,6-bis-[4-(2,3:4,5-di-O-isopropyliden-D-arabinit-1-yloxy)-benzoylamino]-galactitol, MS: *m/z* 929,3 ($[M+H]^+$).

F. 1,32 g 1,6-Didesoxy-2,3:4,5-di-O-isopropyliden-1,6-bis-[4-(2,3:4,5-di-O-isopropyliden-D-arabinit-1-yloxy)-benzoylamino]-galactitol wurde in 21 ml Eisessig/Wasser 2:1 während 4 Stunden bei 100 °C gerührt. Nach dem Abkühlen auf Raumtemperatur wurde 30 ml Wasser zugegeben und filtriert. Das Produkt wurde zweimal mit je 5 ml Wasser gewaschen, dann während 3 Stunden bei 50 °C über Phosphorpentoxid im Hochvakuum getrocknet. So erhielt man 1,6-Bis-(4-D-arabinit-1-yloxy-benzoylamino)-1,6-didesoxy-galactitol, MS: *m/z* 711,6 ($[M+Na]^+$).

G. 0,90 g 1,6-Bis-(4-D-arabinit-1-yloxy-benzoylamino)-1,6-didesoxy-galactitol wurde in Analogie zu Beispiel 1.B. mit Schwefeltrioxid-trimethylamin-komplex umgesetzt. Dabei wurde 1,6-Didesoxy-2,3,4,5-tetra-O-sulfo-1,6-bis-[4-(2,3,4,5-tetra-O-sulfo-D-arabinit-1-yloxy)-benzoylamino)-galactitol Dodekanatriumsalz erhalten, MS: *m/z* 1912,0 (rekonstruiertes M).

Beispiel 45

A. 4,85 g 3-Hydroxy-2-naphthalin-2-carbonsäuremethylester und 7,73 g 2,3:4,5-Di-O-isopropyliden-1-O-(4-methyl-phenylsulfonyl)-D-arabinitol (Beispiel 36.A.) wurde analog Beispiel 33.A. unter Zusatz von 13,82 g Kaliumkarbonat in 300 ml Dimethylformamid während 4 Stunden bei 100 °C umgesetzt und aufgearbeitet. Das Rohprodukt wurde an Kieselgel mit Methylenchlorid und Methanol chromatographiert. Man erhielt dabei 2,3:4,5-Di-O-isopropyliden-1-O-(3-methoxycarbonyl-naphthalin-2-yl)-D-arabinitol, MS: *m/z* 416,0 ($[M]^+$).

B. 6,00 g 2,3:4,5-Di-O-isopropyliden-1-O-(3-methoxycarbonyl-naphthalin-2-yl)-D-arabinitol wurde mit 14,4 ml Natronlauge (28%) in 250 ml Methanol während 16 Stunden bei Raumtemperatur gerührt. Nach Neutralisation mit 6N Salzsäure wurde das Methanol im Wasserstrahlvakuum abdestilliert. Der Rückstand wurde auf Eiswasser gegossen, mit Methylenchlorid extrahiert, die vereinigten Methylenchloridphasen wurden mit Wasser gewaschen, über Magnesiumsulfat getrocknet, filtriert und eingedampft. Das Rohprodukt wurde an Kieselgel mit Methylenchlorid und Methanol chromatographiert. Man erhielt so 1-O-(3-Carboxy-naphthalin-2-yl)-2,3:4,5-di-O-isopropyliden-D-arabinitol, MS: *m/z* 402,0 ($[M]^+$).

C. 1,61 g 1-O-(3-Carboxy-naphthalin-2-yl)-2,3:4,5-di-O-isopropyliden-D-arabinitol und 0,70 g 2-Chloro-4,6-dimethoxy-1,3,5-triazin wurden mit 0,67 g 1,6-Diamino-1,6-didesoxy-2,3:4,5-di-O-isopropyliden-galactitol Dihydrochlorid unter Zusatz von 0,88 ml 4-Methyl-morpholin analog Beispiel 44.E. umgesetzt und aufgearbeitet. Das Rohprodukt wurde an Kieselgel mit Methylenchlorid und Methanol chromatographiert. Man erhielt so 1,6-Didesoxy-2,3:4,5-di-O-isopropyliden-1,6-bis-[3-(2,3:4,5-di-O-isopropyliden-D-arabinit-1-yloxy)-naphthalin-2-ylcarbonylamino]-galactitol, MS: *m/z* 1028,8 ($[M]^+$).

D. 1,95 g 1,6-Didesoxy-2,3:4,5-di-O-isopropyliden-1,6-bis-[3-(2,3:4,5-di-O-isopropyliden-D-arabinit-1-yloxy)-naphthalin-2-ylcarbonylamino]-galactitol wurde analog Beispiel 44.F. umgesetzt und aufgearbeitet. Man erhielt 1,6-Bis-(3-D-arabinit-1-yloxy-naphthalin-2-ylcarbonylamino)-1,6-didesoxy-galactitol, MS: *m/z* 788,8 ($[M]^+$).

E. 1,11 g 1,6-Bis-(3-D-arabinit-1-yloxy-naphthalin-2-ylcarbonylamino)-1,6-didesoxy-galactitol wurde in Analogie zu Beispiel 1.B. mit Schwefeltrioxidtrimethylamin-komplex umgesetzt. Dabei wurde 1,6-Didesoxy-2,3,4,5-tetra-O-sulfo-1,6-bis-[3-(2,3,4,5-tetra-O-sulfo-D-arabinit-1-yloxy)-naphthalin-2-ylcarbonylamino]-galactitol Dodekanatriumsalz erhalten, MS: *m/z* 2013,0 (rekonstruiertes M).

Beispiel 46

A. 27,75 g 3,4-Di-O-isopropyliden-D-mannitol und 65,25 g Dibutylzinnoxid wurden in 2,5 l Benzol unter Verwendung eines Wasserabscheiders während 2 Stunden bei Rückfluss gerührt. Hierauf wurde im Wasserstrahlvakuum auf ein Gesamtvolumen von 700 ml eingeengt. Anschliessend wurde bei Raumtemperatur 46,25 g Tetrabutylammoniumjodid zugegeben, 37,50 ml 3-Brom-propin-1-in zugetropft, und das Reaktionsgemisch wurde während 7 Stunden bei 70 °C gerührt. Nach Einengen im Wasserstrahlvakuum wurde das Rohprodukt an Kieselgel mit n-Hexan und Essigester chromatographiert. So erhielt man 1,6-Di-O-prop-2-inyl-3,4-O-isopropyliden-D-mannitol, MS: *m/z* 298,0 ($[M]^+$).

B. 21,20 g 1,6-Di-O-prop-2-inyl-3,4-O-isopropyliden-D-mannitol wurde in 300 ml Eisessig/Wasser 2:1 während 3 Stunden bei 100 °C gerührt. Das Reaktionsgemisch wurde im Wasserstrahlvakuum eingeengt. Mit zweimal je 50 ml Toluol wurde das Wasser azeotrop abdestilliert. Der Rückstand wurde während 18 Stunden im Hochvakuum über Phosphorpentoxid getrocknet. Man erhielt dabei 1,6-Bis-O-prop-2-inyl-D-mannitol, welches direkt in die nächste Stufe eingesetzt wurde.

C. 27,00 g 1,6-Bis-O-prop-2-inyl-D-mannitol wurde in 300 ml Pyridin gelöst, bei Raumtemperatur unter Argon mit 100 ml Acetanhydrid versetzt und 4 Stunden gerührt. Anschliessend wurde extrahierend mit Wasser und Essigester aufgearbeitet. Das so erhaltene Rohprodukt wurde an Kieselgel mit Methylenchlorid und Methanol chromatographiert. Dabei erhielt man 2,3,4,5-Tetra-O-acetyl-1,6-bis-O-prop-2-inyl-D-mannitol, MS: *m/z* 367,0 ($[M\text{-}OAc]^+$).

D. 9,80 g 4-Iod-phenol und 15,80 g 2,3:4,5-Di-O-isopropyliden-1-O-(4-methyl-phenylsulfonyl)-D-arabinitol (Beispiel 36.A.) wurden unter Zusatz von 56,00 g Kaliumkarbonat in 500 ml Dimethylformamid während 6 Stunden bei 100 °C umgesetzt und analog Beispiel 33.A. aufgearbeitet. Das Rohprodukt wurde an Kieselgel mit Hexan und Essigester chromatographiert. So erhielt man 1-O-(4-Iodphenyl)-2,3:4,5-di-O-isopropyliden-D-arabinitol, MS: *m/z* 434,0 ($[M]^+$).

E. 14,90 g 1-O-(4-Iod-phenyl)-2,3:4,5-di-O-isopropyliden-D-arabinitol wurde analog Beispiel 46.B. umgesetzt und aufgearbeitet. Das Rohprodukt wurde in Ether aufgeschlämmt, filtriert und im Hochvakuum bei 50 °C während 16 Stunden über Phosphorpentoxid getrocknet. Das so erhaltene 1-O-(4-Iod-phenyl)-D-arabinitol wurde direkt in die nächste Stufe eingesetzt.

F. 12,85 g 1-O-(4-Iod-phenyl)-D-arabinitol wurde analog Beispiel 46.C. umgesetzt und aufgearbeitet. Das Rohprodukt wurde aus Essigester und Hexan umkristallisiert. So wurde 2,3,4,5-Tetra-O-acetyl-1-O-(4-iod-phenyl)-D-arabinitol erhalten, MS: *m/z* 522,0 ($[M]^+$).

G. 2,61 g 2,3,4,5-Tetra-O-acetyl-1-O-(4-iod-phenyl)-D-arabinitol, 0,58 g Palladium(0)-tetrakis-triphenylphosphin und 0,19 g Kupfer(I)-iodid wurden in 25 ml Diisopropylamin/Dimethylformamid (1:1) während 45 Minuten bei Raumtemperatur unter Argon gerührt. Anschliessend wurde eine Lösung von 1,07 g 2,3,4,5-Tetra-O-acetyl-1,6-bis-O-prop-2-inyl-D-mannitol in 10 ml Diisopropyl-amin/Dimethylformamid (1:1) zugetropft, und das Reaktionsgemisch wurde während 70 Stunden bei Raumtemperatur gerührt. Der Ansatz wurde hierauf unter Zusatz von 100 ml Toluol eingeengt, dann wurde das Dimethylformamid im Hochvakuum abdestilliert. Das so erhaltene Rohprodukt wurde an Kieselgel mit Methylenchlorid und Methanol chromatographiert. Dabei erhielt man 2,3,4,5-Tetra-O-acetyl-1,6-bis-O-[3-[4-(2,3,4,5-tetra-O-acetyl-D-arabinit-1-yloxy)-phenyl]-prop-2-inyl]-D-mannitol, welches direkt in die nächste Stufe eingesetzt wurde.

H. 3,42 g 2,3,4,5-Tetra-O-acetyl-1,6-bis-O-[3-[4-(2,3,4,5-tetra-O-acetyl-D-arabinit-1-yloxy)-phenyl]-prop-2-inyl]-D-mannitol wurde in 100 ml Methanol gelöst, mit 10 ml Natriummethylatlösung (5,4 molar) versetzt und während 16 Stunden bei Raumtemperatur gerührt. Die Reaktionslösung wurde mit wässriger Salzsäure auf pH 2-3 gestellt und eingeengt. Der Rückstand wurde in Wasser aufgeschlämmt, filtriert, zweimal mit je 10 ml Wasser gewaschen und 5 Stunden im Hochvakuum über Phosphorpentoxid getrocknet. Man erhielt dabei 1,6-Bis-O-[3-(4-D-arabinit-1-yloxy-phenyl)-prop-2-inyl]-D-mannitol, MS: *m/z* 733,6 ($[M+Na]^+$).

I. 1,70 g 1,6-Bis-O-[3-(4-D-arabinit-1-yloxy-phenyl)-prop-2-inyl]-D-mannitol wurde in Analogie zu Beispiel 1.B. mit Schwefeltrioxid-trimethylamin-komplex umgesetzt. Dabei wurde 2,3,4,5-Tetra-O-sulfo-1,6-bis-O-[3-[4-(2,3,4,5-tetra-O-sulfo-D-arabinit-1-yloxy)-phenyl]-prop-2-inyl]-D-mannitol Dodekanatriumsalz erhalten, MS: *m/z* 1934,0 (rekonstruiertes M).

Beispiel 47

A. 14,10 g 2,3-O-Isopropyliden-1,4-bis-O-(4-methyl-phenylsulfonyl)-L-threitol und 6,00 g 4-Benzyloxy-phenol wurden in 300 ml Dimethylformamid gelöst, mit 20,70 g Kaliumkarbonat versetzt und während 6 Stunden bei 100 °C gerührt. Das Reaktionsgemisch wurde extrahierend mit Eiswasser und Methylenchlorid aufgearbeitet. Das Rohprodukt wurde anschliessend an Kieselgel mit n-Hexan und Methylenchlorid chromatographiert. Man erhielt so 1-O-(4-Benzyloxy-phenyl)-2,3-O-isopropyliden-4-O-(4-methyl-phenylsulfonyl)-L-threitol, MS: *m/z* 498,0 ($[M]^+$).

B. 5,81 g 4-(4-Benzyloxy-benzyl)-phenol (Beispiel 43.A.) und 7,73 g 2,3:4,5-Di-O-isopropyliden-1-O-(4-methyl-phenylsulfonyl)-D-arabinitol (Beispiel 36.A.) wurden analog Beispiel 47.A. in Dimethylformamid bei 100 °C umgesetzt und analog aufgearbeitet. Das Rohprodukt wurde an Kieselgel mit n-Hexan und Methylenchlorid chromatographiert. So wurde 1-O-[4-(4-Benzyloxy-benzyl)-phenyl]-2,3:4,5-di-O-isopropyliden-D-arabinitol erhalten, MS: *m/z* 504,0 ($(M]^+$).

C. 8,30 g 1-O-[4-(4-Benzyloxy-benzyl)-phenyl]-2,3:4,5-di-O-isopropyliden-D-arabinitol wurde in 85 ml Tetrahydrofuran unter Zusatz von 1,60 g Palladium auf Kohle (10%) bei Normaldruck und Raumtemperatur hydriert. Nach dem Abfiltrieren des Katalysators wurde das Filtrat eingeengt. Man erhielt dabei 1-O-[4-(4-Hydroxy-benzyl)-phenyl]-2,3:4,5-di-O-isopropyliden-D-arabinitol, MS: *m/z* 414,0 ($[M]^+$).

D. 1,66 g 1-O-[4-(4-Hydroxy-benzyl)-phenyl]-2,3:4,5-di-O-isopropyliden-D-arabinitol und 1,99 g 1-O-(4-Benzyloxy-phenyl)-2,3-O-isopropyliden-4-O-(4-methyl-phenylsulfonyl)-L-threitol wurden analog Beispiel 47.A. in Dimethylformamid bei 100 °C umgesetzt und analog aufgearbeitet. Das Rohprodukt wurde an Kieselgel mit n-Hexan und Methylenchlorid chromatographiert. So erhielt man 1-O-(4-Benzyloxy-phenyl)-4-O-[4-[4-(2,3:4,5-di-O-isopropyliden-D-arabinit-1-yloxy)-benzyl]-phenyl]-L-threitol, MS: *m/z* 740,0 ($[M]^+$).

E. 2,50 g 1-O-(4-Benzyloxy-phenyl)-4-O-[4-[4-(2,3:4,5-di-O-isopropyliden-D-arabinit-1-yloxy)-benzyl]-phenyl]-L-threitol wurde analog Beispiel 47.C. hydriert und aufgearbeitet. So wurde 1-O-(4-Hydroxy-phenyl)-4-O-[4-[4-(2,3:4,5-di-O-isopropyliden-D-arabinit-1-yloxy)-benzyl]-phenyl]-L-threitol erhalten, MS: *m/z* 650,0 ($[M]^+$).

F. 1,80 g 1-O-(4-Hydroxy-phenyl)-4-O-[4-[4-(2,3:4,5-di-O-isopropyliden-D-arabinit-1-yloxy)-benzyl]-phenyl]-L-threitol und 0,79 g 2,3:4,5-Di-O-isopropyliden-1,6-bis-O-(4-methyl-phenylsulfonyl)-galactitol wurden in Analogie zu Beispiel 47.A. in 20 ml Dimethylformamid während 3 Stunden bei Rückfluss umgesetzt und analog aufgearbeitet. Das Rohprodukt wurde an Kieselgel mit Toluol und Essigester chromatographiert. Man erhielt 1,6-Bis-O-[4-[4-O-[4-[4-(2,3:4,5-di-O-isopropyliden-D-arabinit-1-yloxy)-benzyl]-phenyl]-2,3-di-O-isopropyliden-L-threit-1-yloxy]-phenyl]-2,3:4,5-di-O-isopropyliden-galactitol, welches direkt in die nächste Stufe eingesetzt wurde.

G. 0,35 g 1,6-Bis-O-[4-[4-O-[4-[4-(2,3:4,5-di-O-isopropyliden-D-arabinit-1-yloxy)-benzyl]-phenyl]-2,3-di-O-isopropyliden-L-threit-1-yloxy]-phenyl]-2,3:4,5-di-O-isopropyliden-galactitol wurde in Analogie zu Beispiel 35.E. während 3 Stunden bei Rückfluss gerührt und analog aufgearbeitet. So erhielt man 1,6-Bis-O-[4-[4-O-[4-[4-(D-arabinit-1-yloxy)-benzyl]-phenyl]-L-threit-1-yloxy]-phenyl]-galactitol, welches direkt in die nächste Stufe einesetzt wurde.

H. 0,27 g 1,6-Bis-O-[4-[4-O-[4-[4-(D-arabinit-1-yloxy)-benzyl]-phenyl]-L-threit-1-yloxy]-phenyl]-galactitol wurde in Analogie zu Beispiel 1.B. mit Schwefeltrioxid-trimethylamin-komplex umgesetzt. Dabei wurde 2,3,4,5-Tetra-O-sulfo-1,6-bis-O-[4-[4-O-[4-[4-(2,3,4,5-tetra-O-sulfo-D-arabinit-1-yloxy)-benzyl]-phenyl]-2,3-di-O-sulfo-L-threit-1-yloxy]-phenyl]-galactitol Hexadekanatriumsalz erhalten, MS: *m/z* 2840,0 (rekonstruiertes M).

<u>Beispiel 48</u>

A. 5,0 g 2,3:4,5-Di-O-isopropyliden-1,6-bis-O-(4-methyl-phenylsulfonyl)-galactitol, 5,26 g 4-Benzyloxy-phenol und 3,6 g fein gemahlenes wasserfreies Kaliumkarbonat wurden in 60 ml Dimethylformamid suspendiert und während 18 Stunden bei 130 °C unter Argon gerührt. Anschliessend wurde das Reaktionsgemisch mit Wasser verdünnt, und die so gebildeten Kristalle wurden abfiltriert. So wurde 1,6-Bis-O-(4-benzyloxy-phenyl)-2,3:4,5-di-O-isopropyliden-galactitol in Form farbloser Kristalle erhalten, MS: *m/z* 625 ($[M-H]^+$).

B. 4,0 g 1,6-Bis-O-(4-benzyloxy-phenyl)-2,3:4,5-di-O-isopropyliden-galactitol wurde in 150 ml Tetrahydrofuran unter Zusatz von 0,5 g Palladium auf Kohle (10%) in einer Wasserstoffatmosphäre erschöpfend hydriert. Anschliessend wurde das Reaktionsgemisch über einen 0,8 μ Cellulosefilter filtriert. Das Filtrat wurde eingeengt und der Rückstand aus Ether kristallisiert; dabei wurde 1,6-Bis-O-(4-hydroxy-phenyl)-2,3:4,5-di-O-isopropyliden-galactitol als farbloser Festkörper erhalten, MS: *m/z* 446 ($[M]^+$).

C. 1,78 g 1,6-Bis-O-(4-hydroxy-phenyl)-2,3:4,5-di-O-isopropyliden-galactitol, 3,2 g 1-O-Benzyl-2,3-O-isopropyliden-4-O-(4-methyl-phenylsulfonyl)-D-threitol (E. Hungerbühler & D. Seebach, Helvetica Chimica Acta 64, 687 (1981)) und 1,2 g fein gemahlenes wasserfreies Kaliumkarbonat wurden in 25 ml Dimethylformamid suspendiert und während 24 Stunden bei 130 °C unter Argon gerührt. Anschliessend wurde das Reaktionsgemisch mit Wasser verdünnt und die Wasserphase wurde mit Ether extrahiert. Die vereinigten Etherphasen wurden über Magnesiumsulfat getrocknet und eingeengt. Das so erhaltene Rohprodukt wurde an Kieselgel mit 5% Ether in Methylenchlorid chromatographiert. Dabei wurde 1,6-Bis-O-[4-(4-O-benzyl-2,3-O-isopropyliden-D-threit-1-yloxy)-phenyl]-2,3:4,5-di-O-isopropyliden-galactitol in Form farbloser Kristalle erhalten, MS: *m/z* 932,6 ($[M+NH_4]^+$).

D. 2,3 g 1,6-Bis-O-[4-(4-O-benzyl-2,3-O-isopropyliden-D-threit-1-yloxy)-phenyl]-2,3:4,5-di-O-isopropyliden-galactitol wurde in 20 ml Tetrahydrofuran unter Zusatz von 0,2 g Palladium auf Kohle (10%) in einer Wasserstoffatmosphäre erschöpfend hydriert. Anschliessend wurde das Reaktionsgemisch über einen 0,8 μ Cellulosefilter filtriert und eingeengt; so wurde 2,3:4,5-Di-O-isopropyliden-1,6-bis-O-[4-(2,3-O-isopropyliden-D-threit-1-yloxy)-phenyl]-galactitol als farbloses Oel erhalten, MS: *m/z* 734 ($[M]^+$).

E. 1,74 g 2,3:4,5-Di-O-isopropyliden-1,6-bis-O-[4-(2,3-O-isopropyliden-D-threit-1-yloxy)-phenyl]-galactitol, gelöst in 15 ml Pyridin, wurden bei 0 °C mit einer Lösung von 1,35 g p-Toluolsulfonylchlorid in 5 ml Pyridin versetzt und während 60 Std. bei Raumtemperatur gerührt. Hierauf wurde das Reaktionsgemisch mit Eiswasser versetzt, 1,5 Stunden bei Raumtemperatur gerührt und mit Ether extrahiert. Die vereinigten Etherphasen wurden mit IN Salzsäure und Wasser gewaschen, über Magnesiumsulfat getrocknet und eingeengt. Das so erhaltene Rohprodukt wurde an Kieselgel mit 5% Ether in Methylenchlorid chromatographiert. Dabei wurde 2,3:4,5-Di-O-isopropyliden-1,6-bis-O-[4-[2,3-O-isopropyliden-4-O-(4-methyl-phenyl-sulfonyl)-D-threit-1-yloxy]-phenyl]-galactitol als farbloses Oel erhalten, MS: *m*/*z* 870 ($[M\text{-}TosOH]^+$).

F. 2,21 g 2,3:4,5-Di-O-isopropyliden-1,6-bis-O-[4-[2,3-O-isopropyliden-4-O-(4-methyl-phenyl-sulfonyl)-D-threit-1-yloxy]-phenyl]-galactitol, 1,13 g (E)-3-(4-Hydroxy-phenyl)-acrylsäuremethylester und 0,87 g fein gemahlenes wasserfreies Kaliumkarbonat wurden in 10 ml Dimethylformamid suspendiert und während 20 Stunden bei 130 °C unter Argon gerührt. Anschliessend wurde das Reaktionsgemisch eingeengt, mit Wasser verdünnt und mit Methylenchlorid extrahiert. Die vereinigten Methylenchloridphasen wurden mit 1N Natriumhydroxidlösung und Wasser gewaschen, über Magnesiumsulfat getrocknet und eingeengt. Das so erhaltene Rohprodukt 2,3:4,5-Di-O-isopropyliden-1,6-bis-O-[4-[2,3-O-isopropyliden-4-O-[4-[(E)-2-methoxycarbonyl-vinyl]-phenyl]-D-threit-1-yloxy]-phenyl]-galactitol wurde hierauf in 30 ml Methanol gelöst, mit 3 ml konzentrierter Natriumhydroxidlösung und 3 ml Wasser versetzt und 3 Stunden unter Rückfluss erwärmt. Hierauf wurde unter Eiskühlung mit 1 N Salzsäure angesäuert, und der so erhaltene Niederschlag wurde abfiltriert. Dabei resultierte 1,6-Bis-O-[4-[4-O-[4-[(E)-2-carboxy-vinyl]-phenyl]-2,3-O-isopropyliden-D-threit-1-yloxy]-phenyl]-2,3:4,5-di-O-isopropyliden-galactitol in Form farbloser Kristalle, MS: *m*/*z* 1025,4 ($[M\text{-}H]^-$).

G. 1,02 g 1,6-Bis-O-[4-[4-O-[4-[(E)-2-carboxy-vinyl]-phenyl]-2,3-0-isopropyliden-D-threit-1-yloxy]-phenyl]-2,3:4,5-di-O-isopropyliden-galactitol, gelöst in 5 ml Dimethylformamid wurde unter Feuchtigkeitsausschluss bei 0 - 5 °C mit 0,3 ml 4-Methylmorpholin versetzt und während 10 Minuten intensiv gerührt. Hierauf wurde 0,40 g 2-Chlor-4,6-dimethoxy-1,3,5-triazin fest hinzugefügt und weitere 2 Stunden bei 0 - 5 °C gerührt. Anschliessend wurde 0,40 g D-Glucamin zugesetzt und weitere 18 Stunden bei Raumtemperatur gerührt. Hierauf wurde das Reaktionsgemisch mit Wasser verdünnt, eingeengt, erneut mit Wasser versetzt, kurz aufgekocht und filtriert. Dabei resultierte 1,6-Bis-O-[4-[4-O-[4-[(E)-2-D-glucit-1-ylcarbamoyl-vinyl]-phenyl]-2,3-O-isopropyliden-D-threit-1-yloxy]-phenyl]-2,3:4,5-di-O-isopropyliden-galactitol als bräunlicher Festkörper, MS: *m*/*z* 1353,6 ($[M+H]^+$).

H. 0,20 g 1,6-Bis-O-[4-[4-O-[4-[(E)-2-D-glucit-1-ylcarbamoyl-vinyl]-phenyl]-2,3-O-isopropyliden-D-threit-1-yloxy]-phenyl]-2,3:4,5-di-O-isopropyliden-galactitol wurde in 3 ml Dioxan, 0,5 ml Trifluoressigsäure und 0,7 ml Wasser suspendiert und 18 Stunden bei 95 °C unter Rückfluss erwärmt. Anschliessend wurde das Reaktionsgemisch zur Trockene eingeengt; dabei resultierte 1,6-Bis-O-[4-[4-O-[4-[(E)-2-D-glucit-1-ylcarbamoyl-vinyl]-phenyl]-D-threit-1-yloxy]-phenyl]-galactitol als farbloser Festkörper, MS: *m*/*z* 1215,9 ($[M+Na]^+$).

I. Das oben erhaltene 1,6-Bis-O-[4-[4-O-[4-[(E)-2-D-glucit-1-ylcarbamoyl-vinyl]-phenyl]-D-threit-1-yloxy]-phenyl]-galactitol wurde analog Beispiel 1.B. zu 1,6-Bis-O-[4-[4-O-[4-[(E)-2-(2,3,4,5,6-penta-O-sulfo-D-glucit-1-ylcarbamoyl)-vinyl]-phenyl]-2,3-di-O-sulfo-D-threit-1-yloxy]-phenyl]-2,3,4,5-tetra-O-sulfo-galactitol Octadekanatriumsalz, $[a]^{20}_{D}$ -3,6° (*c* 0,7; Wasser) umgesetzt.

Beispiel 49

A. 0,15 g 1,6-Bis-O-[4-[4-O-[4-[(E)-2-(2,3,4,5,6-penta-O-sulfo-D-glucit-1-ylcarbamoyl)-vinyl]-phenyl]-2,3-di-O-sulfo-D-threit-1-yloxy]-phenyl]-2,3,4,5-tetra-O-sulfo-galactitol Octadekanatriumsalz wurde in 5 ml destilliertem Wasser unter Zusatz von 100 mg Palladium auf Kohle (10%) in einer Wasserstoffatmosphäre erschöpfend hydriert. Anschliessend wurde das Reaktionsgemisch über einen 0,8 $\mu$ Cellulosefilter filtriert und das Filtrat lyophilisiert; dabei wurde 1,6-Bis-O-[4-[4-O-[4-[2-(2,3,4,5,6-penta-O-sulfo-D-glucit-1-ylcarbamoyl)-ethyl]-phenyl]-2,3-di-O-sulfo-D-threit-1-yloxy]-phenyl]-2,3,4,5-tetra-O-sulfo-galactitol Octadekanatriumsalz , $[\alpha]^{20}_{D}$ -3,4° (*c* 0,5; Wasser), erhalten.

Beispiel 50

A. 37,6 g 2,3-O-Isopropyliden-1,4-di-O-(4-methyl-phenylsulfonyl)-L-threitol, 16,0 g 3-Hydroxy-naphthalin-2-carbonsäuremethylester und 13 g fein gemahlenes wasserfreies Kaliumkarbonat wurden in 300 ml Acetonitril suspendiert und während 60 Stunden bei 100 °C unter Argon gerührt. Anschliessend wurde das Reaktionsgemisch mit Wasser verdünnt und die Wasserphase wurde mit Ether extrahiert. Die vereinigten Etherphasen wurden über Magnesiumsulfat getrocknet und eingeengt. Das so erhaltene Rohprodukt wurde an Kieselgel mit Ether in Methylenchlorid chromatographiert. Dabei wurde rohes 2,3-O-Isopropyliden-1-O-(3-methoxycarbonyl-naphthalin-2-yl)-4-O-(4-methyl-phenylsulfonyl)-L-threitol erhalten. Dieses wurde in 100 ml Dimethylformamid gelöst und nach Hinzufügen von 15,6 g 4-Benzyloxy-phenol und 15,6 g fein gemahlenem wasserfreiem Kaliumkarbonat während 18 Stunden bei 130 °C unter Argon gerührt. Anschlies-send wurde das Reaktionsgemisch mit Wasser verdünnt, die Wasserphase wurde mit Ether extrahiert. Die vereinigten Etherphasen wurden über Magnesiumsulfat getrocknet und eingeengt. Das so erhaltene

Rohprodukt wurde an Kieselgel mit Ether in Methylenchlorid chromatographiert. Dabei wurde 1-O-(4-Benzyloxy-phenyl)-2,3-O-isopropyliden-4-O-(3-methoxycarbonyl-naphthalin-2-yl)-L-threitol als farbloses Oel erhalten, MS: *m*/*z* 528 ($[M]^+$).

B. 1,72 g 1-O-(4-Benzyloxy-phenyl)-2,3-O-isopropyliden-4-O-(3-methoxy-carbonyl-naphthalin-2-yl)-L-threitol wurde in 40 ml Tetrahydrofuran unter Zusatz von 0,2 g Palladium auf Kohle (10%) in einer Wasserstoffatmosphäre erschöpfend hydriert. Anschliessend wurde das Reaktionsgemisch über einen 0,8 μ Cellulosefilter filtriert, und das Filtrat wurde eingeengt; dabei wurde 1-O-(4-Hydroxy-phenyl)-2,3-O-isopropyliden-4-O-(3-methoxycarbonyl-naphthalin-2-yl)-L-threitol als farbloses Oel erhalten, MS: *m*/*z* 438 ($[M]^+$).

C. 0,57 g 2,3:4,5-Di-O-isopropyliden-1,6-bis-O-(4-methyl-phenylsulfonyl)-galactitol, 0,95 g 1-O-(4-Hydroxy-phenyl)-2,3-O-isopropyliden-4-O-(3-methoxycarbonyl-naph-thalin-2-yl)-L-threitol und 0,3 g fein gemahlenes wasserfreies Kaliumkarbonat wurden in 10 ml Acetonitril suspendiert und während 100 Stunden bei 130 °C unter Argon gerührt. Anschliessend wurde das Reaktionsgemisch mit Wasser verdünnt, die Wasserphase wurde mit Methylenchlorid extrahiert. Die vereinigten Methylenchloridphasen wurden über Magnesiumsulfat getrocknet und eingeengt. Das so erhaltene Rohprodukt wurde an Kieselgel mit Ether in Methylenchlorid chromatographiert. So wurde 2,3:4,5-Di-O-isopropyliden-1,6-bis-O-[4-[2,3-O-isopropyliden-4-O-(3-methoxycarbonyl-naphthalin-2-yl)-L-threit-1-yloxy]-phenyl]-galactitol als farbloser Festkörper erhalten, MS: *m*/*z* 1120,4 ($[M-NH_4]^+$).

D. 0,58 g 2,3:4,5-Di-O-isopropyliden-1,6-bis-O-[4-[2,3-O-isopropyliden-4-O-(3-methoxycarbonyl-naphthalin-2-yl)-L-threit-1-yloxy]-phenyl]-galactitol wurde in 5 ml 2-Methoxyethanol gelöst, mit 5 ml konzentrierter Natriumhydroxidlösung versetzt und 5 Stunden unter Rückfluss erwärmt. Hierauf wurde unter Eiskühlung mit 1 N Salzsäure angesäuert, mit Methylenchlorid extrahiert, die vereinigten Methylenchloridphasen wurden über Magnesiumsulfat getrocknet und eingeengt. Das so erhaltene Rohprodukt wurde an Kieselgel mit Methanol in Methylenchlorid chromatographiert. So wurde 1,6-Bis-O-[4-[4-O-(3-carboxy-naphthalin-2-yl)-2,3-O-isopropyliden-L-threit-1-yloxy]-phenyl]-2,3:4,5-di-O-isopropyliden-galactitol als farbloser Festkörper erhalten, MS: *m*/*z* 1073,2 ([M-H]).

E. 0,30 g 1,6-Bis-O-[4-[4-O-(3-carboxy-naphthalin-2-yl)-2,3-O-isopropyliden-L-threit-1-yloxy]-phenyl]-2,3:4,5-di-O -isopropyliden-galactitol, gelöst in 3 ml Dimethylformamid wurde unter Feuchtigkeitsausschluss bei 0 - 5 °C mit 0,7 ml 4-Methylmorpholin versetzt und während 10 Minuten intensiv gerührt. Hierauf wurde 0,105 g 2-Chlor-4,6-dimethoxy-1,3,5-triazin fest hinzugefügt und weitere 2 Stunden bei 0 - 5 °C gerührt. Anschliessend wurde 0,109 g D-Glucamin zugesetzt und weitere 18 Stunden bei Raumtemperatur gerührt. Hierauf wurde das Reaktionsgemisch mit Wasser verdünnt, eingeengt, erneut mit Wasser versetzt, kurz aufgekocht und filtriert. Dabei resultierte 1,6-Bis-O-[4-[4-O-(3-D-glucit-1-ylcarbamoyl-naphthalin-2-yl)-2,3-0-isopropyliden-L-threit-1-yloxy]-phenyl]-2,3:4,5-di-O-isopropyliden-galactitol als farbloser Festkörper, MS: *m*/*z* 1401,7 ($[M+H]^+$).

F. 0,34 g 1,6-Bis-O-[4-[4-O-(3-D-glucit-1-ylcarbamoyl-naphthalin-2-yl)-2,3-O-isopropyliden-L-threit-1-yloxy]-phenyl]-2,3:4,5-di-O-isopropyliden-galactitol wurde in 4 ml Dioxan, 0,5 ml Trifluoressigsäure und 0,3 ml Wasser suspendiert und 18 Stunden bei 110 °C unter Rückfluss erwärmt. Anschliessend wurde das Reaktionsgemisch zur Trokkene eingeengt; dabei resultierte 1,6-Bis-O-[4-[4-O-(3-D-glucit-1-ylcarbamoyl-naphthalin-2-yl)-L-threit-1-yloxy]-phenyl]-galactitol als farbloser Festkörper, MS: *m*/*z* 1242,7 ($[M+H]^+$).

G. Das oben erhaltene 1,6-Bis-O-[4-[4-O-(3-D-glucit-1-ylcarbamoyl-naphthalin-2-yl)-L-threit-1-yloxy]-phenyl]-galactitol wurde analog Beispiel 1.B.zu 1,6-Bis-O-[4-[4-O-[3-(2,3,4,5,6-penta-O-sulfo-D-glucit-1-ylcarbamoyl)-naphthalin-2-yl]-2,3-di-O-sulfo-L-threit-1-yloxy]-phenyl]-2,3,4,5-tetra-O-sulfo-galactitol Octadekanatriumsalz, $[\alpha]_D^{20}$ +2,3° (*c* 0,6; Wasser), MS: *m*/*z* 3075,0 (rekonstruiertes M) umgesetzt.

Beispiel 51

A. 5,0 g Bis-(4-hydroxy-phenyl)-methan wurde in 40 ml Ethanol gelöst, 33 ml 1 molare Natriumethanolat-Lösung in Ethanol und anschliessend 3,6 ml Benzylchlorid wurden hinzugefügt, und das Reaktionsgemisch wurde während 4 Stunden unter Feuchtigkeitsausschluss unter Rückfluss erwärmt. Hierauf wurde unter Eiskühlung mit 1 N Salzsäure angesäuert, mit Methylenchlorid extrahiert, die vereinigten Methylenchloridphasen wurden über Magnesiumsulfat getrocknet und eingeengt. Das so erhaltene Rohprodukt wurde an Kieselgel mit Ether in Methylenchlorid chromatographiert. So wurde 4-(4-Benzyloxy-benzyl)-phenol in Form farbloser Kristalle erhalten, MS: *m*/*z* 290 ($[M]^+$).

B. 6,0 g 2,3-O-Isopropyliden-1,4-di-O-(4-methyl-phenylsulfonyl)-L-threitol, 2,13 g 4-Hydroxybenzoesäuremethylester und 1,94 g fein gemahlenes wasserfreies Kaliumkarbonat wurden in 100 ml Acetonitril suspendiert und während 18 Stunden bei 100 °C unter Argon gerührt. Anschliessend wurde das Reaktionsgemisch mit Wasser verdünnt, mit Methylenchlorid extrahiert, die vereinigten Methylenchloridphasen wurden über Magnesiumsulfat getrocknet und eingeengt. Der Rückstand wurde mit 5% Ether in Methylenchlorid an Kieselgel chromatographert. So wurde 2,3-O-Isopropyliden-1-O-(4-methoxycarbonyl-phenyl)-4-O-(4-methyl-phenylsulfonyl)-L-threitol als farbloses Oel erhalten, MS: *m*/*z* 450 ($[M]^+$).

C. 2,54 g 2,3-O-Isopropyliden-1-O-(4-methoxycarbonyl-phenyl)-4-O-(4-methyl-phenylsulfonyl)-L-threitol, 1,80 g 4-(4-Benzyloxy-benzyl)-phenol und 0,86 g fein gemahlenes wasserfreies Kaliumkarbonat wurden in 10 ml Dimethylformamid suspendiert und während 18 Stunden bei 130 °C unter Argon gerührt. Anschliessend wurde das Reaktionsgemisch mit Wasser verdünnt und mit Methylenchlorid extrahiert. Die vereinigten Methylenchloridphasen wurden über Magnesiumsulfat getrocknet, eingeengt, und der Rückstand wurde mit Methylenchlorid an Kieselgel chromatographert. So wurde 1-O-[4-(4-Benzyloxy-benzyl)-phenyl]-2,3-O-isopropyliden-4-O-(4-methoxycarbonyl-phenyl)-L-threitol als farbloser Festkörper erhalten, MS: *m*/*z* 568 ([M]$^+$).

D. 2,1 g 1-O-[4-(4-Benzyloxy-benzyl)-phenyl]-2,3-O-isopropyliden-4-O-(4-methoxycarbonyl-phenyl)-L-threitol wurde in 40 ml Tetrahydrofuran unter Zusatz von 0,2 g Palladium auf Kohle (10%) in einer Wasserstoffatmosphäre erschöpfend hydriert. Anschliessend wurde das Reaktionsgemisch über einen 0,8 μ Cellulosefilter filtriert, das Filtrat wurde eingeengt und mit 5% Ether in Methylenchlorid chromatographiert; dabei wurde 1-O-[4-(4-Hydroxy-benzyl)-phenyl]-2,3-O-isopropyliden-4-O-(4-methoxycarbonyl-phenyl)-L-threitol als farbloser Festkörper erhalten, MS: *m*/*z* 478 ([M]$^+$).

E. 0,8 g 2,3:4,5-Di-O-isopropyliden-1,6-bis-O-(4-methyl-phenylsulfonyl)-galactitol, 1,47 g 1-O-[4-(4-Hydroxy-benzyl)-phenyl]-2,3-O-isopropyliden-4-0-(4-methoxycarbonyl-phenyl)-L-threitol und 0,42 g fein gemahlenes wasserfreies Kaliumkarbonat wurden in 10 ml Acetonitril suspendiert und während 200 Stunden unter Rückfluss und unter Argon gerührt. Anschliessend wurde das Reaktionsgemisch mit Wasser verdünnt, mit Methylenchlorid extrahiert, die vereinigten Methylenchloridphasen wurden über Magnesiumsulfat getrocknet, eingeengt. Der Rückstand wurde mit 7% Ether in Methylenchlorid an Kieselgel chromatographert. So wurde 2,3:4,5-Di-O-isopropyliden-1,6-bis-O-[4-[4-[4-O-(4-methoxycarbonyl-phenyl)-2,3-O-isopropyliden-L-threit-1-yloxy]-benzyl]-phenyl]-galactitol in Form farbloser Kristalle erhalten, MS: *m*/*z* 1207,1 ([M+Na]$^+$).

F. 1,05 g 2,3:4,5-Di-O-isopropyliden-1,6-bis-O-[4-[4-[4-O-(4-methoxycarbonyl-phenyl)-2,3-O-isopropyliden-L-threit-1-yloxy]-benzyl]-phenyl]-galactitol wurde in 10 ml 2-Methoxyethanol gelöst, mit 1 ml konzentrierter Natriumhydroxidlösung und 5 ml Wasser versetzt und 5 Stunden unter Rückfluss erwärmt. Hierauf wurde unter Eiskühlung mit 1 N Salzsäure angesäuert, und der gebildete Niederschlag wurde abfiltriert. So wurde 1,6-Bis-O-[4-[4-[4-O-(4-carboxy-phenyl)-2,3-O-isopropyliden-L-threit-1-yloxy]-benzyl]-phenyl]-2,3:4,5-di-O-isopropyliden-galactitol als farbloser Festkörper erhalten, MS: *m*/*z* 1178,1 ([M+Na]$^+$).

G. 0,6 g 1,6-Bis-O-[4-[4-[4-O-(4-carboxy-phenyl)-2,3-O-isopropyliden-L-threit-1-yloxy]-benzyl]-phenyl]-2,3:4,5-di-O-isopropyliden-galactitol, gelöst in 5 ml Dimethylformamid, wurde unter Feuchtigkeitsausschluss bei 0 - 5 °C mit 0,13 ml 4-Methylmorpholin versetzt und während 10 Minuten intensiv gerührt. Hierauf wurde 0,20 g 2-Chlor-4,6-dimethoxy-1,3,5-triazin fest hinzugefügt und weitere 2 Stunden bei 0 - 5 °C gerührt. Anschliessend wurde 0,21 g D-Glucamin zugesetzt und weitere 18 Stunden bei Raumtemperatur gerührt. Hierauf wurde das Reaktionsgemisch mit Wasser verdünnt, eingeengt, erneut mit Wasser versetzt, kurz aufgekocht und filtriert. Dabei resultierte 1,6-Bis-O-[4-[4-[4-O-(4-D-glucit-1-ylcarbamoyl-phenyl)-2,3-O-isopropyliden-L-threit-1-yloxy]-benzyl]-phenyl]-2,3:4,5-di-O-isopropyliden-galactitol als farbloser Festkörper, MS: *m*/*z* 1481,0 ([M]$^+$).

H. 0,7 g 1,6-Bis-O-[4-[4-[4-O-(4-D-glucit-1-ylcarbamoyl-phenyl)-2,3-O-isopropyliden-L-threit-1-yloxy]-benzyl]-phenyl]-2,3:4,5-di-O-isopropyliden-galactitol wurde in 5 ml Dioxan, 1 ml Trifluoressigsäure und 1 ml Wasser suspendiert und 18 Stunden bei 110 °C unter Rückfluss erwärmt. Anschliessend wurde das Reaktionsgemisch zur Trokkene eingeengt, in Wasser suspendiert und filtriert; dabei resultierte 1,6-Bis-O-[4-[4-[4-O-(4-D-glucit-1-ylcarbamoyl-phenyl)-L-threit-1-yloxy]-benzyl]-phenyl]-galactitol als farbloser Festkörper, MS: *m*/*z* 1344,3 ([M+Na]$^+$).

I. Das oben erhaltene 1,6-Bis-O-[4-[4-[4-O-(4-D-glucit-1-ylcarbamoyl-phenyl)-L-threit-1-yloxy]-benzyl]-phenyl]-galactitol wurde analog Beispiel 1.B. zu 1,6-Bis-O-[4-[4-[4-O-[4-(2,3,4,5,6-penta-O-sulfo-D-glucit-1-ylcarbamoyl)-phenyl]-2,3-di-O-sulfo-L-threit-1-yloxy]-benzyl]-phenyl]-2,3,4,5-tetra-O-sulfo-galactitol Octadekanatriumsalz, $[a]_D^{20}$ 0,0° (*c* 0,6; Wasser), MS: *m*/*z* 3158,0 (rekonstruiertes M) umgesetzt.

<u>Beispiel 52</u>

A. 0,50 g 1,6-Bis-O-[4-[4-O-[4-[(E)-2-carboxy-vinyl]-phenyl]-2,3-O-isopropyliden-D-threit-1-yloxy]-phenyl]-2,3:4,5-di-O-isopropyliden-galactitol (Beispiel 48.F.), gelöst in 5 ml Dimethylformamid, wurde unter Feuchtigkeitsausschluss bei 0 - 5 °C mit 0,11 ml 4-Methylmorpholin versetzt und während 10 Minuten intensiv gerührt. Hierauf wurde 0,18 g 2-Chlor-4,6-dimethoxy-1,3,5-triazin fest hinzugefügt und weitere 2 Stunden bei 0 - 5 °C gerührt. Anschliessend wurde 0,21 g N-Methyl-D-glucamin zugesetzt und weitere 18 Stunden bei Raumtemperatur gerührt. Hierauf wurde das Reaktionsgemisch mit Wasser verdünnt, eingeengt, erneut mit Wasser versetzt, kurz aufgekocht und filtriert. Dabei resultierte 1,6-Bis-O-[4-[4-O-[4-[(E)-2-(D-glucit-1-yl-methyl-carbamoyl)-vinyl]-phenyl]-2,3-O-isopropyliden-D-threit-1-yloxy]-phenyl]-2,3:4,5-di-O-isopropyliden-galactitol als farbloser Festkörper, MS: *m*/*z* 1403,7 ([M+Na]$^+$).

B. 0,64 g 1,6-Bis-O- [4-[4-O-[4-[(E)-2-(D-glucit-1-yl-methyl-carbamoyl)-vinyl]-phenyl]-2,3-O-isopropyliden-D-threit-1-yloxy] -phenyl]-2,3:4,5-di-O-isopropyliden-galactitol wurde in 4 ml Dioxan, 1 ml Trifluoressigsäure und 1 ml Wasser suspendiert und 18 Stunden bei 110 °C unter Rückfluss erwärmt. Anschliessend wurde das Reaktionsgemisch zur Trockene eingeengt, mit 10 ml Wasser und 0,2 ml Triethylamin versetzt und filtriert; der so erhaltene Rückstand wurde in 10 ml Essigsäureanhydrid und 10 ml Pyridin gelöst und 18 Std. bei Raumtemperatur gerührt. Anschliessend

wurde das Reaktionsgemisch mit Wasser verdünnt, die Wasserphase wurde mit Methylenchlorid extrahiert, und die vereinigten Methylenchloridphasen wurden über Magnesiumsulfat getrocknet und eingeengt. Das so erhaltene Rohprodukt wurde an Kieselgel mit 5% Isopropanol in Methylenchlorid chromatographiert. Dabei wurde 2,3,4,5-Tetra-O-acetyl-1,6-bis-O-[4-[2,3-di-O-acetyl-4-O-[4-[(E)-2-[(2,3,4,5,6-penta-O-acetyl-D-glucit-1-yl)-methyl-carbamoyl]-vinyl]-phenyl]-D-threit-1-yloxy]-phenyl]-galactitol als farbloser Festkörper erhalten, MS: *m/z* 1978,6 ($[M+H]^+$).

C. 0,34 g 2,3,4,5-Tetra-O-acetyl-1,6-bis-O-[4-[2,3-di-O-acetyl-4-O-[4-[(E)-2-[(2,3,4,5,6-penta-O-acetyl-D-glucit-1-yl)-methyl-carbamoyl]-vinyl]-phenyl]-D-threit-1-yloxy]-phenyl]-galactitol wurde in 4 ml Methanol und 4 ml Tetrahydrofuran gelöst, mit 0,3 ml 1 molarer Natriummethylatlösung in Methanol versetzt und während 18 Stunden bei Raumtemperatur gerührt. Der dabei gebildete Niederschlag wurde abfiltriert und mit Methanol gewaschen. Dabei resultierte 1,6-Bis-O-[4-[4-O-[4-[(E)-2-(D-glucit-1-yl-methyl-carbamoyl)-vinyl]-phenyl]-D-threit-1-yloxy]-phenyl]-galactitol als farbloser Festkörper, MS: *m/z* 1244,0 ($[M+Na]^+$).

D. Das oben erhaltene 1,6-Bis-O-[4-[4-O-[4-[(E)-2-(D-glucit-1-yl-methyl-carbamoyl)-vinyl]-phenyl]-D-threit-1-yloxy]-phenyl]-galactitol wurde analog Beispiel 1.B. zu 1,6-Bis-O-[4-[4-O-[4-[(E)-2-[methyl-(2,3,4,5,6-penta-O-sulfo-D-glucit-1-yl)-carbamoyl]-vinyl]-phenyl]-2,3-di-O-sulfo-D-threit-1-yloxy]-phenyl]-2,3,4,5-tetra-O-sulfo-galactitol Octadekanatriumsalz, $[\alpha]_D^{20}$ +2,4° (*c* 0,8; Wasser), umgesetzt.

Beispiel 53

A. 0,23 g 1,6-Bis-O-[4-[4-O-[4-[(E)-2-[methyl-(2,3,4,5,6-penta-O-sulfo-D-glucit-1-yl)-carbamoyl]-vinyl]-phenyl]-2,3-di-O-sulfo-D-threit-1-yloxy]-phenyl]-2,3,4,5-tetra-O-sulfo-galactitol Octadekanatriumsalz (Beispiel 52.D.) wurde in 5 ml destilliertem Wasser unter Zusatz von 50 mg Palladium auf Kohle (10%)ineinerWasserstoffatmosphäre erschöpfend hydriert. Anschliessend wurde das Reaktionsgemisch über einen 0,8 μ Cellulosefilter filtriert, und das Filtrat wurde lyophilisiert; dabei wurde 1,6-Bis-O-[4-[4-O-[4-[2-[methyl-(2,3,4,5,6-penta-O-sulfo-D-glucit-1-yl)-carbamoyl]-ethyl]-phenyl]-2,3-di-O-sulfo-D-threit-1-yloxy]-phenyl]-2,3,4,5-tetra-O-sulfo-galactitol Oktadeka-natriumsalz erhalten, $[\alpha]_D^{20}$ -2,5° (*c* 0,6; Wasser), MS: *m/z* 3060,5 (rekonstruiertes M).

Beispiel 54

A. 1,42 g 2,3:4,5-Di-O-isopropyliden-1,6-bis-O-(4-methyl-phenylsulfonyl)-galactitol, 1,25 g 3-Benzyloxy-naphthalin-2-ol (E. Weber et al., Chem. Ber. 122, 959 (1989)) und 1,40 g fein gemahlenes wasserfreies Kaliumkarbonat wurden in 20 ml Dimethylformamid suspendiert und während 16 Stunden bei 100 °C unter Argon gerührt. Anschliessend wurde das Reaktionsgemisch mit Eiswasser verdünnt, der gebildete Niederschlag wurde abfiltriert und in Methylenchlorid gelöst. Die Methylenchloridphase wurde über Magnesiumsulfat getrocknet und eingeengt. So wurde 1,6-Bis-O-(3-benzyloxy-naphthalin-2-yl)-2,3:4,5-di-O-isopropyliden-galactitol in Form farbloser Kristalle erhalten, MS: *m/z* 726 ($[M]^+$).

B. 1,4 g 1,6-Bis-O-(3-benzyloxy-naphthalin-2-yl)-2,3:4,5-di-O-isopropyliden-galactitol wurden in 50 ml Ethylacetat unter Zusatz von 0,25 g Palladium auf Kohle (10%) in einer Wasserstoffatmosphäre erschöpfend hydriert. Anschliessend wurde das Reaktionsgemisch über einen 0,8 μ Cellulosefilter filtriert, das Filtrat wurde eingeengt und aus Ether/Hexan umkristallisiert; dabei wurde 1,6-Bis-O-(3-hydroxy-naphthalin-2-yl)-2,3:4,5-di-O-isopropyliden-galactitol als farbloser Festkörper erhalten, MS: *m/z* 546 ($[M]^+$).

C. 3,76 g 2,3-O-Isopropyliden-1,4-bis-O-(4-methyl-phenylsulfonyl)-L-threitol, 1,60 g 3-Hydroxy-naphthalin-2-carbonsäuremethylester und 1,3 g fein gemahlenes wasserfreies Kaliumkarbonat wurden in 50 ml Acetonitril suspendiert und während 60 Stunden unter Rückfluss und unter Argon gerührt. Anschliessend wurde das Reaktionsgemisch mit Wasser verdünnt und mit Methylenchlorid extrahiert. Die vereinigten Methylenchloridphasen wurden über Magnesiumsulfat getrocknet, eingeengt, und der Rückstand wurde mit 5% Ether in Methylenchlorid an Kieselgel chromatographert. So wurde 2,3-O-Isopropyliden-1-O-(3-methoxycarbonyl-naphthalin-2-yl)-4-O-(4-methyl-phenylsulfonyl)-L-threitol als farbloses Oel erhalten, MS: *m/z* 500 ($[M]^+$).

D. 1,3 g 2,3-O-Isopropyliden-1-O-(3-methoxycarbonyl-naphthalin-2-yl)-4-O-(4-methyl-phenylsulfonyl)-L-threitol, 0,62g 1,6-Bis-O-(3-hydroxy-naphthalin-2-yl)-2,3:4,5-di-O-isopropyliden-galactitol und 0,48 g fein gemahlenes wasserfreies Kaliumkarbonat wurden in 20 ml Dimethylformamid suspendiert und während 8 Stunden bei 100 °C unter Argon gerührt. Anschliessend wurde das Reaktionsgemisch mit Wasser verdünnt und mit Methylenchlorid extrahiert. Die vereinigten Methylenchloridphasen wurden über Magnesiumsulfat getrocknet und eingeengt, und der Rückstand wurde mit 5% Ether in Methylenchlorid an Kieselgel chromatographert. So wurde 2,3:4,5-Di-O-isopropyliden-1,6-bis-O-[3-[2,3-O-isopropyliden-4-O-(3-methoxycarbonyl-naphthalin-2-yl)-L-threit-1-yloxy]-naphthalin-2-yl]-galactitol als farbloser Festkörper erhalten, MS: *m/z* 1220,5 ($[M+NH_4]^+$).

E. 0,9 g 2,3:4,5-Di-O-isopropyliden-1,6-bis-O-[3-[2,3-O-isopropyliden-4-O-(3-methoxycarbonyl-naphthalin-2-yl)-L-threit-1-yloxy]-naphthalin-2-yl]-galactitol wurde in 10 ml 2-Methoxyethanol gelöst, mit 1,5 ml konzentrierter Natriumhydroxidlösung und 2 ml Wasser versetzt und 16 Std. unter Rückfluss erwärmt. Hierauf wurde unter Eiskühlung

mit 1 N Salzsäure angesäuert, und der gebildete Niederschlag wurde abfiltriert. So wurde 1,6-Bis-O-[3-[4-O-(3-carboxy-naphthalin-2-yl)-2,3-O-isopropyliden-L-threit-1-yloxy]-naphthalin-2-yl]-2,3:4,5-di-O-isopropyliden-galactitol als farbloser Festkörper erhalten, MS: *m/z* 1173,2 ($[M-H]^-$).

F. 0,9 g 1,6-Bis-O-[3-[4-O-(3-carboxy-naphthalin-2-yl)-2,3-O-isopropyliden-L-threit-1-yloxy]-naphthalin-2-yl]-2,3:4,5-di-O-isopropyliden-galactitol wurde in 16 ml Dioxan, 2 ml Trifluoressigsäure und 2 ml Wasser suspendiert und 18 Stunden bei 110 °C unter Rückfluss erwärmt. Anschliessend wurde das Reaktionsgemisch zur Trockene eingeengt, in Wasser suspendiert und filtriert; dabei resultierte 1,6-Bis-O-[3-[4-O-(3-carboxy-naphthalin-2-yl)-L-threit-1-yloxy]-naphthalin-2-yl]-galactitol als farbloser Festkörper, MS: *m/z* 1013,0 ($[M-H]^-$).

G. 0,75 g 1,6-Bis-O-[3-[4-O-(3-carboxy-naphthalin-2-yl)-L-threit-1-yloxy]-naphthalin-2-yl]-galactitol wurde in 10 ml Essigsäureanhydrid und 10 ml Pyridin gelöst und 24 Std. bei Raumtemperatur gerührt. Anschliessend wurde das Reaktionsgemisch mit Wasser verdünnt, und der gebildete Niederschlag wurde abfiltriert. Dieser wurde hierauf in 20 ml Dioxan suspendiert, mit 10 ml Pyridin und 5 ml Wasser versetzt und 3 Stunden bei Raumtemperatur gerührt. Nun wurde das Reaktionsgemisch erneut eingeengt, mit eiskalter 1 N Salzsäure versetzt, und der dabei gebildete Niederschlag wurde abfiltriert. So wurde 2,3,4,5-Tetra-O-acetyl-1,6-bis-O-[3-[2,3-di-O-acetyl-4-O-(3-carboxy-naphthalin-2-yl)-L-threit-1-yloxy]-naphthalin-2-yl]-galactitol als farbloser Festkörper erhalten, MS: *m/z* 1349,2 ($[M-H]^-$).

H. 0,96 g 2,3,4,5-Tetra-O-acetyl-1,6-bis-O-[3-[2,3-di-O-acetyl-4-O-(3-carboxy-naphthalin-2-yl)-L-threit-1-yloxy]-naphthalin-2-yl]-galactitol, gelöst in 7 ml Dimethylformamid, wurde unter Feuchtigkeitsausschluss bei 0 - 5 °C mit 0,19 ml 4-Methylmorpholin versetzt und während 10 Minuten intensiv gerührt. Hierauf wurde 0,29 g 2-Chlor-4,6-dimethoxy-1,3,5-triazin fest hinzugefügt und weitere 2 Stunden bei 0 - 5 °C gerührt. Anschliessend wurde 0,31 g D-Glucamin zugesetzt und weitere 18 Stunden bei Raumtemperatur gerührt. Hierauf wurde das Reaktionsgemisch mit Wasser verdünnt, eingeengt, erneut mit Wasser versetzt, kurz aufgekocht und filtriert. Dabei resultierte 2,3,4,5-Tetra-O-acetyl-1,6-bis-O-[3-[2,3-di-O-acetyl-4-O-(3-D-glucit-1-ylcarbamoyl-naphthalin-2-yl)-L-threit-1-yloxy]-naphthalin-2-yl]-galactitol als farbloser Festkörper, MS: *m/z* 1678 ($[M+H]^+$).

I. 0,50 g 2,3,4,5-Tetra-O-acetyl-1,6-bis-O-[3-[2,3-di-O-acetyl-4-O-(3-D-glucit-1-ylcarbamoyl-naphthalin-2-yl)-L-threit-1-yloxy]-naphthalin-2-yl]-galactitol wurde in 8 ml Methanol und 8 ml Tetrahydrofuran gelöst, mit 0,6 ml 1 molarer Natriummethylatlösung in Methanol versetzt, und das Reaktionsgemisch wurde während 18 Stunden bei Raumtemperatur gerührt. Der dabei gebildete Niederschlag wurde abfiltriert und mit Methanol gewaschen. Dabei resultierte 1,6-Bis-O-[3-[4-O-(3-D-glucit-1-ylcarbamoyl-naphthalin-2-yl)-L-threit-1-yloxy]-naphthalin-2-yl]-galactitol als farbloser Festkörper, MS: *m/z* 1341,2 ($[M+H]^+$).

K. Das oben erhaltene 1,6-Bis-O-[3-[4-O-(3-D-glucit-1-ylcarbamoyl-naphthalin-2-yl)-L-threit-1-yloxy]-naphthalin-2-yl]-galactitol wurde analog Beispiel 1.B. zu 1,6-Bis-O-[3-[4-O-[3-(2,3,4,5,6-penta-O-sulfo-D-glucit-1-ylcarbamoyl)-naphthalin-2-yl]-2,3-di-O-sulfo-L-threit-1-yloxy]-naphthalin-2-yl]-2,3,4,5-tetra-O-sulfo-galactitol Octadekanatriumsalz, $[\alpha]_D^{20}$ +6,1° (*c* 0,9; Wasser) umgesetzt.

Beispiel 55

A. 9,5 g 2,3-O-Isopropyliden-1,4-bis-O-(4-methyl-phenylsulfonyl)-L-threitol und 5,86 g 4-(4-Benzyloxy-benzyl)-phenol (Beispiel 51.A.) wurden unter Zusatz von 27,9 g Kaliumkarbonat in 300 ml Dimethylformamid während 6 Stunden bei 100 °C umgesetzt. Das Reaktionsgemisch wurde anschliessend extrahierend mit Eiswasser und Methylenchlorid aufgearbeitet. Das Rohprodukt wurde an Kieselgel mit n-Hexan/Essigsäureethylester chromatographiert. Man erhielt so 1-O-[4-(4-Benzyloxy-benzyl)-phenyl]-2,3-O-isopropyliden-4-O-(4-methyl-phenylsulfonyl)-L-threitol als farbloses Oel, MS: *m/z* 588,0 ($[M]^+$).

B. 3,0 g 1-O-[4-(4-Benzyloxy-benzyl)-phenyl]-2,3-O-isopropyliden-4-O-(4-methyl-phenylsulfonyl)-L-threitol; 1,0 g 1,6-Bis-O-(4-hydroxy-phenyl)-2,3:4,5-di-O-isopropyliden-galactitol (Beispiel 48.B.) und 0,93 g fein gemahlenes wasserfreies Kaliumkarbonat wurden in 30 ml Dimethylformamid suspendiert und während 48 Stunden bei 100 °C unter Argon gerührt. Anschliessend wurde das Reaktionsgemisch mit Wasser verdünnt und mit Ether extrahiert. Die vereinigten Etherphasen wurden über Magnesiumsulfat getrocknet, eingeengt, und der Rückstand wurde mit 5% Ether in Methylenchlorid an Kieselgel chromatographert. So wurde 1,6-Bis-O-[4-[4-O-[4-(4-Benzyloxy-benzyl)-phenyl]-2,3-O-isopropyliden-L-threit-1-yloxy]-phenyl]-2,3:4,5-di-O-isopropyliden-galactitol als gelbliches Oel erhalten, MS: *m/z* 1296,4 ($[M+NH_4]^+$).

C. 0,5 g 1,6-Bis-O-[4-[4-O-[4-(4-Benzyloxy-benzyl)-phenyl]-2,3-O-isopropyliden-L-threit-1-yloxy]-phenyl]-2,3:4,5-di-O-isopropyliden-galactitol wurde in 10 ml Tetrahydrofuran unter Zusatz von 0,1 g Palladium auf Kohle (10%) in einer Wasserstoffatmosphäre erschöpfend hydriert. Anschliessend wurde das Reaktionsgemisch über einen 0,8 µ Cellulosefilter filtriert und das Filtrat eingeengt; dabei wurde 1,6-Bis-O-[4-[4-O-[4-(4-hydroxy-benzyl)-phenyl]-2,3-0-isopropyliden-L-threit-1-yloxy]-phenyl]-2,3:4,5-di-O-isopropyliden-galactitol als farbloser Festkörper erhalten, MS: *m/z* 1097,2 ($[M-H]^-$).

D. 1,0 g 1,6-Bis-O-[4-[4-O-[4-(4-hydroxy-benzyl)-phenyl]-2,3-O-isopropyliden-L-threit-1-yloxy]-phenyl]-2,3:4,5-di-O-isopropyliden-galactitol, 0,9 g 2,3-0-Isopropyliden-1-O-(4-methoxycarbonyl-phenyl)-4-O-(4-methyl-phenyl-sulfonyl)-L-threitol (Beispiel 51.B.) und 1,0 g fein gemahlenes wasserfreies Kaliumkarbonat wurden in 3 ml Dimethylformamid suspendiert und während 18 Stunden bei 100 °C unter Argon gerührt. Anschliessend wurde das Reaktionsgemisch mit Wasser verdünnt und mit Ether extrahiert, die vereinigten Etherphasen wurden über Magnesiumsulfat getrocknet und eingeengt. Der Rückstand mit 2% - 5% Ether in Methylenchlorid an Kieselgel chromatographert. So wurde 2,3:4,5-Di-O-isopropyliden-1,6-bis-O-[4-[2,3-O-isopropyliden-4-O-[4-[4-[2,3-O-isopropyliden-4-O-(4-methoxycarbonyl-phenyl)-L-threit-1-yloxy]-benzyl]-phenyl]-L-threit-1-yloxy]-phenyl]-galactitol als farbloser Festkörper erhalten, MS: *m/z* 1673,8 ($[M+NH_4]^+$).

E. 0,428 g 2,3:4,5-Di-O-isopropyliden-1,6-bis-O-[4-[2,3-O-isopropyliden-4-O-[4-[4-[2,3-O-isopropyliden-4-O-(4-methoxycarbonyl-phenyl)-L-threit-1-yloxy]-benzyl]-phenyl]-L-threit-1-yloxy]-phenyl]-galactitol wurde in 5 ml 2-Methoxyethanol gelöst, mit 1 ml konzentrierter Natriumhydroxidlösung und 0,5 ml Wasser versetzt und 3 Std. unter Rückfluss erwärmt. Hierauf wurde unter Eiskühlung mit 1 N Salzsäure angesäuert, und der gebildete Niederschlag wurde abfiltriert. So wurde 1,6-Bis-O-[4-[4-O-[4-[4-[4-O-(4-carboxy-phenyl)-2,3-O-isopropyliden-L-threit-1-yloxy]-benzyl]-phenyl]-2,3-O-isopropyliden-L-threit-1-yloxy]-phenyl]-2,3:4,5-di-O-isopropyliden-galactitol als farbloser Festkörper erhalten, MS: *m/z* 1626,5 ($[M-H]^-$).

F. 0,345 g 1,6-Bis-O-[4-[4-O-[4-[4-[4-O-(4-carboxy-phenyl)-2,3-O-isopropyliden-L-threit-1-yloxy]-benzyl]-phenyl]-2,3-O-isopropyliden-L-threit-1-yloxy]-phenyl]-2,3:4,5-di-O-isopropyliden-galactitol, gelöst in 2 ml Dimethylformamid, wurde unter Feuchtigkeitsausschluss bei 0 - 5 °C mit 0,1 ml 4-Methylmorpholin versetzt und während 10 Minuten intensiv gerührt. Hierauf wurde 0,08 g 2-Chlor-4,6-dimethoxy-1,3,5-triazin fest hinzugefügt und weitere 2 Stunden bei 0 - 5 °C gerührt. Anschliessend wurde 0,085 g D-Glucamin zugesetzt und weitere 18 Stunden bei Raumtemperatur gerührt. Hierauf wurde das Reaktionsgemisch mit Wasser verdünnt, eingeengt, erneut mit Wasser versetzt, kurz aufgekocht und filtriert. Dabei resultierte 1,6-Bis-O-[4-[4-O-[4-[4-[4-O-(4-D-glucit-1-ylcarbamoyl-phenyl)-2,3-O-isopropyliden-L-threit-1-yloxy]-benzyl]-phenyl]-2,3-O-isopropyliden-L-threit-1-yloxy]-phenyl]-2,3:4,5-di-O-isopropyliden-galactitol als farbloser Festkörper, MS: *m/z* 1953,4 ($[M+H]^+$).

G. 0,38 g 1,6-Bis-O-[4-[4-O-[4-[4-[4-O-(4-D-glucit-1-ylcarbamoyl-phenyl)-2,3-0-isopropyliden-L-threit-1-yloxy]-benzyl]-phenyl]-2,3-O-isopropyliden-L-threit-1-yloxy]-phenyl]-2,3:4,5-di-O-isopropyliden-galactitol wurde in 9 ml Dioxan, 1,6 ml Trifluoressigsäure und 3 ml Wasser suspendiert und 18 Stunden bei 110 °C unter Rückfluss erwärmt. Anschliessend wurde das Reaktionsgemisch zur Trockene eingeengt, in Wasser suspendiert und filtriert; dabei resultierte 1,6-Bis-O-[4-[4-O-[4-[4-[4-O-(4-D-glucit-1-ylcarbamoyl-phenyl)-L-threit-1-yloxy]-benzyl]-phenyl]-L-threit-1-yloxy]-phenyl]-galactitol als farbloser Festkörper, MS: *m/z* 1737,8 ($[M+Na]^+$).

H. Das oben erhaltene 1,6-Bis-O-[4-[4-O-[4-[4-[4-O-(4-D-glucit-1-ylcarbamoyl-phenyl)-L-threit-1-yloxy]-benzyl]-phenyl]-L-threit-1-yloxy]-phenyl]-galactitol wurde analog Beispiel 1.B. zu 1,6-Bis-O-[4-[4-O-[4-[4-[4-O-[4-(2,3,4,5,6-penta-O-sulfo-D-glucit-1-ylcarbamoyl)-phenyl]-2,3-di-O-sulfo-L-threit-1-yloxy]-benzyl]-phenyl]-2,3-di-O-sulfo-L-threit-1-yloxy]-phenyl]-2,3,4,5-tetra-O-sulfo-galactitol Docosanatriumsalz, $[\alpha]_D^{20}$ +4,7° (*c* 0,6; Wasser), MS: *m/z* 3959,0 (rekonstruiertes M) umgesetzt.

Beispiel 56

A. 1,2 g 1,6-Bis-O-(3-carboxy-naphthalin-1-yl)-2,3:4,5-di-O-isopropyliden-galactitol (Beispiel 19.A.) wurde in 15 ml Dioxan, 5 ml Trifluoressigsäure und 5 ml Wasser suspendiert und 17 Stunden bei 110 °C unter Rückfluss erwärmt. Hierauf wurde das Reaktionsgemisch eingeengt, mit Wasser versetzt und filtriert. Dabei resultierte 1,6-Bis-O-(3-carboxy-naphthalin-1-yl)-galactitol als farbloser Festkörper, MS: *m/z* 521,2 ($[M-H]^-$).

B. 0,96 g 1,6-Bis-O-(3-carboxy-naphthalin-1-yl)-galactitol wurde in einem Gemisch von 6 ml Essigsäureanhydrid und 11 ml Pyridin während 18 Stunden bei Raumtemperatur gerührt. Hierauf wurde das Reaktionsgemisch eingeengt, mit Eiswasser und verdünnter Salzsäure versetzt, und der so gebildete Niederschlag wurde abfiltriert. Dabei resultierte 2,3,4,5-Tetra-O-acetyl-1,6-bis-O-(3-carboxy-naphthalin-1-yl)-galactitol als farbloser Festkörper, MS: *m/z* 689,2 ($[M-H]^-$).

C. Eine Lösung von 30 g Benzyl-2-benzyloxycarbonylamino-2-desoxy-α-D-glucopyranosid (Heyns und Paulsen, Chem. Ber. **88**, 188 (1955)) in 116 ml Pyridin wurde bei 0 °C mit einer Lösung von 19,85 g p-Tolylsulfonylchlorid in 30 ml Dichlormethan versetzt und 4 Stunden bei Raumtemperatur gerührt. Dann wurde auf eiskalte 2 n Schwefelsäure gegossen und mit Dichlormethan extrahiert. Die organischen Phasen wurden mit wässriger Natriumhydrogenkarbonatlösung gewaschen, über Magnesiumsulfat getrocknet und eingeengt. Der Rückstand wurde mit Hexan/ Essigester über Kieselgel chromatographiert und ergab Benzyl-2-benzyloxycarbonylamino-2-desoxy-6-O-(p-tolylsulfonyl)-α-D-glucopyranosid, $[\alpha]_D^{20}$ +101,8° (*c* 0,5; Dioxan), MS: *m/z* 580 ($[M+Na]^+$).

D. Eine Lösung von 32,14 g Benzyl-2-benzyloxycarbonylamino-2-desoxy-6-O-(p-tolylsulfonyl)-α-D-glucopyranosid in 75 ml Dimethylsulfoxid wurde bei Raumtemperatur mit 7,5 g Natriumazid versetzt und 3 Stunden bei 90 °C gerührt. Dann wurde auf Eis/Wasser gegossen. Ausgefallene Kristalle wurden abgenutscht, mit Wasser gewaschen und getrocknet. Es wurde Benzyl-6-azido-2-benzyloxycarbonylamino-2,6-didesoxy-α-D-glucopyranosid erhalten, $[\alpha]_D^{20}$ +119,6° (*c* 0,5; Dioxan), MS: *m/z* 428 ($[M+H]^+$).

E. Eine Lösung von 1,1 g Benzyl-6-azido-2-benzyloxycarbonylamino-2,6-didesoxy-α-D-glucopyranosid in 7,5 ml Tetrahydrofuran und 69 µl Wasser wurde bei Raumtemperatur mit 674 mg Triphenylphosphin versetzt und 24 Stunden gerührt. Dann wurde zu dem dicken Brei 1 ml Wasser gegeben, 30 Minuten weitergerührt und eingeengt. Der Rückstand wurde aus Methanol kristallisiert und ergab Benzyl-6-amino-2-benzyloxycarbonylamino-2,6-didesoxy-α-D-glucopyranosid, $[\alpha]_D^{20}$ +124,2° (*c* 0,6; Aceton), MS: *m*/*z* 403 ($[M+H]^+$).

F. Eine Suspension von 3,0 g Benzyl-6-amino-2-benzyloxycarbonylamino-2,6-didesoxy-α-D-glucopyranosid und 1,33 g D-Gluconsäure-γ-lacton in 60 ml Dioxan wurde bei 80 °C in Lösung gebracht und für weitere 18 Stunden bei dieser Temperatur gehalten. Das ausgefallene Produkt wurde abgenutscht, mit Dioxan gewaschen, getrocknet und ergab D-Gluconsäure-(benzyl-2-benzyloxycarbonylamino-2,6-didesoxy-α-D-glucopyranosid-6-yl)-amid, $[\alpha]_D^{20}$ +120,0° (*c* 0,2; Dioxan), MS: *m*/*z* 603,2 ($[M+Na]^+$).

G. Eine Lösung von 1,4 g D-Gluconsäure-(benzyl-2-benzyloxycarbonylamino-2,6-didesoxy-α-D-glucopyranosid-6-yl)-amid in 5 ml Pyridin wurde mit 2 ml Acetanhydrid während 18 Stunden bei Raumtemperatur acetyliert, eingeengt und ergab 2,3,4,5,6-Penta-O-acetyl-D-gluconsäure-(benzyl-3,4-di-O-acetyl-2-benzyloxycarbonylamino-2,6-didesoxy-α-D-glucopyranosid-6-yl)-amid, $[\alpha]_D^{20}$ +84,0° (*c* 0,2; Dioxan), MS: *m*/*z* 897,3 ($[M+Na]^+$).

H. Eine Lösung von 250 mg 2,3,4,5,6-Penta-O-acetyl-D-gluconsäure-(benzyl-3,4-di-O-acetyl-2-benzyloxycarbonylamino-2,6-didesoxy-α-D-glucopyranosid-6-yl)-amid in 20 ml Dioxan und 2 ml Wasser wurde in Gegenwart von Palladium auf Kohle (10 %) bei Raumtemperatur hydriert. Nach 2 Stunden wurde über Filterhilfe vom Katalysator abfiltriert und eingeengt. Der Rückstand wurde aus Essigester/ Ether kristallisiert und ergab 2,3,4,5,6-Penta-O-acetyl-D-gluconsäure-(benzyl-3,4-di-O-acetyl-2-amino-2,6-didesoxy-α-D-glucopyranosid-6-yl)-amid, $[\alpha]_D^{20}$ +96,0° (*c* 0,2; Dioxan), MS: *m*/*z* 741,1 ($[M+H]^+$).

I. Eine Lösung von 0,60 g 2,3,4,5-Tetra-O-acetyl-1,6-bis-O-(3-carboxy-naphthalin-1-yl)-galactitol in 8 ml Dimethylformamid wurde unter Feuchtigkeitsausschluss bei 0 - 5 °C mit 0,21 ml 4-Methylmorpholin versetzt und während 10 Minuten intensiv gerührt. Hierauf wurde 0,31 g 2-Chlor-4,6-dimethoxy-1,3,5-triazin fest hinzugefügt und weitere 2 Stunden bei 0 - 5 °C gerührt. Anschliessend wurde 1,27 g 2,3,4,5,6-Penta-O-acetyl-D-gluconsäure (benzyl-3,4-di-O-acetyl-2-amino-2,6-didesoxy-a-D-gluco-pyranosid-6-yl)-amid zugesetzt und weitere 18 Stunden bei Raumtemperatur gerührt. Hierauf wurde das Reaktionsgemisch mehrmals mit Wasser verdünnt und unter vermindertem Druck bei 40 - 50 °C eingedampft und zwischen Wasser/Methanol und Methylenchlorid verteilt. Die vereinigten Methylenchloridphasen wurden über Magnesiumsulfat getrocknet, filtriert, eingeengt, und der so erhaltene Rückstand wurde an Kieselgel mit Methylenchlorid/Methanol chromatographiert; dabei wurde 1,6-Bis-O-[3-[benzyl-2,6-didesoxy-6-(2,3,4,5,6-penta-O-acetyl-D-gluconoylamino)-3,4-di-O-acetyl-α-D-glucopyranosid-2-ylcarbamoyl]-naphthalin-1-yl]-2,3,4,5-tetra-O-acetyl-galactitol als farbloses Festkörper erhalten, MS: *m*/*z* 2157,4 ($[M+Na]^+$).

K. 0,71 g 1,6-Bis-O-[3-[benzyl-2,6-didesoxy-6-(2,3,4,5,6-penta-O-acetyl-D-gluconoylamino)-3,4-di-O-acetyl-α-D-glucopyranosid-2-ylcarbamoyl]-naphthalin-1-yl]-2,3,4,5-tetra-O-acetyl-galactitol wurde in 8 ml Methanol und 8 ml Tetrahydrofuran gelöst, mit 0,3 ml 1 molarer Natriummethylat-Lösung in Methanol versetzt und während 18 Stunden bei Raumtemperatur gerührt. Der dabei gebildete Niederschlag wurde abfiltriert. So resultierte 1,6-Bis-O-[3-(benzyl-2,6-didesoxy-6-D-gluconoylamino-α-D-glucopyranosid-2-ylcarbamoyl)-naphthalin-1-yl]-galactitol als farbloser Festkörper, MS: *m*/*z* 1401,7 ($[M+Na]^+$).

L. Das oben erhaltene 1,6-Bis-O-[3-(benzyl-2,6-didesoxy-6-D-gluconoylamino-α-D-glucopyranosid-2-ylcarbamoyl)-naphthalin-1-yl]-galactitol wurde analog Beispiel 1.B. zu 1,6-Bis-O-[3-[benzyl-2,6-didesoxy-6-(2,3,4,5,6-penta-O-sulfo-D-gluconoylamino)-3,4-di-O-sulfo-α-D-glucopyranosid-2-ylcarbamoyl]-naphthalin-1-yl]-2,3,4,5-tetra-O-sulfo-galactitol Oktadekanatriumsalz, $[\alpha]_D^{20}$ +60,9° (*c* 0,7; Wasser), MS: *m*/*z* 3216,5 (rekonstruiertes M), umgesetzt.

Beispiel 57

A. 5,3 g 2,3:4,5-Di-O-isopropyliden-1,6-bis-O-[(E)-4-(2-methoxycarbonyl-vinyl)-phenyl]-galactitol (Beispiel 3.A.) wurde in 120 ml Dioxan, 15 ml Trifluoressigsäure und 30 ml Wasser suspendiert und 4 Stunden bei 110 °C unter Rückfluss erwärmt. Hierauf wurde das Reaktionsgemisch eingeengt, mit Wasser versetzt und filtriert. Dabei resultierte 1,6-Bis-O-[4-[(E)-2-methoxycarbonyl-vinyl]-phenyl]-galactitol als farbloser Festkörper, MS: *m*/*z* 502 ($[M]^+$).

B. 4,5 g 1,6-Bis-O-[4-[(E)-2-methoxycarbonyl-vinyl]-phenyl]-galactitol, 50 ml Methanol und 40 ml konzentrierte wässrige Natronlauge wurden bei 110 °C 24 Stunden unter Rückfluss erwärmt. Hierauf wurde der grösste Teil des Methanol unter vermindertem Druck abdestilliert, der Rückstand wurde mit Eiswasser verdünnt, mit verdünnter wässriger Salzsäure angesäuert, und die dabei gebildeten Kristalle wurden abfiltriert. So wurde 1,6-Bis-O-[4-[(E)-2-carboxy-vinyl]-phenyl]-galactitol in Form farbloser Kristalle erhalten, MS: *m*/*z* 473,4 ($[M-H]^-$).

C. 4,23 g 1,6-Bis-O-[4-[(E)-2-carboxy-vinyl]-phenyl]-galactitol wurde in einem Gemisch von 25 ml Essigsäureanhydrid und 50 ml Pyridin während 18 Stunden bei Raumtemperatur gerührt. Hierauf wurde das Reaktionsgemisch eingeengt und mit Eiswasser und verdünnter Salzsäure versetzt. Der so gebildete Niederschlag wurde abfiltriert, in 95 ml Dioxan gelöst, mit 50 ml Pyridin und 15 ml Wasser versetzt und weitere 3 Stunden bei Raumtemperatur gerührt; hierauf wurde eingeengt und erneut mit Eiswasser und verdünnter Salzsäure versetzt, und der so gebildete Niederschlag wurde abfiltriert. Dabei resultierte 2,3,4,5-Tetra-O-acetyl-1,6-bis-O-[4-[(E)-2-carboxy-vinyl]-phenyl]-galactitol als farbloser Festkörper, MS: *m/z* 641,2 ([M-H]⁻).

D. 0,55 g 2,3,4,5-Tetra-O-acetyl-1,6-bis-O-[4-[(E)-2-carboxy-vinyl]-phenyl]-galactitol in 8 ml Dimethylformamid wurden unter Feuchtigkeitsaus-schluss bei 0 - 5 °C mit 0,21 ml 4-Methylmorpholin versetzt und während 10 Minuten intensiv gerührt. Hierauf wurde 0,31 g 2-Chlor-4,6-dimethoxy-1,3,5-triazin fest hinzugefügt und weitere 2 Stunden bei 0 - 5 °C gerührt. Anschliessend wurde 1,27 g 2,3,4,5,6-Penta-O-acetyl-D-gluconsäure (benzyl-3,4-di-O-acetyl-2-amino-2,6-didesoxy-α-D-glucopyranosid-6-yl)-amid (Beispiel 56.H.) zugesetzt und weitere 18 Stunden bei Raumtemperatur gerührt. Hierauf wurde das Reaktionsgemisch mehrmals mit Wasser verdünnt und unter vermindertem Druck bei 40 - 50 °C eingedampft und zwischen Wasser/Methanol und Methylenchlorid verteilt. Die vereinigten Methylenchloridphasen wurden über Magnesiumsulfat getrocknet, filtriert, eingeengt, und der so erhaltene Rückstand wurde an Kieselgel mit Methylenchlorid/Methanol chromatographiert; dabei wurde 2,3,4,5-Tetra-O-acetyl-1,6-bis-O-[4-[(E)-2-[benzyl-3,4-di-O-acetyl-2,6-didesoxy-6-(2,3,4,5,6-penta-O-acetyl-D-gluconoylamino)-α-D-glucopyranosid-2-ylcarbamoyl]-vinyl]-phenyl]-galactitol als farbloses Festkörper erhalten, MS: *m/z* 2088,4 ([M+H]⁺).

E. 0,44 g 2,3,4,5-Tetra-O-acetyl-1,6-bis-O-[4-[(E)-2-[benzyl-3,4-di-O-acetyl-2,6-didesoxy-6-(2,3,4,5,6-penta-O-acetyl-D-gluconoylamino)-α-D-glucopyranosid-2-ylcarbamoyl]-vinyl]-phenyl]-galactitol wurde in 5 ml Methanol und 5 ml Tetrahydrofuran gelöst, mit 0,2 ml 1 molarer Natriummethylat-Lösung in Methanol versetzt und während 18 Stunden bei Raumtemperatur gerührt. Der dabei gebildete Niederschlag wurde abfiltriert. So resultierte 1,6-Bis-O-[4-[(E)-2-(benzyl-2,6-didesoxy-6-D-gluconoylamino-a-D-glucopyranosid-2-ylcarbamoyl)-vinyl]-phenyl]-galactitol als farbloser Festkörper, MS: *m/z* 1354,5 ([M+Na]⁺).

F. Das oben erhaltene 1,6-Bis-O-[4-[(E)-2-(benzyl-2,6-didesoxy-6-D-gluconoylamino-α-D-glucopyranosid-2-ylcarbamoyl)-vinyl]-phenyl]-galactitol wurde analog Beispiel 1.B. zu 1,6-Bis-O-[4-[(E)-2-[benzyl-2,6-didesoxy-6-(2,3,4,5,6-penta-O-sulfo-D-gluconoylamino)-3,4-di-O-sulfo-α-D-gluco-pyranosid-2-ylcarbamoyl]-vinyl]-phenyl]-2,3,4,5-tetra-O-sulfo-galactitol Octadekanatriumsalz, $[\alpha]^{20}_{D}$ +45,6° (*c* 0,7; Wasser), MS: *m/z* 3167,5 (rekonstruiertes M), umgesetzt.

Beispiel 58

A. 0,88 g 2,3,4,5-Tetra-O-acetyl-1,6-bis-O-[4-[(E)-2-[benzyl-3,4-di-O-acetyl-2,6-didesoxy-6-(2,3,4,5,6-penta-O-acetyl-D-gluconoylamino)-α-D-glucopyranosid-2-ylcarbamoyl]-vinyl]-phenyl]-galactitol (Beispiel 57.D.) wurde in 20 ml Tetrahydrofuran unter Zusatz von 350 mg Palladium auf Kohle (10%) in einer Wasserstoffatmosphäre erschöpfend hydriert. Anschliessend wurde das Reaktionsgemisch über einen 0,8 µ Cellulosefilter filtriert und eingeengt. Der so erhaltene Rückstand wurde mit Methylenchlorid/ Methanol an Kieselgel chromatographiert; dabei wurde 2,3,4,5-Tetra-O-acetyl-1,6-bis-O-[4-[2-[benzyl-3,4-di-O-acetyl-2,6-didesoxy-6-(2,3,4,5,6-penta-O-acetyl-gluconoylamino)-a-D-glucopyranosid-2-ylcarbamoyl]-ethyl]-phenyl]-galactitol als farbloses Oel, MS: *m/z* 2114,5 ([M+Na]⁺), erhalten.

B. 0,24 g 2,3,4,5-Tetra-O-acetyl-1,6-bis-O-[4-[2-[benzyl-3,4-di-O-acetyl-2,6-didesoxy-6-(2,3,4,5,6-penta-O-acetyl-gluconoylamino)-α-D-glucopyranosid-2-ylcarbamoyl]-ethyl]-phenyl]-galactitol wurde in 2 ml Methanol und 2 ml Tetrahydrofuran gelöst, mit 0,15 ml 1 molarer Natriummethylat-Lösung in Methanol versetzt und während 18 Stunden bei Raumtemperatur gerührt. Der dabei gebildete Niederschlag wurde abfiltriert. So resultierte 1,6-Bis-O-[4-[2-(benzyl-2,6-didesoxy-6-gluconoylamino-α-D-glucopyranosid-2-ylcarbamoyl)-ethyl]-phenyl]-galactitol als farbloser Festkörper, MS: *m/z* 1357,4 ([M+Na]⁺).

C. Das oben erhaltene 1,6-Bis-O-[4-[2-(benzyl-2,6-didesoxy-6-gluconoylamino-α-D-glucopyranosid-2-ylcarbamoyl)-ethyl]-phenyl]-galactitol wurde analog Beispiel 1.B. zu 1,6-Bis-O-[4-[2-[benzyl-2,6-didesoxy-6-(2,3,4,5,6-penta-O-sulfo-D-gluconoylamino)-3,4-di-O-sulfo-α-D-glucopyranosid-2-ylcarbamoyl]-ethyl]-phenyl]-2,3,4,5-tetra-O-sulfo-galactitol Oktadekanatriumsalz, $[\alpha]^{20}_{D}$ +48,8° (*c* 0,5; Wasser), MS: *m/z* 3172,5 (rekonstruiertes M), umgesetzt.

Beispiel 59

A. Eine Lösung von 7,4 g 4-Fluor-3-nitro-benzoesäure in 100 ml Dimethylformamid wurde mit 16,0 g D-Glucamin versetzt und 4 Stunden bei Raumtemperatur gerührt. Nach Zugabe von 6 ml Triethylamin wurde 16 Stunden bei 40 °C weitergerührt. Die Reaktionslösung wurde eingedampft. Der Rückstand wurde mit 400 ml Pyridin und 200 ml Essigsäureanhydrid 5 Stunden bei Raumtemperatur gerührt. Nach Einengen wurde der erhaltene Rückstand mit Wasser versetzt und mit 5 %iger Salzsäurelösung auf pH = 2 -3 angesäuert und mit Essigester extrahiert. Die organischen Extrakte wurden mit Eiswasser und gesättigter Kochsalzlösung gewaschen, über Magnesiumsulfat getrocknet und eingedampft. Der Rückstand wurde mit Essigester über Kieselgel chromatographiert. Die Produktfraktionen wurden eingeengt, aus Ether kristallisiert und ergaben 4-(2,3,4,5,6-Penta-O-acetyl-D-glucit-1-ylamino)-3-nitro-benzoesäure als gelbe Kristalle, $[\alpha]^{20}_{D}$ -23,0° (c 0,5; DMSO), MS: *m/z* 579,7 ([M+Na]⁺).

B. Zu einer Lösung von 27,8 g 4-(2,3,4,5,6-Penta-O-acetyl-D-glucit-1-ylamino)-3-nitro-benzoesäure und 5,32

g 4-Methylmorpholin in 150 ml absolutem Dimethylformamid wurde 8,78 g 2-Chlor-2,4-dimethoxy-1,3,5-triazin bei 0 °C gegeben. Das Reaktionsgemisch wurde 2 Stunden bei dieser Temperatur gerührt und dann mit 14,0 g Benzyl-2-amino-2-desoxy-α-D-glucopyranosid (Meyer zu Reckendorf, Chem. Ber. **107**, 869 (1974)) versetzt. Das Gemisch wurde 18 Stunden weitergerührt und dann im Vakuum konzentriert. Der verbleibende Sirup wurde durch Chromatographie an Kieselgel mit Methylenchlorid/ Isopropanol gereinigt und ergab Benzyl-2-desoxy-2-[3-nitro-4-(2,3,4,5,6-penta-O-acetyl-D-glucit-1-ylamino)-benzoylamino]-α-D-glucopyranosid, $[\alpha]_D^{20}$ +33,6° (c 0,5; DMSO), MS: *m/z* 808,4 ([M+H]$^+$).

C. Zu einer Lösung von 30,0 g Benzyl-2-desoxy-2-[3-nitro-4-(2,3,4,5,6-penta-O-acetyl-D-glucit-1-ylamino)-benzoylamino]-α-D-glucopyranosid in 250 ml absolutem Pyridin wurde 10,9 g p-Toluolsulfonylchlorid in Portionen zugegeben. Nach beendeter Zugabe wurde das Reaktionsgemisch 7 Stunden bei Raumtemperatur gerührt und dann eingeengt. Der Rückstand wurde in Essigester aufgenommen und mit Wasser extrahiert. Die organischen Phasen wurden mit verdünnter Schwefelsäure, Wasser und gesättigter Kochsalzlösung gewaschen, über Magnesiumsulfat getrocknet und eingedampft. Der Rückstand wurde mit Methylenchlorid/Isopropanol auf Kieselgel chromatographiert und ergab Benzyl-2-desoxy-2-[3-nitro-4-(2,3,4,5,6-penta-O-acetyl-D-glucit-1-ylamino)-benzoylamino]-6-O-(p-tolylsulfonyl)-α-D-glucopyranosid, $[\alpha]_D^{20}$ +31,4° (*c* 0,5; DMSO), MS: *m/z* 984,7 ([M+K]$^+$).

D. Zu einer Lösung von 31,0 g Benzyl-2-desoxy-2-[3-nitro-4-(2,3,4,5,6-penta-O-acetyl-D-glucit-1-ylamino)-benzoylamino]-6-O-(p-tolylsulfonyl)-α-D-glucopyranosid in 250 ml absolutem Dimethylformamid wurde 6,5 g Natriumazid gegeben. Das Reaktionsgemisch wurde 6 Stunden bei 65 °C gerührt und dann eingeengt. Der Rückstand wurde in Eiswasser gegossen und mit Essigester extrahiert. Die organischen Phasen wurden mit Wasser und gesättigter Kochsalzlösung gewaschen, über Magnesiumsulfat getrocknet und eingedampft. Der Rückstand wurde mit Methylenchlorid/Isopropanol auf Kieselgel chromatographiert und ergab Benzyl-6-azido-2,6-didesoxy-2-[3-nitro-4-(2,3,4,5,6-penta-O-acetyl-D-glucit-1-ylamino)-benzoylamino]-α-D-glucopyranosid, $[\alpha]_D^{20}$ +30,2° (*c* 0,5; DMSO), MS: *m/z* 855,6 ([M+Na]$^+$).

E. Zu einer Lösung von 4,16 g Benzyl-6-azido-2,6-didesoxy-2-[3-nitro-4-(2,3,4,5,6-penta-O-acetyl-D-glucit-1-ylamino)-benzoylamino]-α-D-glucopyranosid in 50 ml Tetraydrofuran und 1,8 ml Wasser wurde 2,15 g Triphenylphosphin gegeben, 20 Stunden bei Raumtemperatur gerührt und dann eingeengt. Der Rückstand wurde mit Essigester/ Methanol über Kieselgel chromatographiert und ergab Benzyl-6-amino-2,6-didesoxy-2-[3-nitro-4-(2,3,4,5,6-penta-O-acetyl-D-glucit-1-ylamino)-benzoylamino]-α-D-glucopyranosid, $[\alpha]_D^{20}$ +37,4° (*c* 0,5; DMSO), MS: *m/z* 829,7 ([M+Na]$^+$).

F. Zu einer Lösung von 301 mg 1,6-Bis-O-(6-carboxy-naphthalin-2-yl)-2,3:4,5-di-O-isopropyliden-galactitol (Beispiel 16.A.) und 137 mg N-methylmorpholin in 3 ml absolutem Dimethylformamid wurde 2-Chlor-2,4-dimethoxy-1,3,5-triazin bei 0 °C gegeben. Das Reaktionsgemisch wurde 2 Stunden bei dieser Temperatur gerührt und dann mit 1,21 g Benzyl-6-amino-2,6-didesoxy-2-[3-mtro-4-(2,3,4,5,6-penta-O-acetyl-D-glucit-1-ylamino)-benzoylamino]-α-D-glucopyranosid versetzt. Das Reaktionsgemisch wurde 20 Stunden bei Raumtemperatur gerührt und dann eingeengt. Der Rückstand wurde mit 20 ml Acetanhydrid in 30 ml Pyridin acetyliert und nach 5 Stunden bei Raumtemperatur eingeengt, dann in Essigester aufgenommen und mit Wasser extrahiert. Die organischen Phasen wurden mit verdünnter Schwefelsäure, Wasser und gesättigter Natriumbikarbonatlösung gewaschen, über Magnesiumsulfat getrocknet und eingedampft. Der Rückstand wurde mit Essigester/Methanol über Kieselgel chromatographiert und ergab 1,6-Bis-O-[6-[benzyl-3,4-di-O-acetyl-2,6-didesoxy-2-[3-nitro-4-(2,3,4,5,6-penta-O-acetyl-D-glucit-1-ylamino)-benzoylamino]-α-D-glucopyranosid-6-ylcarbamoyl]-naphthalin-2-yl]-2,3:4,5-di-O-isopropyliden-galactitol, $[\alpha]_D^{20}$ +43,6° (c 0,5; Chloroform), MS: *m/z* 2370,9 ([M+Na]$^+$).

G. Eine Lösung von 1,0 g 1,6-Bis-O-[6-[benzyl-3,4-di-O-acetyl-2,6-didesoxy-2-[3-nitro-4-(2,3,4,5,6-penta-O-acetyl-D-glucit-1-ylamino)-benzoylamino]-α-D-glucopyranosid-6-ylcarbamoyl]-naphthalin-2-yl]-2,3:4,5-di-O-isopropyliden-galactitol in 100 ml 80 %iger Essigsäure wurde auf dem Dampfbad 3 Stunden erhitzt und dann eingeengt. Der Rückstand wurde mit 20 ml Acetanhydrid in 30 ml Pyridin acetyliert und nach 18 Stunden bei Raumtemperatur eingeengt, dann in Essigester aufgenommen und mit Wasser extrahiert. Die organischen Phasen wurden mit verdünnter Schwefelsäure, gesättigter Natriumbikarbonatlösung und gesättigter Kochsalzlösung gewaschen, über Magnesiumsulfat getrocknet und eingedampft. Der Rückstand wurde mit Methylenchlorid/Isopropanol über Kieselgel chromatographiert und ergab 2,3,4,5-Tetra-O-acetyl-1,6-bis-O-[6-[benzyl-3,4-di-O-acetyl-2,6-didesoxy-2-[3-nitro-4-(2,3,4,5,6-penta-O-acetyl-D-glucit-1-ylamino)-benzoylamino]-α-D-glucopyranosid-6-ylcarbamoyl]-naphthalin-2-yl]-galactitol, $[\alpha]_D^{20}$ +44,6° (*c* 0,5; Chloroform), MS: *m/z* 2358,9 ([M+Na]$^+$).

H. Eine Lösung von 820 mg 2,3,4,5-Tetra-O-acetyl-1,6-bis-O-[6-[benzyl-3,4-di-O-acetyl-2,6-didesoxy-2-[3-nitro-4-(2,3,4,5,6-penta-O-acetyl-D-glucit-1-ylamino)-benzoylamino]-α-D-glucopyranosid-6-ylcarbamoyl]-naphthalin-2-yl]-galactitol in 24 ml Methanol und 6 ml Dimethoxyethan wurde mit 1 ml einer 2 %igen methanolischen Natriummethanolatlösung versetzt und 6 Stunden bei Raumtemperatur gerührt. Der entstandene Niederschlag wurde abgenutscht, mit Methanol gewaschen, am Vakuum bei 60 °C getrocknet und ergab 1,6-Bis-O-[6-[benzyl-2,6-didesoxy-2-(4-D-glucit-1-ylamino-3-nitro-benzoylamino)-α-D-glucopyranosid-6-ylcarbamoyl]-naphthalin-2-yl]-galactitol, $[\alpha]_D^{20}$ +33,5° (*c* 0,5; Dimethylsulfoxid), MS: *m/z* 1701,8 (M + Na)$^+$.

I. Sulfatierung von 1,6-Bis-O-[6-[benzyl-2,6-didesoxy-2-(4-D-glucit-1-ylamino-3-mtro-benzoylamino)-α-D-glucopyranosid-6-ylcarbamoyl]-naphthalin-2-yl]-galactitol wie in Bsp. 26.C. beschrieben ergab 1,6-Bis-O-[6-[benzyl-2,6-didesoxy-2-[3-nitro-4-(2,3,4,5,6-penta-O-sulfo-D-glucit-1-ylamino)-benzoylamino]-3,4-di-O-sulfo-α-D-glucopyranosid-6-ylcarbamoyl]-naphthalin-2-yl]-2,3,4,5-tetra-O-sulfo-galactitol Oktadekanatriumsalz, $[\alpha]_D^{20}$ +43,2° (c 0,5; Wasser), MS: *m*/*z* 3515 (rekonstruiertes M).

Beispiel 60

A. Umsetzung von Benzyl-6-amino-2,6-didesoxy-2-[3-nitro-4-(2,3,4,5,6-penta-O-acetyl-D-glucit-1-ylamino)-benzoylamino]-α-D-glucopyranosid (Beispiel 59.E.) mit 4,4'-Dioxo-5,5'-(2-acetoxy-propan-1,3-diyldioxy)-di-4H-1-benzopyran-2-carbonsäure (hergestellt aus 4,4'-Dioxo-5,5'-(2-hydroxy-propan-1,3-diyldioxy)-di-4H-1-benzopyran-2-carbonsäure analog Beispiel 57.C.) wie unter Bsp. 59.F. beschrieben ergab 4,4'-Dioxo-5,5'-(2-acetoxy-propan-1,3-diyldioxy)-di-4H-1-benzopyran-2-carbonsäure-N,N'-bis-[benzyl-3,4-di-O-acetyl-2,6-didesoxy-2-[4-(2,3,4,5,6-penta-O-acetyl-D-glucit-1-ylamino)-3-nitro-benzoylamino]-α-D-glucopyranosid-6-yl]-amid], $[\alpha]_D^{20}$ +80,0° (*c* 0,5; Chloroform), MS: *m*/*z* 2370,9 ([M+Na]$^+$).

B. Deacetylierung von 4,4'-Dioxo-5,5'-(2-acetoxy-propan-1,3-diyldioxy)-di-4H-1-benzopyran-2-carbonsäure-N,N'-bis-[benzyl-3,4-di-O-acetyl-2,6-didesoxy-2-[4-(2,3,4,5,6-penta-O-acetyl-D-glucit-1-ylamino)-3-nitro-benzoylamino]-α-D-glucopyranosid-6-yl]-amid] wie unter Bsp. 59.H. beschrieben ergab 4,4'-Dioxo-5,5'-(2-hydroxy-propan-1,3-diyldioxy)-di-4H-1-benzopyran-2-carbonsäure-N,N'-bis-[[benzyl-2,6-didesoxy-2-(4-D-glucit-1-ylamino-3-nitro-benzoylaimno)-α-D-glucopyranosid-6-yl]-amid], $[\alpha]_D^{20}$ +47,0° (*c* 0,5; Dimethylsulfoxid), MS: *m*/*z* 1648,8 ([M+Na]$^+$).

C. Sulfatierung von 4,4'-Dioxo-5,5'-(2-hydroxy-propan-1,3-diyldioxy)-di-4H-1-benzopyran-2-carbonsäure-N,N'-bis-[[benzyl-2,6-didesoxy-2-(4-D-glucit-1-ylamino-3-nitro-benzoylamino)-α-D-glucopyranosid-6-yl]-amid] wie unter Bsp. 59.I. beschrieben ergab 4,4'-Dioxo-5,5'-(2-sulfooxy-propan-1,3-diyldioxy)-di-4H-1-benzopyran-2-carbonsäure-N,N'-bis-[[benzyl-2,6-didesoxy-2-[4-(2,3,4,5,6-penta-O-sulfo-D-glucit-1-ylamino)-3-nitro-benzoylamino]-3,4-di-O-sulfo-α-D-glucopyranosid-6-yl]-amid] Pentadekanatriumsalz, $[\alpha]_D^{20}$ +87,0° (*c* 0,2; Wasser), MS: *m*/*z* 3156,5 (rekonstruiertes M).

Beispiel 61

A. Umsetzung von Benzyl-6-amino-2,6-didesoxy-2-[3-nitro-4-(2,3,4,5,6-penta-O-acetyl-D-glucit-1-ylamino)-benzoylamino]-α-D-glucopyranosid (Beispiel 59.E.) mit 2,3,4,5-Tetra-O-acetyl-1,6-bis-O-[4-((E)-2-carboxy-vinyl)-phenyl]-galactitol (Beispiel 57.C.) wie unter Bsp. 59.F. beschrieben ergab 2,3,4,5-Tetra-O-acetyl-1,6-bis-O-[4-[(E)-2-[benzyl-3,4-di-O-acetyl-2,6-didesoxy-2-[4-(2,3,4,5,6-penta-O-acetyl-D-glucit-1-ylamino)-3-nitro-benzoylamino]-α-D-glucopyranosid-6-ylcarbamoyl]-vinyl]-phenyl]-galactitol, $[\alpha]_D^{20}$ +42,4° (*c* 0,5; Chloroform), MS: *m*/*z* 2411,9 ([M+Na]$^+$).

B. Deacetylierung von 2,3,4,5-Tetra-O-acetyl-1,6-bis-O-[4-[(E)-2-[benzyl-3,4-di-O-acetyl-2,6-didesoxy-2-[4-(2,3,4,5,6-penta-O-acetyl-D-glucit-1-ylamino)-3-nitro-benzoylamino]-α-D-glucopyranosid-6-ylcarbamoyl]-vinyl]-phenyl]-galactitol wie unter Bsp. 26.C. beschrieben ergab 1,6-Bis-O-[4-[(E)-2-[benzyl-2,6-didesoxy-2-(4-D-glucit-1-ylamino-3-nitro-benzoylamino)-α-D-glucopyranosid-6-ylcarbamoyl]-vinyl]-phenyl]-galactitol, $[\alpha]_D^{20}$ +45,6° (*c* 0,5; Dimethylsulfoxid), MS: *m*/*z* 1654,8 ([M+Na]$^+$).

C. Sulfatierung von 1,6-Bis-O-[4-[(E)-2-[benzyl-2,6-didesoxy-2-(4-D-glucit-1-ylamino-3-nitro-benzoylamino)-α-D-glucopyranosid-6-ylcarbamoyl]-vinyl]-phenyl]-galactitol wie unter Beispiel 26.C. beschrieben ergab 1,6-Bis-O-[4-[(E)-2-[benzyl-2,6-didesoxy-2-[4-(2,3,4,5,6-penta-O-sulfo-D-glucit-1-ylamino)-3-nitro-benzoylamino]-3,4-di-O-sulfo-α-D-glucopyranosid-6-ylcarbamoyl]-vinyl]-phenyl]-2,3,4,5-tetra-O-sulfo-galactitol Oktadekanatriumsalz, $[\alpha]_D^{20}$ +40,2° (*c* 0,2; Wasser), MS: *m*/*z* 3469,0 (rekonstruiertes M).

Beispiel 62

A. Umsetzung von Benzyl-6-amino-2,6-didesoxy-2-[3-nitro-4-(2,3,4,5,6-penta-O-acetyl-D-glucit-1-ylamino)-benzoylamino]-α-D-glucopyranosid (Beispiel 59.E.) mit 1,4-Bis-O-[3-carboxy-naphthalin-2-yl)-2,3-O-isopropyliden-L-threitol (Beispiel 13.A.) wie unter Bsp. 59.F. beschrieben ergab 1,4-Bis-O-[3-[benzyl-3,4-di-O-acetyl-2,6-didesoxy-2-[4-(2,3,4,5,6-penta-O-acetyl-D-glucit-1-ylamino)-3-nitro-benzoylamino]-α-D-glucopyranosid-6-ylcarbamoyl]-naphthalin-2-yl]-2,3-O-isopropyliden-L-threitol, $[\alpha]_D^{20}$ +63,4° (*c* 0,5; Chloroform), MS: *m*/*z* 2271,0 ([M+Na]$^+$).

B. Umsetzung von 1,4-Bis-O-[3-[benzyl-3,4-di-O-acetyl-2,6-didesoxy-2-[4-(2,3,4,5,6-penta-O-acetyl-D-glucit-1-ylamino)-3-nitro-benzoylamino]-α-D-glucopyranosid-6-ylcarbamoyl]-naphthalin-2-yl]-2,3-O-isopropyliden-L-threitol wie unter Bsp. 59.G. beschrieben ergab 2,3-Di-O-acetyl-1,4-bis-O-[3-[benzyl-3,4-di-O-acetyl-2,6-didesoxy-2-[4-(2,3,4,5,6-penta-O-acetyl-D-glucit-1-ylamino)-3-nitro-benzoylamino]-α-D-glucopyranosid-6-ylcarbamoyl]-naphthalin-2-yl]-L-threitol, $[\alpha]_D^{20}$ +62,4° (*c* 0,5; Chloroform), MS: *m*/*z* 2315,9 ([M+Na]$^+$).

C. Deacetylierung von 2,3-Di-O-acetyl-1,4-bis-O-[3-[benzyl-3,4-di-O-acetyl-2,6-didesoxy-2-[4-(2,3,4,5,6-penta-O-acetyl-D-glucit-1-ylamino)-3-nitro-benzoylamino]-α-D-glucopyranosid-6-ylcarbamoyl]-naphthalin-2-yl]-L-threitol wie unter Bsp. 59.H. beschrieben ergab 1,4-Bis-O-[3-[benzyl-2,6-didesoxy-2-(4-D-glucit-1-ylamino)-3-nitro-benzoylamino)-α-D-glucopyranosid-6-ylcarbamoyl]-naphthalin-2-yl]-L-threitol, $[\alpha]_D^{20}$ +68,8° (*c* 0,5; Dimethylsulfoxid), MS: *m*/*z* 1642,3 ($[M+Na]^+$).

D. Sulfatierung von 1,4-Bis-O-[3-[benzyl-2,6-didesoxy-2-(4-D-glucit-1-ylamino)-3-nitro-benzoylamino)-α-D-glucopyranosid-6-ylcarbamoyl]-naphthalin-2-yl]-L-threitol wie unter Bsp. 26.C. beschrieben ergab 1,4-Bis-O-[3-[benzyl-2,6-didesoxy-2-[4-(2,3,4,5,6-penta-O-sulfo-D-glucit-1-ylamino)-3-nitro-benzoylamino]-3,4-di-O-sulfo-α-D-glucopyranosid-6-ylcarbamoyl]-naphthalin-2-yl]-2,3-di-O-sulfo-L-threitol Hexadekanatriumsalz, $[\alpha]_D^{20}$ +49,6° (*c* 0,5; Wasser), MS: *m*/*z* 3252,5 (rekonstruiertes M).

Beispiel 63

A. Umsetzung von Benzyl-6-amino-2,6-didesoxy-2-[3-nitro-4-(2,3,4,5,6-penta-O-acetyl-D-glucit-1-ylamino)-benzoylamino]-α-D-glucopyranosid (Beispiel 59.E.) mit 1,6-Bis-O-(4'-carboxy-biphenyl-4-yl)-2,3:4,5-di-O-isopropyliden-galactitol (Beispiel 12.B.) wie unter Bsp. 59.F. beschrieben ergab 1,6-Bis-O-[4'-[benzyl-3,4-di-O-acetyl-2,6-didesoxy-2-[3-nitro-4-(2,3,4,5,6-penta-O-acetyl-D-glucit-1-ylamino)-benzoylamino]-α-D-glucopyranosid-6-ylcarbamoyl]-biphenyl-4-yl]-2,3:4,5-di-O-isopropyliden-galactitol, MS: *m*/*z* 2422,8 ($[M+Na]^+$).

B. Umsetzung von 1,6-Bis-O-[4'-[benzyl-3,4-di-O-acetyl-2,6-didesoxy-2-[3-nitro-4-(2,3,4,5,6-penta-O-acetyl-D-glucit-1-ylamino)-benzoylamino]-α-D-glucopyranosid-6-ylcarbamoyl]-biphenyl-4-yl]-2,3:4,5-di-O-isopropyliden-galactitol wie unter Bsp. 59.G. beschrieben ergab 2,3,4,5-Tetra-O-acetyl-1,6-bis-O-[4'-[benzyl-3,4-di-O-acetyl-2,6-didesoxy-2-[3-nitro-4-(2,3,4,5,6-penta-O-acetyl-D-glucit-1-ylamino)-benzoylamino]-α-D-glucopyranosid-6-ylcarbamoyl]-biphenyl-4-yl]-galactitol, $[\alpha]_D^{20}$ +49,8° (*c* 0,5; Chloroform), MS: *m*/*z* 2510,8 ($[M+Na]^+$).

C. Deacetylierung von 2,3,4,5-Tetra-O-acetyl-1,6-bis-O-[4'-[benzyl-3,4-di-O-acetyl-2,6-didesoxy-2-[3-nitro-4-(2,3,4,5,6-penta-O-acetyl-D-glucit-1-ylamino)-benzoylamino]-α-D-glucopyranosid-6-ylcarbamoyl]-biphenyl-4-yl]-galactitol wie unter Bsp. 59.H. beschrieben ergab 1,6-Bis-O-[4'-[benzyl-2,6-didesoxy-2-(4-D-glucit-1-ylamino-3-nitro-benzoylamino)-α-D-glucopyranosid-6-ylcarbamoyl]-biphenyl-4-yl]-galactitol, $[\alpha]_D^{20}$ +32,0° (*c* 0,5; Dimethylsulfoxid).

D. Sulfatierung von 1,6-Bis-O-[4'-[benzyl-2,6-didesoxy-2-(4-D-glucit-1-ylamino-3-nitro-benzoylamino)-α-D-glucopyranosid-6-ylcarbamoyl]-biphenyl-4-yl]-galactitol wie unter Bsp. 26.C. beschrieben ergab 1,6-Bis-O-[4'-[benzyl-2,6-didesoxy-2-[3-nitro-4-(2,3,4,5,6-penta-O-sulfo-D-glucit-1-ylamino)-benzoylamino]-3,4-di-O-sulfo-α-D-glucopyranosid-6-ylcarbamoyl]-biphenyl-4-yl]-2,3,4,5-tetra-O-sulfo-galactitol Oktadekanatriumsalz, $[\alpha]_D^{20}$ +41,5° (*c* 0,5; Wasser), MS: *m*/*z* 3269,0 (rekonstruiertes M).

Beispiel 64

A. 13,80 g 4-Hydroxy-benzoesäure und 45,20 g tert.-Butyl-dimethylchlorsilan wurden unter Zusatz von 40,80 g Imidazol in 500 ml Dimethylformamid während 18 Stunden unter Argon bei Raumtemperatur gerührt. Das Lösungsmittel wurde im Wasserstrahlvakuum abdestilliert und der Rückstand extrahierend mit Eiswasser und Methylenchlorid aufgearbeitet. Das so erhaltene Rohprodukt wurde an Kieselgel mit Methylenchlorid und Acetonitril chromatographiert. Man erhielt dabei 4-(tert.-Butyl-dimethyl-silanyloxy)-benzoesäure, Elementaranalyse berechnet für $C_{13}H_{20}O_3Si$: C= 61,87%, H= 7,99%; gefunden: C= 61,71 %, H= 8,00%.

B. 15,60 g Fmoc-2,4-dimethoxy-4'-(carboxymethyloxy)-benzhydrylamin gebunden an Aminomethyl-Polystyrolharz (0,55 mMol/g, Bachem AG, Bubendorf, Schweiz; No D-1675) wurde mit 20 ml Piperidin in 80 ml Dimethylformamid 30 Minuten bei Raumtemperatur gerührt und dann filtriert. Das Harz wurde zweimal mit je 20 ml Dimethylformamid gewaschen und 15 Minuten am Hochvakuum getrocknet. Das so erhaltene Harz wurde in 80 ml Dimethylformamid suspendiert, dazu wurden 4,42 g 4-(tert.-Butyl-dimethyl-silanyloxy)-benzoesäure, 5,20 g 2-(2-oxo-2H-pyridin-1-yl)-1,1,3,3-tetramethyl-uroniumtetrafluoroborat und 7,53 ml Ethyl-diisopropyl-amin zugegeben und 1 Stunde bei Raumtemperatur gerührt; nach der Filtration des Reaktionsgemisches wurde das Harz nacheinander mit Wasser, Isopropanol und Methylenchlorid gewaschen und während 18 Stunden im Wasserstrahlvakuum bei 40 °C getrocknet.

C. 14,50 g Harz 64.B. wurden in 50 ml Tetrahydrofuran mit 50 ml Tetrabutyl-ammoniumfluorid-Lösung (1 molar in Tetrahydrofuran) versetzt und 1 Stunde bei Raumtemperatur gerührt. Nach Filtration und Waschen des Harzes mit Tetrahydrofuran wurde dieses während 6 Stunden im Wasserstrahlvakuum bei 40 °C getrocknet.

D. 28,40 g Harz 64.C. und 47,00 g 2,3-O-Isopropyliden-1,4-di-O-(4-methyl-phenylsulfonyl)-L-threitol wurden unter Zusatz von 34,80 g Kaliumkarbonat in 100 ml Dimethylformamid während 6 Stunden bei 100 °C gerührt. Nach dem Abkühlen auf Raumtemperatur wurde filtriert und nacheinander mit Wasser, Isopropanol und Methylenchlorid gewaschen und während 18 Stunden im Wasserstrahlvakuum bei 40 °C getrocknet.

E. 23,00 g Harz 64.D. und 24,65 g Bis-(4-hydroxy-phenyl)-methan wurden unter Zusatz von 34,80 g Kaliumkarbonat in 100 ml Dimethylformamid während 6 Stunden bei 100 °C gerührt und analog Beispiel 64.D. aufgearbeitet.

F. 22,00 g Harz 64.E wurden analog Beispiel 64.D. umgesetzt und aufgearbeitet.

G. 23,40 g Harz 64.F. wurden analog Beispiel 64.E. umgesetzt und aufgearbeitet.

H. 22,85 g Harz 64.G. wurden mit 32,93 g Toluol-4-sulfonsäure-(R)-2,2-dimethyl-[1,3]dioxolan-4-ylmethylester unter Zusatz von 33,40 g Kaliumkarbonat in 100 ml Dimethylformamid während 6 Stunden bei 140 °C umgesetzt und analog Beispiel 64.D. aufgearbeitet.

I. 24,60 g Harz 64.H. wurden in 250 ml 95prozentiger wässriger Trifluoressigsäure während 1 Stunde bei Raumtemperatur gerührt; danach wurde filtriert, und das Filtrat wurde eingeengt. Der so erhaltene Rückstand wurde in 75 ml Pyridin mit 25 ml Acetanhydrid gelöst und während 16 Stunden bei Raumtemperatur gerührt. Hierauf wurde das Reaktionsgemisch extrahierend mit Eiswasser und Methylenchlorid aufgearbeitet, und das Rohprodukt wurde an Kieselgel mit Methylenchlorid und Methanol chromatographiert. Man erhielt so (S)-2,2',3,3'-Tetra-O-acetyl-4-O-[4-[4-(2,3-bis-acetoxy-propoxy)-benzyl]-phenyl]-4'-O-(4-carbamoyl-phenyl)-1,1'-(4,4'-methylen-diphenylen)-di-L-threitol, MS: *m/z* 1058,8 ($[M+Na]^+$).

K. 1,50 g (S)-2,2',3,3'-Tetra-O-acetyl-4-O-[4-[4-(2,3-bis-acetoxy-propoxy)-benzyl]-phenyl]-4'-O-(4-carbamoyl-phenyl)-1,1'-(4,4'-methylen-diphenylen)-di-L-threitol wurde in 50 ml Methanol mit 1 ml Natriummethylatlösung (5,4 molar in Methanol) versetzt und 22 Stunden bei Raumtemperatur gerührt. Das Reaktionsgemisch wurde mit Salzsäure auf pH 4-5 gestellt und eingeengt. Das so erhaltene (R)-4'-O-(4-Carbamoyl-phenyl)-4-O-[4-[4-(2,3-dihydroxy-propoxy)-benzyl]-phenyl]-1,1'-(4,4'-methylen-diphenylen)-di-L-threitol wurde während 4 Stunden am Hochvakuum bei Raumtemperatur über Phosphorpentoxid getrocknet und direkt in die nächste Stufe eingesetzt.

L. 1,00 g (R)-4'-O-(4-Carbamoyl-phenyl)-4-O-[4-[4-(2,3-dihydroxy-propoxy)-benzyl]-phenyl]-1,1'-(4,4'-methylen-diphenylen)-di-L-threitol wurden in Analogie zu Beispiel 1.B. mit Schwefeltrioxid-trimethylamin-komplex umgesetzt. Dabei wurde (S)-4-O-[4-[4-(2,3-Bis-sulfooxy-propoxy)-benzyl]-phenyl]-4'-O-(4-carbamoyl-phenyl)-2,2',3,3'-tetra-O-sulfo-1,1-(4,4'-methylen-diphenylen)-di-L-threitol Hexanatriumsalz erhalten, MS: *m/z* 1396,0 (rekonstruiertes M).

Beispiel A

| Tablette: | | |
|---|---|---|
| 1 | Verbindung der Formel Ia oder Ib | 500 mg |
| 2 | Laktose, wasserfrei | 150 mg |
| 3 | Mikrokristalline Cellulose | 150 mg |
| 4 | Polyvinylpyrrolidon | 40 mg |
| 5 | Talk | 50 mg |
| 6 | Magnesiumstearat | 10 mg |
| | Tablettengewicht | 900 mg |

Die Bestandteile 1-4 werden gesiebt und gemischt. Diese Mischung wird mit demineralisiertem Wasser granuliert und das getrocknete Granulat mit den Bestandteilen 5 und 6 vermischt. Man verpresst zu Tabletten geeigneter Form.

Beispiel B

| Pellets: | | |
|---|---|---|
| 1 | Verbindung der Formel Ia oder Ib | 500 mg |
| 2 | Mikrokristalline Cellulose | 200 mg |
| 3 | PRIMOJEL | 70 mg |
| 4 | Aromapulver | 10 mg |
| 5 | Talk | 20 mg |

Die gemischten und gesiebten Bestandteile 1-3 werden mit demineralisiertem Wasser ausreichend befeuchtet und mittels Extruder durch eine geeignete Lochscheibe gepresst. Das Extrudat wird auf einen Pelletierteller überführt, zu Kügelchen ausgerundet und anschliessend getrocknet. Man versetzt mit den gesiebten Bestandteilen 4 und 5 und füllt in Papiersachets (oder ähnliches) ab.

Beispiel C

Injektionslösung:

Zur Herstellung einer Injektionslösung werden 50 mg Verbindung der Formel Ia oder Ib sowie 0,5 mg Tris-Puffer in Wasser für Injektionszwecke ad 1 ml gelöst und der pH-Wert auf 7,4 eingestellt. Die Lösung wird sterilfiltriert und nach Abfüllen in Ampullen autoklaviert.

## Patentansprüche

1. Verbindungen enthaltend mindestens drei Reste von Zuckeralkoholen oder Zuckeralkohol-ähnlichen Verbindungen der allgemeinen Formel Ia und Ib

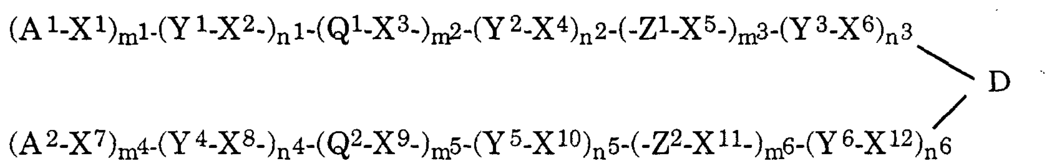

Ia

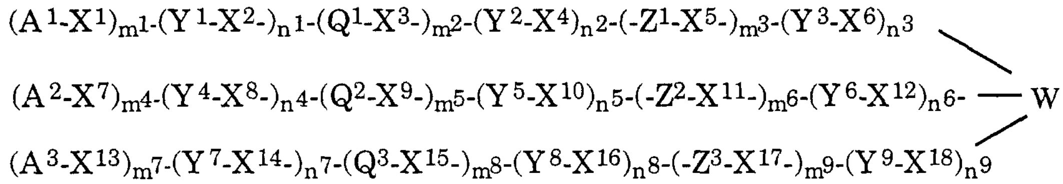

Ib

worin

$n^1$ - $n^9$ unabhängig voneinander je 0 oder 1 sind; und
$m^1$ - $m^9$ unabhängig voneinander je 0 oder 1 sind, jedoch mit der Massgabe, dass mindestens eines von $m^1$, $m^2$ und $m^3$, mindestens eines von $m^4$, $m^5$ und $m^6$ und, falls vorhanden,mindestens eines von $m^7$, $m^8$ und $m^9$, 1 sind; und worin
$X^1$ - $X^{18}$ unabhängig voneinander je -O-, -CONR$^1$-,-NR$^1$CO-, -NR$^1$-;
$R^1$ Wasserstoff oder $C_1$-$C_7$ Alkyl;
W einen Benzolrest oder einen s-Triazinrest;
$Y^1$ - $Y^9$ unabhängig voneinander je aromatische Ringsysteme;
$A^1$ - $A^3$ unabhängig voneinander je einen um die 1-Hydroxygruppe verminderten Rest eines Zuckeralkohols oder eines Derivats davon, einen um die 1-Carboxygruppe verminderten Rest einer Zuckersäure oder eines Derivats davon oder den Tris-(hydroxymethyl)-methylrest;
D den um 2 Hydroxygruppen verminderten Di-Rest eines Zuckeralkohols oder eines Derivats davon oder den um 2 Carboxygruppen verminderten Di-Rest einer Zuckerdicarbonsäure oder eines Derivats davon;
$Q^1$ - $Q^3$ und $Z^1$ - $Z^3$ unabhängig voneinander je den um 2 Hydroxygruppen verminderten Di-Rest eines Zuckeralkohols oder eines Derivats davon oder den um 2 Carboxygruppen verminderten Di-Rest einer Zuckerdicarbonsäure oder eines Derivats davon oder einen Didesoxyglycopyranosid-Rest oder ein Derivat davon bedeuten, wobei mindestens eine Hydroxygruppe der Reste $A^1$ - $A^3$, D, $Q^1$ - $Q^3$ und $Z^1$ - $Z^3$ mit Schwefelsäure verestert ist;
Derivate der Zuckeralkohole sind einfach oder mehrfach desoxygenierte Zuckeralkohole, ein entsprechender Ether an $C_1$ oder eine Verbindung der Formel

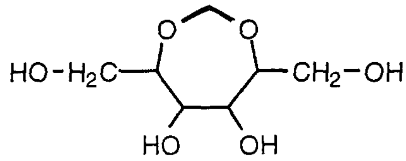

;

Derivate von Zuckermono- und Zuckerdicarbonsäuren sind Zuckersäuren, deren Hydroxyfunktionen frei oder durch Acetonide. Methylenacetale, Arylmethyläther, Alkyl-oder Arylester geschützt, mit Desoxystellen zu Oxiran-Ringen oder mit Carbonsäurefunktionen zu 5 oder 6 gliedrigen Laktonen geschlossen sind;
Derivate des Didesoxyglycopyranosid-Rests sind die entsprechende Ether an $C_1$;

und pharmazeutisch anwendbare Salze davon.

**2.** Verbindungen gemäss Anspruch 1, worin die aromatischen Ringsysteme $Y^1$ - $Y^9$ in Formel Ia und Ib Reste der Formel a -h,

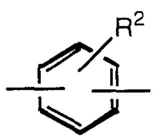 a) 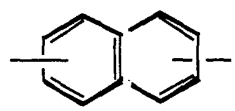 e)

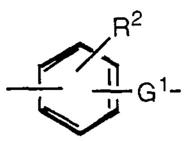 b) 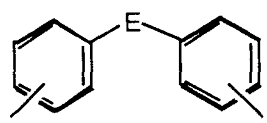 f)

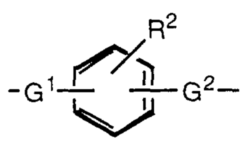 c) 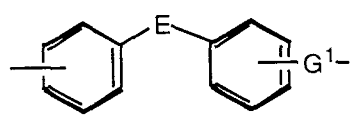 g)

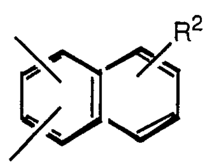 d) 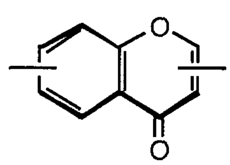 h)

worin

$R^2$ Wasserstoff, Halogen, Nitro, $C_1$-$C_7$ Alkyl, $C_1$-$C_7$ Alkoxy, $C_1$-$C_7$ Alkoxy, welches von Phenyl- und Biphenylresten substituiert ist, Carbamoyl oder den Glycerolrest, wobei gegebenenfalls vorhandene Hydroxygruppen auch sulfatiert sein können,
$G^1$ und $G^2$ $C_1$-$C_7$ Alkylen, $C_2$-$C_7$ Alkenylen, $C_2$-$C_7$ Alkinylen oder $C_1$-$C_7$ Alkylenoxy;
E eine Kohlenstoff-Kohlenstoff-Bindung; -O-, -CO, $-CH_2-$, $-CH_2-CH_2-$,-CH=CH-, -C≡C-, $-NR^3-CO-$, $-CO-NR^3-$; und

$R^3$ Wasserstoff oder $C_1$-$C_7$ Alkyl bedeuten.

**3.** Verbindungen gemäss Anspruch 1 oder 2, worin die von D bzw. W ausgehenden Seitenketten identisch sind.

**4.** Verbindungen gemäss einem der Ansprüche 1-3, worin in Formel Ib $m^2$, $m^3$, $m^5$, $m^6$, $m^8$, $m^9$ und $n^2$, $n^3$, $n^5$, $n^6$, $n^8$, $n^9$ 0 sind.

**5.** Verbindungen gemäss einem der Ansprüche 1-4, worin sich die Reste $A^1$-$A^3$, D, $Q^1$-$Q^3$, $Z^1$-$Z^3$ von einem Hexitol, wie Glucitol, Galaktitol, Mannitol und Gulitol , einem Pentitol, wie Arabinitol, Ribitol und Xylitol, einem Tetritol wie Threitol und Erythritol, von Glycerol oder von Tris-(hydroxy-methyl)-methan ableiten.

**6.** Verbindungen gemäss einem der Ansprüche 1-4, worin sich die Reste $A^1$-$A^3$ von Ribonsäure, Glukonsäure oder Gulonsäure ableiten.

**7.** Verbindungen gemäss einem der Ansprüche 1-3, worin sich die Reste D, $Q^1$-$Q^3$, $Z^1$-$Z^3$ von Weinsäure, Galaktarsäure oder Glukarsäure ableiten.

**8.** Verbindungen gemäss einem der Ansprüche 1-3, worin sich die Reste $Q^1$-$Q^3$, $Z^1$-$Z^3$ von einem Glycopyranosid ableiten.

**9.** 1,6-Bis-O-[4-[2-(2,3,4,5,6-penta-O-sulfo-D-glucit-1-ylcarbamoyl)-ethyl]-phenyl]-2,3,4,5-tetra-O-sulfo-galactitol Tetradekanatriumsalz,

1,6-Bis-O-[4-[2-[4-(2,3,4,5,6-penta-O-sulfo-D-glucit-1-ylcarbamoyl)-phenyl]-ethyl]-phenyl]-2,3,4,5-tetra-O-sulfo-galactitol Tetradekanatriumsalz,

1,6-Bis-O-[3-(2,3,4,5,6-penta-O-sulfo-D-glucit-1-ylcarbamoyl)-naphthalin-2-yl]-2,3,4,5-tetra-O-sulfo-galactitol Tetradekanatriumsalz,

1,6-Bis-O-[6-(2,3,4,5,6-penta-O-sulfo-D-glucit-1-ylcarbamoyl)-naphthalin-2-yl]-2,3,4,5-tetra-O-sulfo-galactitol Tetradekanatriumsalz,

1,6-Bis-O-[3-biphenyl-4-ylmethoxy-5-(2,3,4,5,6-penta-O-sulfo-D-glucit-1-ylcarbamoyl)-phenyl]-2,3,4,5-tetra-O-sulfo-galactitol Tetradekanatriumsalz,

1,6-Bis-O-[6-[(S)-2,3-bis-sulfooxy-propoxy]-3-(2,3,4,5,6-penta-O-sulfo-D-glucit-1-ylcarbamoyl)-naphthalin-2-yl]-2,3,4,5-tetra-O-sulfo-galactitol Octadekanatriumsalz,

1,6-Bis-O-[3-(2,3,4,5-tetra-O-sulfo-D-arabinit-1-yloxy)-naphthalin-2-yl]-2,3,4,5-tetra-O-sulfo-galactitol Dodekanatriumsalz,

2,3,4,5-Tetra-O-sulfo-1,6-bis-O-[4-[4-(2,3,4,5-tetra-O-sulfo-D-arabinit-1-yloxy)-benzyl]-phenyl]-D-mannitol Dodekanatriumsalz,

1,6-Didesoxy-2,3,4,5-tetra-O-sulfo-1,6-bis-[3-(2,3,4,5-tetra-O-sulfo-D-arabinit-1-yloxy)-naphthalin-2-ylcarbonylamino]-galactitol Dodekanatriumsalz,

1,6-Bis-O-[4-[4-O-[4-[(E)-2-(2,3,4,5,6-penta-O-sulfo-D-glucit-1-ylcarbamoyl)-vinyl]-phenyl]-2,3-di-O-sulfo-D-threit-1-yloxy]-phenyl]-2,3,4,5-tetra-O-sulfo-galactitol Octadekanatriumsalz,

1,6-Bis-O-[4-[4-O-[4-[(E)-2-[methyl-(2,3,4,5,6-penta-O-sulfo-D-glucit-1-yl)-carbamoyl]-vinyl]-phenyl]-2,3-di-O-sulfo-D-threit-1-yloxy]-phenyl]-2,3,4,5-tetra-O-sulfo-galactitol Octadekanatriumsalz,

1,6-Bis-O-[3-[benzyl-2,6-didesoxy-6-(2,3,4,5,6-penta-O-sulfo-D-gluconoylamino)-3,4-di-O-sulfo-a-D-glucopyranosid-2-ylcarbamoyl]-naphthalin-1-yl]-2,3,4,5-tetra-O-sulfo-galactitol Oktadekanatriumsalz,

1,6-Bis-O-[4-[2-[benzyl-2,6-didesoxy-6-(2,3,4,5,6-penta-O-sulfo-D-gluconoylamino)-3,4-di-O-sulfo-a-D-glucopyranosid-2-ylcarbamoyl]-ethyl]-phenyl]-2,3,4,5-tetra-O-sulfo-galactitol Oktadekanatriumsalz,

1,6-Bis-O-[6-[benzyl-2,6-didesoxy-2-[3-nitro-4-(2,3,4,5,6-penta-O-sulfo-D-glucit-1-ylamino)-benzoylamino]-3,4-di-O-sulfo-a-D-glucopyranosid-6-ylcarbamoyl]-naphthalin-2-yl]-2,3,4,5-tetra-O-sulfo-galactitol Oktadekanatriumsalz,

1,6-Bis-O-[4-[(E)-2-[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-ylcarbamoyl]-vinyl]-phenyl]-2,3,4,5-tetra-O-sulfo-galactitol Oktadekanatriumsalz.

**10.** Arzneimittel, insbesondere zur Behandlung und/oder Prophylaxe von Krankheiten, die durch exzessive oder destructive Proliferation von glatten Muskelzellen gekennzeichnet sind sowie von arteriosklerotischen Gefässwandveränderungen, enthaltend eine Verbindung nach einem der Ansprüche 1-9 oder ein pharmazeutisch anwendbares Salz davon und einen therapeutisch inerten Träger.

**11.** Verfahren zur Herstellung von Verbindungen nach einem der Ansprüche 1-9, dadurch gekennzeichnet, dass man eine entsprechende, nicht sulfatierte Verbindung Ia oder Ib mit einem Sulfatierungsmittel umsetzt.

**12.** Verbindungen nach einem der Ansprüche 1-9 zur Anwendung als therapeutische Wirkstoffe, insbesondere zur Behandlung und/oder Prophylaxe von Krankheiten, die durch exzessive oder destructive Proliferation von glatten Muskelzellen gekennzeichnet sind sowie von arteriosklerotischen Gefässwandveränderungen.

**13.** Verwendung von Verbindungen nach einem der Ansprüche 1-9 oder von Salzen davon zur Herstellung eines Arzneimittel insbesondere zur Behandlung und/oder Prophylaxe von Krankheiten, die durch exzessive oder destructive Proliferation von glatten Muskelzellen gekennzeichnet sind sowie von arteriosklerotischen Gefässwandveränderungen.

## Claims

**1.** Compounds containing at least three, residues of sugar alcohols or sugar alcohol-like compounds of general formula Ia and Ib

$$\left.\begin{array}{l}(A^1\text{-}X^1)_{m^1}\text{-}(Y^1\text{-}X^2\text{-})_{n^1}\text{-}(Q^1\text{-}X^3\text{-})_{m^2}\text{-}(Y^2\text{-}X^4)_{n^2}\text{-}(\text{-}Z^1\text{-}X^5\text{-})_{m^3}\text{-}(Y^3\text{-}X^6)_{n^3} \\ (A^2\text{-}X^7)_{m^4}\text{-}(Y^4\text{-}X^8\text{-})_{n^4}\text{-}(Q^2\text{-}X^9\text{-})_{m^5}\text{-}(Y^5\text{-}X^{10})_{n^5}\text{-}(\text{-}Z^2\text{-}X^{11}\text{-})_{m^6}\text{-}(Y^6\text{-}X^{12})_{n^6}\end{array}\right\rangle D \qquad \text{Ia}$$

$$\left.\begin{array}{l}(A^1\text{-}X^1)_{m^1}\text{-}(Y^1\text{-}X^2\text{-})_{n^1}\text{-}(Q^1\text{-}X^3\text{-})_{m^2}\text{-}(Y^2\text{-}X^4)_{n^2}\text{-}(\text{-}Z^1\text{-}X^5\text{-})_{m^3}\text{-}(Y^3\text{-}X^6)_{n^3} \\ (A^2\text{-}X^7)_{m^4}\text{-}(Y^4\text{-}X^8\text{-})_{n^4}\text{-}(Q^2\text{-}X^9\text{-})_{m^5}\text{-}(Y^5\text{-}X^{10})_{n^5}\text{-}(\text{-}Z^2\text{-}X^{11}\text{-})_{m^6}\text{-}(Y^6\text{-}X^{12})_{n^6}\text{-} \\ (A^3\text{-}X^{13})_{m^7}\text{-}(Y^7\text{-}X^{14}\text{-})_{n^7}\text{-}(Q^3\text{-}X^{15}\text{-})_{m^8}\text{-}(Y^8\text{-}X^{16})_{n^8}\text{-}(\text{-}Z^3\text{-}X^{17}\text{-})_{m^9}\text{-}(Y^9\text{-}X^{18})_{n^9}\end{array}\right\rangle W \qquad \text{Ib}$$

wherein

$n^1$-$n^9$ are each independently 0 or 1; and
$m^1$-$m^9$ are each independently 0 or 1, but with the proviso that at least one of $m^1$, $m^2$ and $m^3$, at least one of $m^4$, $m^5$ and $m^6$ and, when present, at least one of $m^7$, $m^8$ and $m^9$ is 1; and wherein
$X^1$-$X^{18}$ each independently signify -O-, -CONR$^1$-, -NR$^1$CO-, -NR$^1$-;
$R^1$ signifies hydrogen or $C_1$-$C_7$ alkyl;
W signifies a benzene residue or a s-triazine residue;
$Y^1$-$Y^9$ each independently signify aromatic ring systems;

$A^1$-$A^3$ each independently signify a residue of a sugar alcohol devoid of the 1-hydroxy group or a derivative thereof, a residue of a sugar acid devoid of the 1-carboxy group or a derivative thereof or the tris-(hydroxymethyl)-methyl residue;

D signifies the di-residue of a sugar alcohol devoid of 2 hydroxy groups or a derivative thereof or the di-residue of a sugar dicarboxylic acid devoid of 2 carboxy groups or a derivative thereof;

$Q^1$-$Q^3$ and $Z^1$-$Z^3$ each independently signify the di-residue of a sugar alcohol devoid of 2 hydroxy groups or a derivative thereof or the di-residue of a sugar dicarboxylic acid devoid of 2 carboxy groups or a derivative thereof or a didesoxycyglycopyranoside residue or a derivative thereof, with at least one hydroxy group of residues $A^1$-$A^3$, D, $Q^1$-$Q^3$ and $Z^1$-$Z^3$ being esterified with sulphuric acid;

derivatives of sugar alcohols are mono- or multiply-desoxgenated sugar alcohols, a corresponding ether at $C_1$ or a compound of the formula

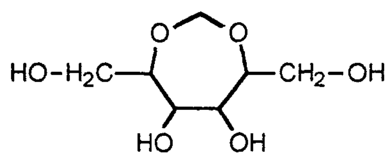

;

derivatives of sugar monocarboxylic acids and sugar dicarboxylic acids are sugar acids in which the hydroxy functions are free or protected by acetonides, methylene acetals, arylmethyl ethers, alkyl esters or aryl esters, [or] closed with desoxy sites to oxirane rings or with carboxylic acid functions to 5- or 6-membered lactones; derivatives of the didesoxyglycopyranoside residue are the corresponding ethers at $C_1$; and pharmaceutically usable salts thereof.

2. Compounds in accordance with claim 1, wherein the aromatic ring systems $Y^1$-$Y^9$ in formula Ia and Ib signify residues of formula a-h:

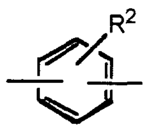
a)

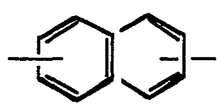
e)

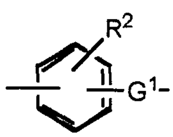
b)

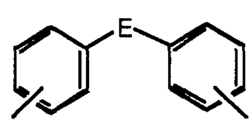
f)

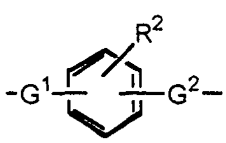
c)

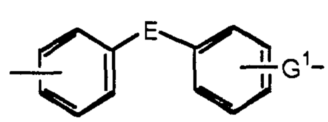
g)

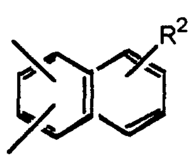
d)

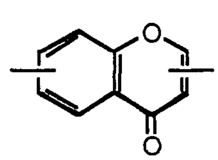
h)

wherein

$R^2$ signifies hydrogen, halogen, nitro, $C_1$-$C_7$ alkyl, $C_1$-$C_7$ alkoxy, $C_1$-$C_7$ alkoxy, which is substituted by phenyl and biphenyl residues, carbamoyl or the glycerol residue, with hydroxy groups which may be present also being optionally sulphated,
$G^1$ and $G^2$ signify $C_1$-$C_7$ alkylene, $C_2$-$C_7$ alkenylene, $C_2$-$C_7$ alkynylene or $C_1$-$C_7$ alkyleneoxy;
E signifies a carbon-carbon bond; -O-, -CO, $-CH_2-$, $-CH_2-CH_2-$, -CH=CH-, -C≡C-, $-NR^3-CO-$, $-CO-NR^3-$; and
$R^3$ signifies hydrogen or $C_1$-$C_7$ alkyl.

**3.** Compounds in accordance with claim 1 or 2, wherein the side-chains emanating from D and, respectively, W are identical.

**4.** Compounds in accordance with any one of claims 1-3, wherein $m^2$, $m^3$, $m^5$, $m^6$, $m^8$, $m^9$ and $n^2$, $n^3$, $n^5$, $n^6$, $n^8$, $n^9$ in formula Ib are 0.

**5.** Compounds in accordance with any one of claims 1-4, wherein the residues $A^1$-$A^3$, D, $Q^1$-$Q^3$, $Z^1$-$Z^3$ are derived from a hexitol such as glucitol, galactitol, mannitol and gulitol, a pentitol such as arabinitol, ribitol and xylitol, a tetritol such as threitol and erythritol, from glycerol or from tris-(hydroxymethyl)-methane.

**6.** Compounds in accordance with any one of claims 1-4, wherein the residues $A^1$-$A^3$ are derived from ribonic acid, gluconic acid or gulonic acid.

**7.** Compounds in accordance with any one of claims 1-3, wherein the residues D, $Q^1$-$Q^3$, $Z^1$-$Z^3$ are derived from tartaric acid, galactaric acid or glucaric acid.

**8.** Compounds in accordance with any one of claims 1-3, wherein the residues $Q^1$-$Q^3$, $Z^1$-$Z^3$ are derived from a glycopyranoside.

**9.** 1,6-Bis-O-[4-[2-(2,3,4,5,6-penta-O-sulpho-D-glucit-1-ylcarbamoyl)-ethyl]-phenyl]-2,3,4,5-tetra-O-sulpho-galactitol tetradecasodium salt,

1,6-bis-O-[4-[2-[4-(2,3,4,5,6-penta-O-sulpho-D-glucit-1-ylcarbamoyl)-phenyl]-ethyl]-phenyl]-2,3,4,5-tetra-O-sulpho-galactitol tetradecasodium salt,
1,6-bis-O-[3-(2,3,4,5,6-penta-O-sulpho-D-glucit-1-ylcarbamoyl)-naphthalen-2-yl]-2,3,4,5-tetra-O-sulpho-galactitol tetradecasodium salt,
1,6-bis-O-[6-(2,3,4,5,6-penta-O-sulpho-D-glucit-1-ylcarbamoyl)-naphthalen-2-yl]-2,3,4,5-tetra-O-sulpho-galactitol tetradecasodium salt,
1,6-bis-O-[3-biphenyl-4-ylmethoxy-5-(2,3,4,5,6-penta-O-sulpho-D-glucit-1-ylcarbamoyl)-phenyl]-2,3,4,5-tetra-O-sulpho-galactitol tetradecasodium salt,
1,6-bis-O-[6-[(S)-2,3-bis-sulphooxy-propoxy]-3-(2,3,4,5,6-penta-O-sulpho-D-glucit-1-ylcarbamoyl)-naphthalen-2-yl]-2,3,4,5-tetra-O-sulpho-galactitol octadecasodium salt,
1,6-bis-O-[3-(2,3,4,5-tetra-O-sulpho-D-arabinit-1-yloxy) -naphthalen-2-yl]-2,3,4,5-tetra-O-sulpho-galactitol dodecasodium salt,
2,3,4,5-tetra-O-sulpho-1,6-bis-O- [4- [4-(2,3,4,5-tetra-O-sulpho-D-arabinit-1-yl-oxy)-benzyl]-phenyl]-D-mannitol dodecasodium salt,
1,6-didesoxy-2,3,4,5-tetra-O-sulpho-1,6-bis-[3-(2,3,4,5-tetra-O-sulpho-D-arabinit-1-yloxy)-naphthalen-2-ylcarbonylamino]-galactitol dodecasodium salt,
1,6-bis-O-[4-[4-O-[4-[(E)-2-(2,3,4,5,6-penta-O-sulpho-D-glucit-1-ylcarbamoyl)-vinyl] -phenyl] -2,3-di-O-sulpho-D-threit-1-yloxy] -phenyl] -2,3,4,5-tetra-O-sulpho-galactitol octadecasodium salt,
1,6-bis-O-[4-[4-O-[4- [(E)-2-[methyl- (2,3,4,5,6-penta-O-sulpho-D-glucit-1-yl)-carbamoyl] -vinyl] -phenyl] -2,3-di-O-sulpho-D-threit- 1-yloxy] -phenyl] -2,3,4,5-tetra-O-sulpho-galactitol octadecasodium salt,
1,6-bis-O-[3-[benzyl 2,6-didesoxy-6-(2,3,4,5,6-penta-O-sulpho-D-gluconoyl-amino)-3,4-di-O-sulpho-a-D-glucopyranosid-2-ylcarbamoyl] -naphthalen-1-yl]-2,3,4,5-tetra-O-sulpho-galactitol octadecasodium salt,
1,6-bis-O-[4-[2-[benzyl 2,6-didesoxy-6-(2,3,4,5,6-penta-O-sulpho-D-gluconoyl-amino)-3,4-di-O-sulpho-a-D-glucopyranosid-2-ylcarbamoyl]-ethyl]-phenyl]-2,3,4,5-tetra-O-sulpho-galactitol octadecasodium salt,
1,6-bis-O-[6-[benzyl 2,6-didesoxy-2-[3-nitro-4-(2,3,4, 5,6-penta-O-sulpho-D-glucit-1-ylamino)-benzoylamino]-3,4-di-O-sulpho-a-D-glucopyranosid-6-ylcarbamoyl]-naphthalen-2-yl]-2,3,4,5-tetra-O-sulpho-galactitol octadecasodium salt,

1,6-bis-O-[4-[(E)-2-[benzyl 2,6-didesoxy-2- [4-(2,3,4,5,6-penta-O-sulpho-D-glucit-1-ylamino)-3-nitro-benzoylamino]-3,4-di-O-sulpho-a-D-glucopyranosid-6-ylcarbamoyl]-vinyl]-phenyl]-2,3,4,5-tetra-O-sulpho-galactitol octadecasodium salt.

**10.** A medicament, especially for the treatment and/or prophylaxis of conditions which are characterized by excessive or destructive proliferation of smooth muscle cells and of arteriosclerotic vascular wall changes, containing a compound according to any one of claims 1-9 or a pharmaceutically usable salt thereof and a therapeutically inert carrier.

**11.** A process for the manufacture of compounds according to any one of claims 1-9, characterized by reacting a corresponding, non-sulphated compound Ia or Ib with a sulphating agent.

**12.** Compounds according to any one of claims 1-9 for use as therapeutically active substances, especially for the treatment and/or prophylaxis of conditions which are characterized by excessive or destructive proliferation of smooth muscle cells and of arteriosclerotic vascular wall changes.

**13.** The use of compounds according to any one of claims 1-9 or of salts thereof for the production of a medicament especially for the treatment and/or prophylaxis of conditions which are characterized by excessive or destructive proliferation of smooth muscle cells and of arteriosclerotic vascular wall changes.

## Revendications

**1.** Composés contenant au moins trois résidus d'alcools de sucre ou de composés analogues aux alcools de sucre, de formule générale la et Ib

$$\left.\begin{array}{l}(A^1\text{-}X^1)_{m1}\text{-}(Y^1\text{-}X^2\text{-})_{n1}\text{-}(Q^1\text{-}X^3\text{-})_{m2}\text{-}(Y^2\text{-}X^4)_{n2}\text{-}(\text{-}Z^1\text{-}X^5\text{-})_{m3}\text{-}(Y^3\text{-}X^6)_{n3} \\ (A^2\text{-}X^7)_{m4}\text{-}(Y^4\text{-}X^8\text{-})_{n4}\text{-}(Q^2\text{-}X^9\text{-})_{m5}\text{-}(Y^5\text{-}X^{10})_{n5}\text{-}(\text{-}Z^2\text{-}X^{11}\text{-})_{m6}\text{-}(Y^6\text{-}X^{12})_{n6}\end{array}\right\rangle D \quad \text{Ia}$$

$$\left.\begin{array}{l}(A^1\text{-}X^1)_{m1}\text{-}(Y^1\text{-}X^2\text{-})_{n1}\text{-}(Q^1\text{-}X^3\text{-})_{m2}\text{-}(Y^2\text{-}X^4)_{n2}\text{-}(\text{-}Z^1\text{-}X^5\text{-})_{m3}\text{-}(Y^3\text{-}X^6)_{n3} \\ (A^2\text{-}X^7)_{m4}\text{-}(Y^4\text{-}X^8\text{-})_{n4}\text{-}(Q^2\text{-}X^9\text{-})_{m5}\text{-}(Y^5\text{-}X^{10})_{n5}\text{-}(\text{-}Z^2\text{-}X^{11}\text{-})_{m6}\text{-}(Y^6\text{-}X^{12})_{n6}\text{-} \\ (A^3\text{-}X^{13})_{m7}\text{-}(Y^7\text{-}X^{14}\text{-})_{n7}\text{-}(Q^3\text{-}X^{15}\text{-})_{m8}\text{-}(Y^8\text{-}X^{16})_{n8}\text{-}(\text{-}Z^3\text{-}X^{17}\text{-})_{m9}\text{-}(Y^9\text{-}X^{18})_{n9}\end{array}\right\rangle W \quad \text{Ib}$$

où

$n^1$-$n^9$ représentent chacun indépendamment de l'autre 0 ou 1 ; et

$m^1$-$m^9$ représentent chacun indépendamment des autres 0 ou 1, mais à la condition qu'au moins l'un des indices $m^1$, $m^2$ et $m^3$, au moins l'un des indices $m^4$, $m^5$ et $m^6$ et, s'ils sont présents, au moins l'un des indices $m^7$, $m^8$ et $m^9$, vaillent 1 ;

et où

$X^1$-$X^{18}$ représentent chacun indépendamment des autres -O-, -CONR$^1$-, -NR$^1$CO-, -NR$^1$- ;

$R^1$ est un atome d'hydrogène ou un groupe alkyle en $C_1$-$C_7$;

W est un résidu de benzène ou d'une s-triazine ;

$Y^1$-$Y^9$ représentent chacun indépendamment des autres un système cyclique aromatique ;

$A^1$-$A^3$ représentent chacun indépendamment des autres un résidu, diminué d'un groupe hydroxy, d'un alcool de sucre ou d'un dérivé de ce dernier, un résidu, diminué d'un groupe carboxy, d'un acide saccharique ou d'un dérivé de ce dernier, ou encore le radical tris-(hydroxyméthyl)-méthyle ;

D est le di-résidu, diminué de 2 groupes hydroxy, d'un alcool de sucre ou d'un dérivé de ce dernier, ou encore le di-résidu, diminué de deux groupes carboxy, d'un acide saccharine dicarboxylique ou d'un de ses dérivés ;

$Q^1$-$Q^3$ et $Z^1$-$Z^3$ représentent chacun indépendamment des autres le di-résidu, diminué de 2 groupes hydroxy, d'un alcool de sucre ou d'un dérivé de ce dernier, ou le di-résidu, diminué de 2 groupes carboxy, d'un acide saccharine-dicarboxylique ou d'un dérivé de ce dernier, ou le résidu didésoxyglycopyrannoside ou un dérivé de ce derènier, où au moins un groupe hydroxy des résidus $A^1$-$A^3$, D, $Q^1$-$Q^3$ et $Z^1$-$Z^3$ est estérifié par l'acide sulfurique ;

les dérivés de l'alcool de sucre sont des alcools de sucre une ou plusieurs fois désoxygénés, un éther correspondant en Ci ou un composé de formule

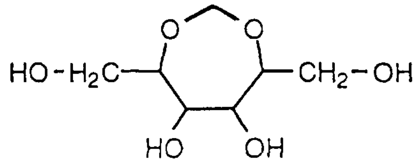 ;

les dérivés des acides saccharinemono- et saccharine-dicarboxyliques sont des alcools sacchariques dont les fonctions hydroxy sont libres ou sont protégées par des acétonides, des méthylènes acétals, des arylméthyléthers, des esters alkyliques ou aryliques, et sont fermées avec des sites désoxy pour donner des noyaux oxiranne ou sont fermées avec des fonctions acide carboxylique pour donner des lactones à 5 ou 6 chaînons ;

les dérivés du résidu didésoxyglycopyrannoside sont les esters correspondants sur $C_1$ ; et leurs sels acceptables d'un point de vue pharmaceutique.

**2.** Composés selon la revendication 1, dans lesquels les systèmes cycliques aromatiques $Y^1$-$Y^9$ des formules la et Ib sont des radicaux de formule a-h

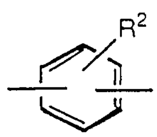 a)

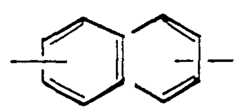 e)

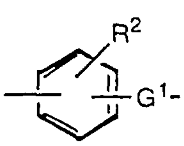 b)

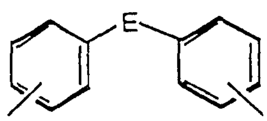 f)

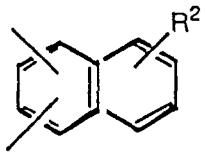

d)

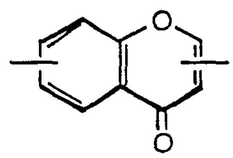

h)

où

$R^2$ est un atome d'hydrogène ou d'halogène, un groupe nitro, alkyle en $C_1$-$C_7$, alcoxy en $C_1$-$C_7$, alcoxy en $C_1$-$C_7$ qui est substitué par des substituants phényle ou biphényle, carbamoyle ou le résidu glycéryle, où les groupes hydroxy éventuellement présents peuvent aussi être sulfatés,

$G^1$ et $G^2$ représentent des groupes alkylène en $C_1$-$C_7$, alcénylène en $C_2$-$C_7$, alcynylène en $C_2$-$C_7$ ou alkylènoxy en $C_1$-$C_7$ ;

E est une liaison carbone-carbone ; -O-, -CO-, $-CH_2-$, $-CH_2-CH_2-$, -CH=CH-, -C≡C-, $-NR^3-CO-$, $-CO-NR^3-$ ; et

$R^3$ est un atome d'hydrogène ou un groupe alkyle en $C_1$-$C_7$.

**3.** Composés selon la revendication 1 ou 2, dans lesquels les chaînes latérales partant de D ou de W sont identiques.

**4.** Composés selon l'une des revendications 1 à 3, dans lesquels, dans la formule Ib, $m^2$, $m^3$, $m^5$, $m^6$, $m^8$, $m^9$ et $n^2$, $n^3$, $n^5$, $n^6$, $n^8$, $n^9$ valent 0.

**5.** Composés selon l'une des revendications 1 à 4, dans lesquels les résidus $A^1$-$A^3$, D, $Q^1$-$Q^3$, $Z^1$-$Z^3$, dérivent d'un hexitol tel que le glucitol, le galactitol, le mannitol et le gulitol, d'un pentitol tel que l'arabinitol, le ribitol et le xylitol, d'un tétritol tel que le thréitol et l'érythritol, du glycérol ou du tris-(hydroxyméthyl)-méthane.

**6.** Composés selon l'une des revendications 1 à 4, dans lesquels les résidus $A^1$-$A^3$ dérivent de l'acide ribonique, de l'acide gluconique ou de l'acide gulonique.

**7.** Composés selon l'une des revendications 1 à 3, dans lesquels les résidus D, $Q^1$-$Q^3$, $Z^1$-$Z^3$, dérivent de l'acide tartrique, de l'acide galactarique ou de l'acide glucarique.

**8.** Composés selon l'une des revendications 1 à 3, dans lesquels les résidus $Q^1$-$Q^3$, $Z^1$-$Z^3$, dérivent d'un glycopyrannoside.

**9.** Sel tétradécasodique du 1,6-bis-O-[4-[2-(2,3,4,5,6-penta-O-sulfo-D-glucit-1-ylcarbamoyl)-éthyl]-phényl]-2,3,4,5-tétra-O-sulfogalactitol,

Sel tétradécasodique du 1,6-bis-O-[4-[2-[4-(2,3,4,5,6-penta-O-sulfo-D-glucit-1-ylcarbamoyl)-phényl]-éthyl]-phényl]-2,3,4,5-tétra-O-sulfogalactitol,

Sel tétradécasodique du 1,6-bis-O-[3-(2,3,4,5,6-penta-O-sulfo-D-glucit-1-ylcarbamoyl)-naphtalène-2yl)-2,3,4,5-tétra-O-sulfogalactitol,

Sel tétradécasodique du 1,6-bis-O-[6-(2,3,4,5,6-penta-O-sulfo-D-glucit-1-ylcarbamoyl)-naphtalène-2-yl)-2,3,4,5-tétra-O-sulfogalactitol,

Sel tétradécasodique du 1,6-bis-O-[3-biphényl-4-ylméthoxy-5-(2,3,4,5,6-penta-O-sulfo-D-glucit-1-yl-carbamoyl)-phényl]-2,3,4,5-tétra-O-sulfogalactitol,

Sel octadécasodique du 1,6-bis-O-[6-[(S)-2,3-bis-sulfooxypropoxy]-3-(2,3,4, 5,6-penta-O-sulfo-D-glucit-1-yl-carbamoyl)-naphtalène-2-yl]-2,3,4,5-tétra-O-sulfogalactitol,

Sel dodécasodique du 1,6-bis-O-[3-(2,3,4,5-tétra-O-sulfo-D-arabinit-1-yloxy)-naphtalène-2-yl]-2,3,4,5-tétra-O-sulfogalactitol,

Sel dodécasodique du 2,3,4,5-tétra-O-sulfo-1,6-bis-O-[4-[4-(2,3,4,5-tétra-O-sulfo-D-arabinit-1-yloxy)-benzyl]-phényl]-D-mannitol,

Sel dodécasodique du 1,6-didésoxy-2,3,4,5-tétra-O-sulfo-1,6-bis-[3-(2,3,4, 5-tétra-O-sulfo-D-arabinit-1-yloxy)-naphtalène-2-ylcarbonylamino]-galactitol,

Sel octadécasodique du 1,6-bis-O-[4-[4-O-[4-[(E)-2-(2,3,4,5,6-penta-O-sulfo-D-glucit-1-ylcarbamoyl)-vinyl]-phényl]-2,3-di-O-sulfo-D-thréit-1-yloxy]-phényl]-2,3,4,5-tétra-O-sulfogalactitol,

Sel octadécasodique du 1,6-bis-O-[4-[4-O-[4[(E)-2-[méthyl-(2,3,4,5,6-penta-O-sulfo-D-glucit-1-yl)-carbamoyl]-vinyl]-phényl]-2, 3-di-O-sulfo-D-threit-1-yloxy]-phényl]-2,3,4,5-tétra-O-sulfogalactitol,

Sel octadécasodique du 1,6-bis-O-[3-[benzyl-2,6-didésoxy-6-(2,3,4,5,6-penta-O-sulfo-D-gluconoylamino)-3,4-di-O-sulfo-a-D-glucopyrannoside-2-ylcarbamoyl]-naphtalène-1-yl]-2,3,4,5-tétra-O-sulfogalactitol,

Sel octadécasodique du 1,6-bis-O-[4-[2-[benzyl-2,6-didésoxy-6-(2,3,4,5,6-penta-O-sulfo-D-gluconoylamino)-3,4-di-O-sulfo-a-D-glucopyrannoside-2-ylcarbamoyl]-éthyl]-phényl]-2,3,4,5-tétra-O-sulfogalactitol,

Sel octadécasodique du 1,6-bis-O-[6-[benzyl-2,6-didésoxy-2-[3-nitro-4-(2,3, 4,5,6-penta-O-sulfo-D-glucit-1-ylcarbamoyl)-benzoylamino]-3,4-di-O-sulfo-a-D-glucopyrannoside-6-ylcarbamoyl]-naphtalène-2-yl]-2,3,4,5-tétra-O-sulfogalactitol,

Sel octadécasodique du 1,6-bis-O-[4-[(E)-2-[benzyl-2,6-didésoxy-2-[4-(2,3, 4,5,6-penta-O-sulfo-D-glucit-1-ylamino)-3-nitrobenzoylamino]-3,4-di-O-sulfo-a-D-glucopyrannoside-6-ylcarbamoyl]-vinyl]-phényl]-2,3,4,5-tétra-O-sulfogalactitol.

**10.** Médicament, en particulier pour le traitement et/ou la prophylaxie de maladies qui sont caractérisées par une prolifération excessive ou destructive de cellules musculaires lisses, ainsi que de lésions artériosclérotiques de la paroi artérielle, contenant un composé selon l'une des revendications 1 à 9 ou l'un de ses sels acceptables d'un point de vue pharmaceutique, et un excipient inerte d'un point de vue thérapeutique.

**11.** Procédé pour préparer des composés selon l'une des revendications 1 à 9, caractérisé en ce qu'on fait réagir avec un agent de sulfatation un composé correspondant la ou Ib, non-sulfaté.

**12.** Composés selon l'une des revendications 1 à 9, pour utilisation en tant que principes actifs thérapeutiques, en particulier pour le traitement et/ou la prophylaxie de maladies caractérisées par une prolifération excessive ou destructive des cellules musculaires lisses, ainsi que de lésions artériosclérotiques de la paroi artérielle.

**13.** Utilisation de composés selon l'une des revendications 1 à 9 ou de leurs sels pour préparer un médicament destiné en particulier au traitement et à la prophylaxie de maladies caractérisées par une prolifération excessive ou destructive de cellules musculaires lisses, ainsi que de lésions artériosclérotiques de la paroi artérielle.